Europäisches Patentamt

(19) European Patent Office

Office européen des brevets

(11) Publication number: **0 314 161**
**A1**

(12) **EUROPEAN PATENT APPLICATION**

(21) Application number: 88117998.0

(22) Date of filing: 28.10.88

(51) Int. Cl.⁴: **C12N 15/00** , **A61K 39/395** ,
**C12N 5/00**

(30) Priority: 28.10.87 US 114632
16.09.88 US 254004

(43) Date of publication of application:
**03.05.89 Bulletin 89/18**

(84) Designated Contracting States:
**AT BE CH DE ES FR GB GR IT LI LU NL SE**

(71) Applicant: **Bristol-Myers Company**
**345 Park Avenue**
**New York New York 10154(US)**

(72) Inventor: **Harris, Linda J.**
**1214 Sixteenth Avenue East**
**Seattle Washington 98112(US)**
Inventor: **Lipsich, Leah A.**
**2506 Fourth Avenue West**
**Seattle Washington 98119(US)**
Inventor: **Walls, Michael A.**
**1909 Franklin Avenue East**
**Seattle Washington 98102(US)**

(74) Representative: **Kinzebach, Werner, Dr.**
**Patentanwälte Reitstötter, Kinzebach und**
**Partner Sternwartstrasse 4 Postfach 86 06 49**
**D-8000 München 86(DE)**

(54) **Human immunoglobulines produced by recombinant DNA techniques.**

(57) Recombinant human immunoglobulins and methods for their production are provided. DNA constructs containing operably linked genes coding for human light and heavy chains are transfected into eucaryotic host cells capable of expressing the human immunoglobulin sequences. Using these methods the isotype of an immunoglobulin may .be switched, or a monoclonal antibody may be produced in a new host cell free of a transforming virus' genome. Also disclosed is the use of cytomegalovirus transcriptional enhancer sequences to increase expression of immunoglobulin genes. The recombinant immunoglobulins find use in a number of ways, including prophylaxis and therapy, diagnosis, and as immunogens.

EP 0 314 161 A1

## HUMAN IMMUNOGLOBULINS PRODUCED BY RECOMBINANT DNA TECHNIQUES

### I. FIELD OF THE INVENTION

The present invention relates to novel methods employing recombinant DNA techniques to provide human immunoglobulin molecules which immunoglobulins may find use in treating or diagnosing disease.

### II. BACKGROUND OF THE INVENTION

Monoclonal antibodies have revolutionized the diagnosis of many diseases and hold great promise to do the same for the treatment and prevention of disease. To be safely administered repeatedly to humans, a therapeutic agent must be able to escape recognition as foreign by the human immune system. Monoclonal antibodies of non-human species, such as mouse or rat, may stimulate vigorous immune responses that neutralize the therapeutic or prophylactic effect of the antibodies, or may trigger harmful anaphylactic or allergic reactions. Human immunoglobulins, on the other hand, are not as likely to elicit such responses and should persist longer in circulation than would immunoglobulins of non-human species. Also, human antibodies may interact more effectively than non-human antibodies with human effector cells.

While a number of monoclonal antibodies have been made to therapeutically important antigenic targets, unfortunately the vast majority of these antibodies are non-human, usually murine. Cell lines which secrete human monoclonal antibodies with desired antigenic specificities are largely unavailable. The generation of human monoclonal antibodies has been hampered by several factors which include, among others, an inability to immunize humans with antigens of interest due to ethical considerations, a low frequency of hybrid formation between human cells, and a low frequency of viral transformation of human cells. Thus, despite the large number of murine cell lines which produce monoclonal antibodies to a wide variety of antigens, in most instances, the comparable human cell lines have not been produced.

When stable cell lines producing human monoclonal antibodies to desired therapeutic targets have been isolated, additional problems have stood between their isolation and eventual use of the antibody as a therapeutic product. For instance, human cell lines generally produce low quantities of antibody when cultured in vitro, 1 to 10 μg/ml, compared to from 40 to 100 μg/ml produced by mouse hybridomas in culture. Human cell lines are poor producers of ascites fluid when injected into mice of other animals, whereas mouse ascites fluid is a convenient source of large quantities of murine antibodies. Another problem arises when the monoclonal antibody-producing cells have been transformed with a virus, a convenient laboratory technique used to immortalize cell lines which produce human monoclonal antibodies. The transformed cells may continue to harbor the viral genome or shed virus into culture supernatants, requiring additional laborious purification and/or sterilization procedures before they can be administered to humans.

It has also been discovered that virally transformed lymphoblastoid cells more frequently produce antibodies of the IgM class, whereas antibodies of the IgG class are often preferred for therapeutic use. This preference for IgG antibodies exists because researchers generally have had more experience in formulating IgG antibodies for therapeutics and because IgG antibodies, but not IgM, pass across the placenta from mother to fetus, thus promoting immunity of newborns to infection. However, in certain instances, an IgM antibody may be preferred, for example, when a particular antigenic determinant or epitope with which the antibody is reactive is scarce on the surface of an antigen such as one located on a bacterial cell. In such cases, an IgM antibody, being pentameric, and therefore better able to "bridge" scarce epitopes, may bind with increased avidity and thereby be able to fix complement more efficiently and more effectively promote killing of the bacterial cell.

Investigators have resorted to recombinant DNA techniques to circumvent the limited repertoire of human monoclonal antibodies to the desired antigens. Since the antigen binding specificity of an antibody molecule is encoded in its variable region domains ($F_V$), genes which code for murine heavy and light chain variable regions ($V_H$ and $V_L$, respectively) of an antibody of a preselected specificity have been combined with genes which code for human immunoglobulin heavy and light chain constant regions ($C_H$ and $C_L$, respectively). This procedure generates "chimeric" mouse/human genes, which then produce a chimeric mouse-human antibody. The chimeric antibody has the antigen binding specificity of the murine antibody and possesses the biological effector functions of the human constant region. Although these chimeric antibodies are partially human in composition, and hence should be less immunogenic when administered to humans, the potential still exists for undesirable immunological reactions to the mouse V regions, especially during periods of prolonged or repeated administration. Ideally, it would be desirable to administer human antibodies of the desired specificity and isotype which are non-chimeric, i.e., are totally human immunoglobulin molecules, and thus less likely to evoke undesirable effects.

What is needed in the art are methods for the efficient and convenient production of human immunoglobulin molecules of a desired antigen binding specificity and isotype. The methods should be able to take advantage of the library of antigen binding specificities of human monoclonal antibodies which have been produced by conventional means. For antibodies which are secreted by cells immortalized by viral transformation, it would be desirable to produce the antibodies or the variable regions thereof apart from the entire transformed cells. The methods should also be capable of producing a functional antibody of a certain class or subclass or possessing other desired characteristics of the antibody effector (constant) region. Quite surprisingly, the present invention fulfills these and other related needs.

## III. SUMMARY OF THE INVENTION

Methods are provided for producing a recombinant human immunoglobulin in a eucaryotic host cell, which methods comprise transfecting into the host cell operably linked first and second genes which code for human heavy chain variable and constant regions, respectively. The host cell is also transfected with operably linked genes coding for the variable and constant regions of a human light chain. The transfections into the host cell may occur simultaneously or sequentially. The transfected cell is cultured and recombinant human immunoglobulins, having variable regions of the desired binding specificity, are recovered from the cell culture. The transfected genes which code for the heavy chain constant and variable regions may be cloned from the same source, usually a cell line, or they may be cloned from different cell sources. In certain preferred embodiments, the genes coding for the variable regions of the heavy and light chains are cloned from the same donor cell line, which cell line typically will already produce an immunoglobulin possessing a desired antigen binding specificity, such as an immortalized cell line producing monoclonal antibodies. The recombinant human immunoglobulins produced by the methods of the invention may be single heavy-light chain dimers, or the dimers may be capable of forming disulfide bonds to other dimers, thereby forming tetramers, or aggregates thereof. In preferred embodiments, the transfected cell may be mammalian, such as human or murine, and may be a lymphocyte, including those selected from the group consisting of lymphoblastoid cells, B cells, and plasmacytomas. Desirably, the recombinant human immunoglobulin produced by the transfected cell will be substantially free of other human proteins.

In another aspect of the invention, a method is provided for switching the class of a human immunoglobulin from a first class to a second class. This method comprises transfecting into a eucaryotic host cell a gene from the donor cell coding for the variable region of the immunoglobulin heavy chain, which gene is operably linked to a gene coding for the constant region of a heavy chain of the desired class or subclass. The host cell is also transfected with a gene coding for the light chain variable region of the immunoglobulin, which gene is operably linked to a gene coding for a light chain constant region. Again, transfections may be performed simultaneously or sequentially. The host cell is then cultivated and recombinant human immunoglobulin having the characteristics of the second class recovered from the medium. The first class may be IgM and the second class may be IgG. The genes coding for the heavy chain variable and constant regions, as well as those coding for the light chain, may be operably linked in expression vectors, such as plasmids or bacteriophage.

In yet another aspect of the invention, compositions of recombinant human immunoglobulin molecules are provided. The immunoglobulins may comprise heavy-light chain pairs, which pairs may be capable of forming disulfide bonds to other pairs, thereby forming tetramers or aggregates thereof. The recombinant immunoglobulin may be comprised of a heavy chain where the variable and constant regions may or may not be coded for by genes cloned from the same donor cell. The variable regions of the heavy and light chains of the composition may be coded for by genes cloned from the same or different donor cell and, further, the constant regions of these chains need not necessarily be cloned from the same donor cell. Desirably, the immunoglobulin composition will be capable of specifically binding to a preselected antigen, will possess effector functions, and may further be combined with pharmaceutically acceptable excipients for therapeutic or prophylactic administration to humans, such as in treating or preventing bacterial infections, as but one example.

The invention also contemplates recombinant human immunoglobulin molecules produced by the process of transfecting a eucaryotic host cell with operably linked genes coding for variable and constant regions of a human heavy chain, and with operably linked genes which code for a human light chain. The resulting transfected host cell is cultured and the recombinant human immunoglobulin recovered from the culture medium. The transfection of the two sets of operably-linked genes may occur simultaneously or sequentially. The operably linked genes coding for the human heavy chain may be cloned from the same or different donor cells, and the genes coding for the variable regions of the heavy and light chains may be cloned from the same or different donor cell line. Although the variable region genes for the heavy and light chains may be obtained from different donor cell lines, in many instances the donor cell source will produce monoclonal antibodies which bind to the same antigen.

In another embodiment, the invention comprises recombinant human immunoglobulins produced by culturing a eucaryotic host cell line transfected with a DNA sequence coding for a human heavy chain and/or a DNA sequence coding for a human light chain, where the host cell line is capable of expressing the transfected sequences. In the instances where only one DNA sequence is used for transfection, the host cell line should be a plasmacytoma producing either a human light or heavy chain which is capable of forming heavy-light chain pairs with the molecule coded for by the transfected sequence.

In yet another embodiment, the invention comprises DNA constructs for expressing recombinant human immunoglobulin light or heavy chains, which constructs comprise a first DNA sequence for a human variable region polypeptide of one of the chains, and a second DNA sequence coding for a constant region polypeptide, which sequence is joined directly or through an intron in reading frame at its 5′ end to the 3′ end of the first DNA sequence. In preferred embodiments, the DNA constructs will have transcriptional and translational initiation and termination regulatory sequences recognized by a transfected eucaryotic host cell, which sequences are in regulatory relationship with the first and second DNA sequences encoding the immunoglobulin molecules.

Also provided are eucaryotic cells producing recombinant human immunoglobulin molecules. The cell lines may contain DNA constructs which comprise sequences that code for human heavy chain variable and constant region polypeptides, wherein the sequence coding for the constant region is joined directly or through an intron in reading frame at its 5′ end to the 3′ end of the sequence coding for the variable region. The cell lines will also have DNA sequences which code for human light chain variable and constant region polypeptides. In certain preferred embodiments, the DNA sequences coding for the heavy chain constant and variable regions are cloned from different donor cell lines, and the DNA sequences coding for the heavy and light chain variable regions are cloned from the same donor cell line.

In a related aspect of the invention, methods are provided for increasing the expression of DNA sequences which code for human heavy or light chain immunoglobulin polypeptides in mammalian lymphocytes. A mammalian lymphocyte line is transfected with a DNA construct which codes for the immunoglobulin polypeptide wherein the construct contains a DNA sequence coding for a functional region of a mammalian heavy chain transcriptional enhancer.

The invention also encompasses DNA constructs for expressing immunoglobulin polypeptides in mammalian cells, wherein the construct contains a cytomegalovirus transcriptional enhancer sequence from the upstream region of the major immediate early genes of cytomegalovirus. The transcriptional enhancer sequence may be selected from the group consisting of a sequence for the enhancer of human, simian, or murine cytomegalovirus. In another aspect, the DNA construct may also contain a DNA sequence coding for a cytomegalovirus promoter sequence. The promoter sequence may be selected from the group consisting of a sequence for the promoter of human cytomegalovirus, simian cytomegalovirus, and murine cytomegalovirus.

An additional aspect discloses methods for enhancing the expression of DNA sequences which code for immunoglobulin polypeptides in a mammalian cell. A DNA construct which codes for the immunoglobulin polypeptide and contains a DNA sequence from the upstream region of the immediate early genes of cytomegalovirus coding for a transcriptional enhancer is inserted into the mammalian cell for expression. The enhancer sequence may be inserted in a DNA construct either upstream of the variable region, in the intron between the variable and constant regions or downstream of the constant region genes. The enhancer sequence may be selected from the group consisting of sequences for the enhancers from human, simian, or murine cytomegaloviruses. In preferred embodiments, the enhancer sequences are derived from the human cytomegalovirus and the immunoglobulin genes are human.

## IV. BRIEF DESCRIPTION OF THE FIGURES

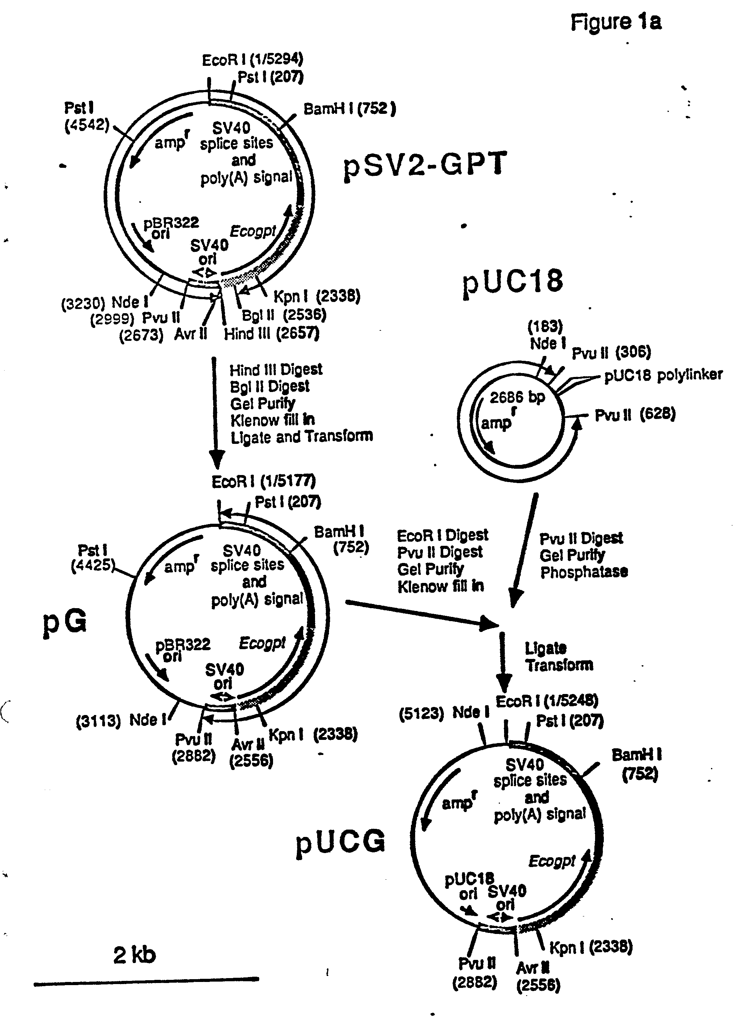

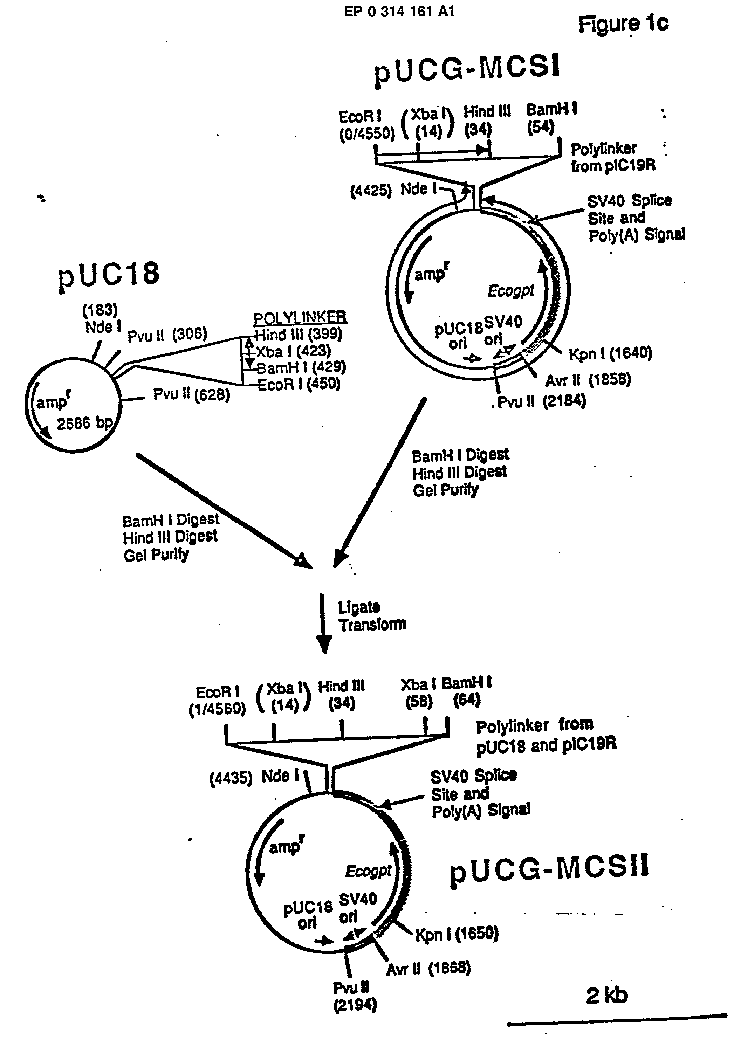

Figure 1a shows the construction of pG from pSV2-gpt and the construction of pUCG from pG and pUC18. Within each construct, the circular portion designates vectors before the addition of immunoglobulin genes. Polylinker regions in each plasmid are expanded, but not all restriction sites in the polylinker are shown. Where full restriction endonuclease names are not used, the following abbreviations apply: E is EcoR I; S is Sac I or Sst 1; Ba is BamH I; Bg is Bg1 II; H is Hind III; and X is Xba I. Amp$^r$ refers to the gene encoding ampicillin resistance and ORI refers to an origin of replication derived from SV40, pUC18 or pBR322, as shown. Ecogpt refers to a gene derived from E. coli conferring resistance to mycophenolic acid in mammalian cells and neo refers to a gene derived from E. coli conferring resistance to neomycin/kanamycin in bacteria and G418 in mammalian cells. The outer arc with arrows shows the fragment used to construct the next plasmid in the construction strategy and inner arcs show direction of transcription. The approximate base pair number is shown next to restriction sites and refers to the 5$'$ most nucleotide in the recognition sequence. Within the plasmid circle, thin black lines are sequences derived from bacterial plasmids and thick black lines are sequences derived from SV40; stipled lines refer to the selectable marker used in mammalian cells; and cross-hatched lines refer to enhancer sequences, if present. These descriptions are also applicable to the Figures which follow.

Figure 1b shows the construction of pUCG-MCSI from pIC19R and pUCG.

Figure 1c shows the construction of pUCG-MCSII from pUC18 and pUCG-MCSI. In pUCG-MCSI and pUCG-MCSII, and Xba I site shown in parentheses is only restricted in dam$^-$ bacteria.

Figure 1d shows the construction of pMHE from pUC18 and pN2JHRI and the construction of pUCG-MCSII-MHE from pMHE and pUCG-MCSII. For pN2JHRI, only the relevant region of the clone is shown. In this Figure and in Figures 1e, 1f, 13, 22 and 24a-c, the inner arc with arrow by the enhancer indicates the orientation of the enhancer in its gene of origin.

Figure 1e shows the construction of pHCMV(e+p) from pUC CMV-IE and pEMBL18 and the construction of pHCMVE from pHCMV(e+p) and pIC19R.

Figure 1f shows the construction of pUCG-MCSII-HCMVE from pUCG-MCSII and pHCMVE.

Figure 2a shows the construction of the vector pN.1 from pSV2-neo.

Figure 2b shows the construction of the vector pN.2 from pN.1, using Not I linkers. The asterisk at the Nde I/Not I site in this Figure and in Figures 11, 12, 17 and 18 indicates that the original Nde I site was not necessarily reconstructed during the process of creating the Not I site.

Figure 3 shows the construction of pγ2 from Phage 5A and pUC18. Wavy lines at the ends of Phage 5A refer to bacteriophage arms, as do the wavy lines in Figures 16, 17, 18, 20a and 22.

Figure 4 shows the construction of pGγ2 from pG and pγ2.

Figure 5 shows the construction of pNγ2 from pN.1 and pγ2. The intermediate, pN(γ2)2, contains two copies of the γ2 BamH I fragment inserted in a head to tail configuration into pN.1.

Figure 6 shows the construction of pγ1.1 from Phage 3A and pUC18. Wavy arms at the end of the phage refer to bacteriophage arms.

Figure 7 shows the construction of pγ1.2 from pγ1.1. pγ1.2 lacks the approximately 4 kb of DNA 5$'$ of the coding region of the Cγ1 gene present in pγ1.1.

Figure 8 shows the construction of pγ1.3 from pγ1.2 and pIC19R by digestion of pγ1.2 with Hind III and BamH I. The asterisk at the Pst I site in this Figure and in Figures 9-12 and 16-18 marks a Pst I site in the Cγ1 gene that is not unique.

Figure 9 shows the construction of pγ1.4 from pγ1.2 and pIC19R by digestion of pγ1.2 with Hind III and Pvu II.

Figure 10 shows the construction of a Cγ1 heavy chain cassette, pNγ1.1, from pγ1.1 and pN.1. The Bgl II site in parentheses was deleted during cloning.

Figure 11 shows the construction of another Cγ1 heavy chain cassette, pNγ1.2, from pγ1.3 and pN.2.

Figure 12 shows the construction of yet other Cγ1 heavy chain cassettes, pNγ1.3 and pNγ1.4, from pγ1.4 and pN.2. pNγ1.3 contains the Cγ1 gene in opposite orientation to the neo gene, while pNγ1.4 contains the Cγ1 gene in the same orientation as the neo gene. Both cassettes are missing the switch region and membrane exon sequences.

Figure 13 shows the construction of pGEMCx from pUCG-MCSII-HCMVE and pCx. Cx is shown as an insert in the EcoR I site of pCx and pGEMCx.

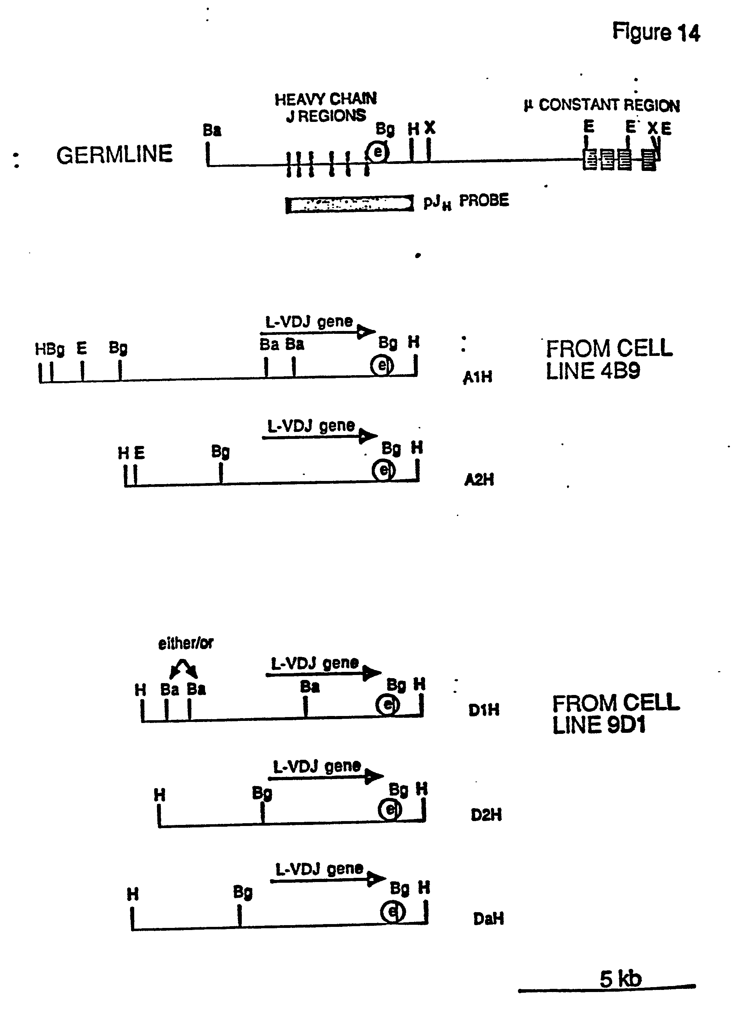

Figure 14 shows maps of the germline heavy chain J region and of the heavy chain variable region clones derived from cell lines 4B9 and 9D1. In the germline map, the J regions and exons of the heavy chain μ constant region are shown as boxes filled with diagonal and horizontal lines, respectively. pJH, the probe used to detect the variable region genes, is shown as a thick solid black line below the J regions. In the maps of the variable region genes derived from cell lines 4B9 and 9D1, the approximate location of the L-VDJ genes is shown as an arrow pointing in the direction of transcription. For clone D1H, "either/or" and two arrows means that only one of the two indicated BamH I sites is present.

Figures 15a and 15b show the structures of pG$\gamma$2-A1H and pG$\gamma$2-A2H, respectively. The $\gamma$2 constant region gene is shown as an insert into the BamH I site of pG; the variable region genes, A1H and A2H, are shown as inserts into the Hind III site of the constant region gene. Arrows above the immunoglobulin genes show the direction of transcription. In Figures 15a and 15b, two arrows indicate the EcoR I sites for restriction of the construct prior to transfection.

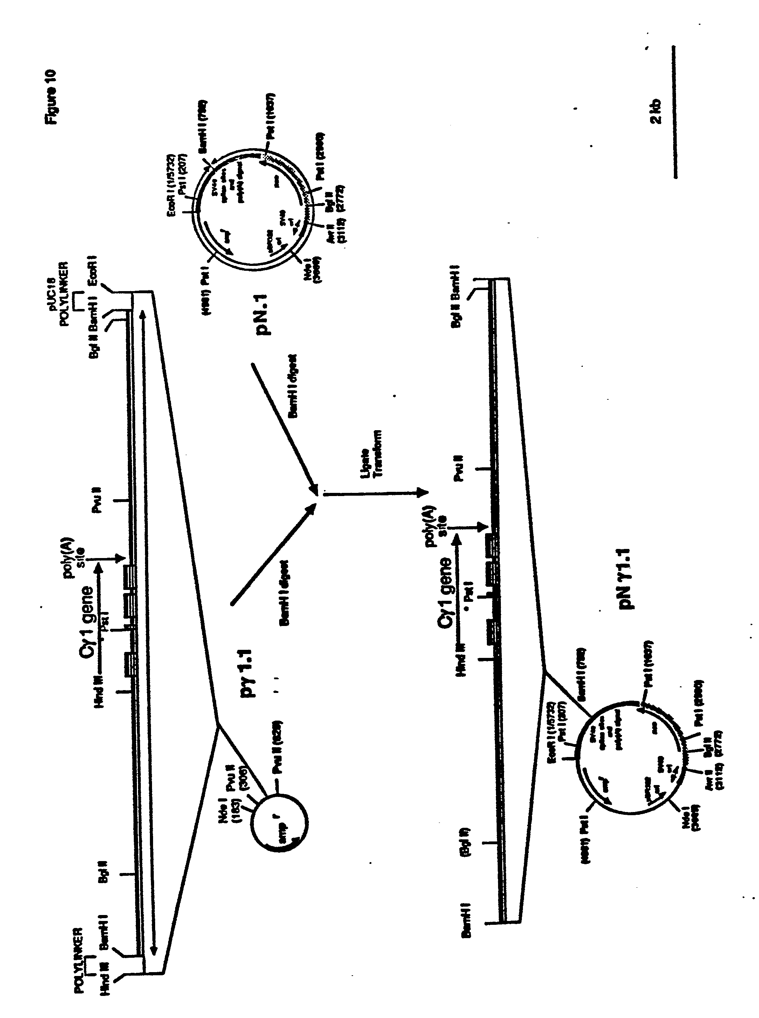

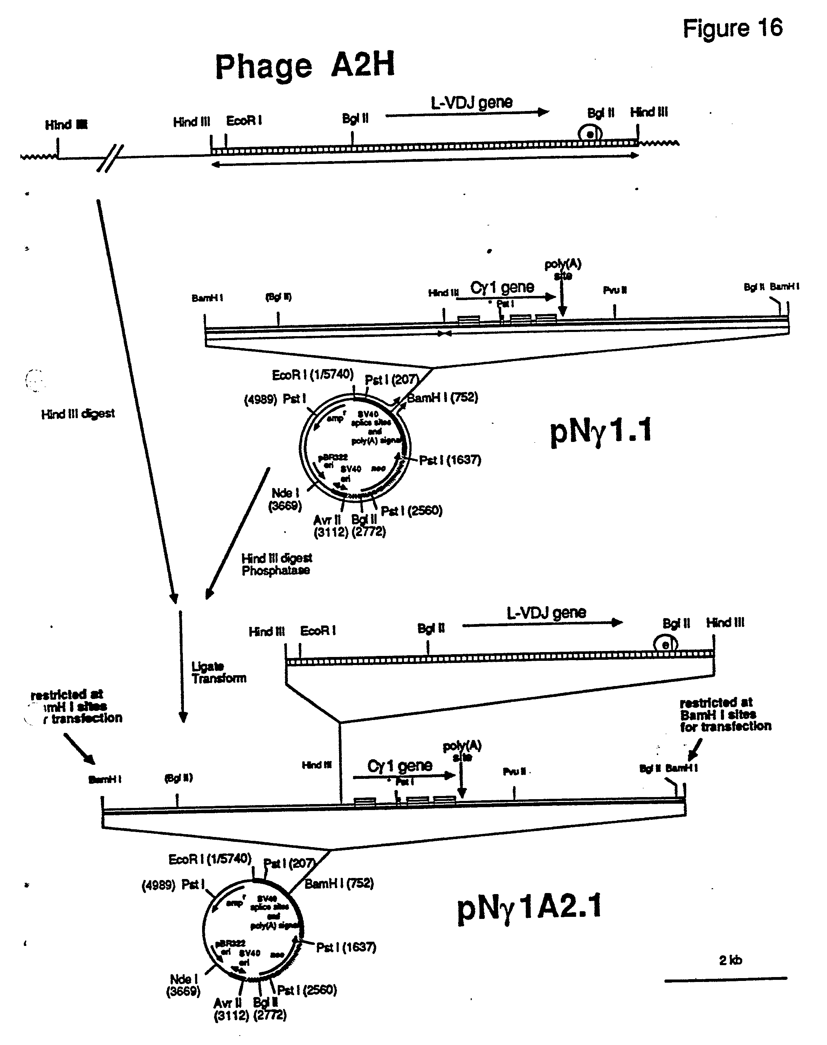

Figure 16 shows the construction of a 4B9/$\gamma$1 heavy chain gene vector or construct from phage A2H, which contains the variable region gene of 4B9, and pN$\gamma$1.1, which contains a C$\gamma$1 gene. The resulting construct was designated pN$\gamma$1A2.1. The $\gamma$1 constant region gene is shown as an insert into the BamH I site of pN$\gamma$1.1 (originally, pN.1, see Figure 10); the variable region gene, A2H, is shown as an insert into the Hind III site of the constant region gene. Arrows above the immunoglobulin genes show the direction of transcription and two arrows pointing toward the pN$\gamma$1A2.1 construct indicate the BamH I sites for restriction of the construct prior to transfection.

Figure 17 shows the construction of another 4B9/$\gamma$1 heavy chain construct, pN$\gamma$1A2.2, from phage A2H and pN$\gamma$1.2. The $\gamma$1 constant region gene is shown as an insert into the BamH I and EcoR I sites of pN$\gamma$1.2 (originally, pN.2, see Figure 11); the variable region gene, A2H, is shown as an insert into the Hind III site of the constant region gene. Arrows above the immunoglobulin genes show the direction of transcription and arrows at pN$\gamma$1A2.2 indicate that the construct can be restricted at EcoR I sites prior to transfection.

Figure 18 shows the construction of still another 4B9/$\gamma$1 heavy chain construct, pN$\gamma$1A2.3, from phage pA2H and pN$\gamma$1.3. The $\gamma$1 constant region gene is shown as an insert into the EcoR I site of pN$\gamma$1.3 (originally pN.2, see Figure 12); the variable region gene, A2H, is shown as an insert into the Hind III site of the constant region gene. Arrows above the immunoglobulin genes show the direction of transcription and an arrow at pN$\gamma$1A2.3 indicates the BamH I site for restriction prior to transfection.

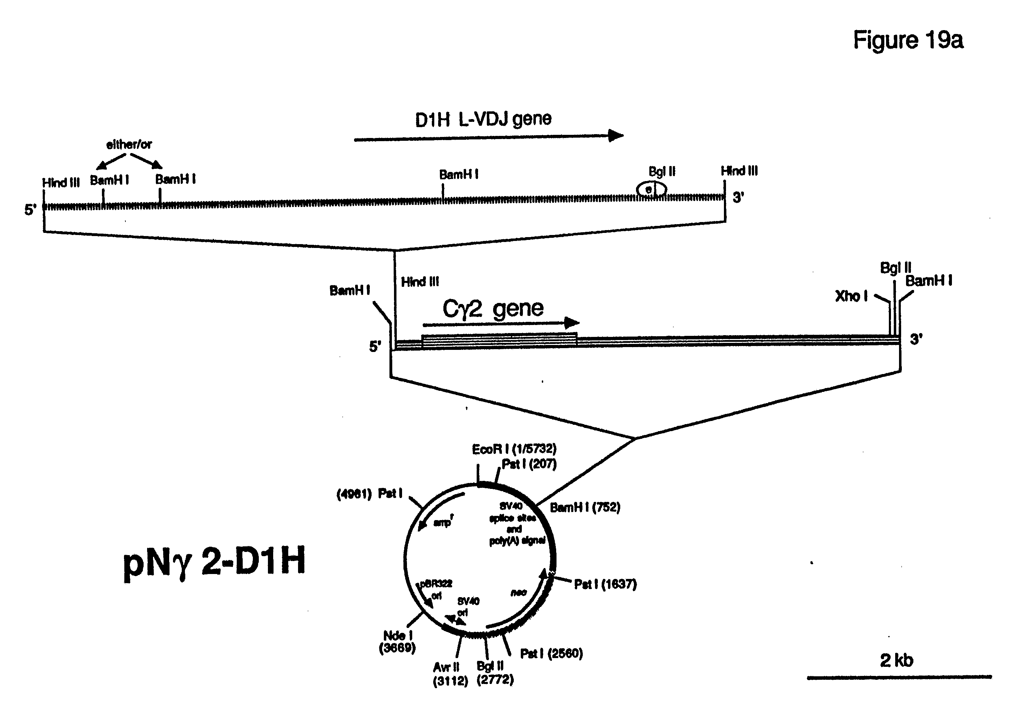

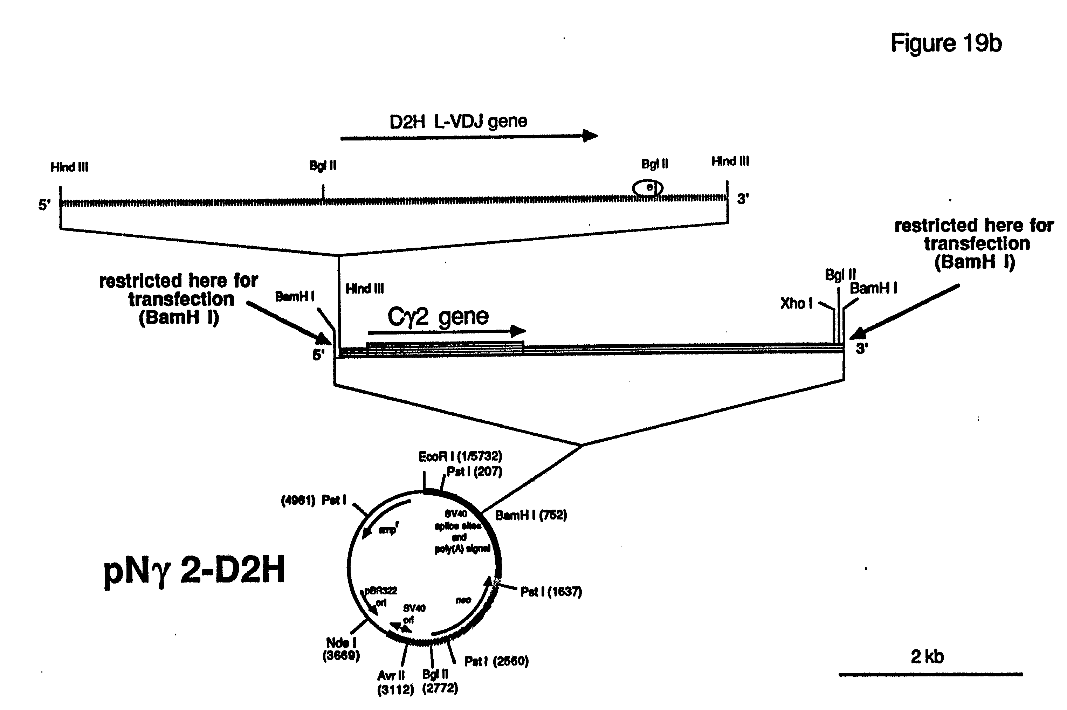

Figures 19a and 19b show the structures of pN$\gamma$2-D1H and pN$\gamma$2-D2H, respectively. The $\gamma$2 constant region gene is shown as an insert into the BamH I site of pN.1; the variable region genes, D1H and D2H, are shown as inserts into the Hind III site of the constant region gene. Arrows above the immunoglobulin genes show the direction of transcription. In Figure 19a, "either/or" and two arrows indicates that only one of the two indicated BamH I sites is present. pN$\gamma$2-D1H was not restricted prior to transfection. In Figure 19b, two arrows indicate the BamH I sites for restriction of the construct prior to transfection.

Figure 20a shows the construction of p$\mu$.2, containing a $\mu$ constant region gene, from Phage $\mu$ and pUC18.

Figure 20b shows the construction of a 4B9/$\mu$ heavy chain gene by a 3-way ligation of p$\mu$.2, pA2H, and pN.2. The construct was designated pN$\mu$A2.1. Arrows above the immunoglobulin genes show the direction of transcription and an arrow at pN$\mu$A2.1 indicates the BamH I site for restriction of the construct prior to transfection.

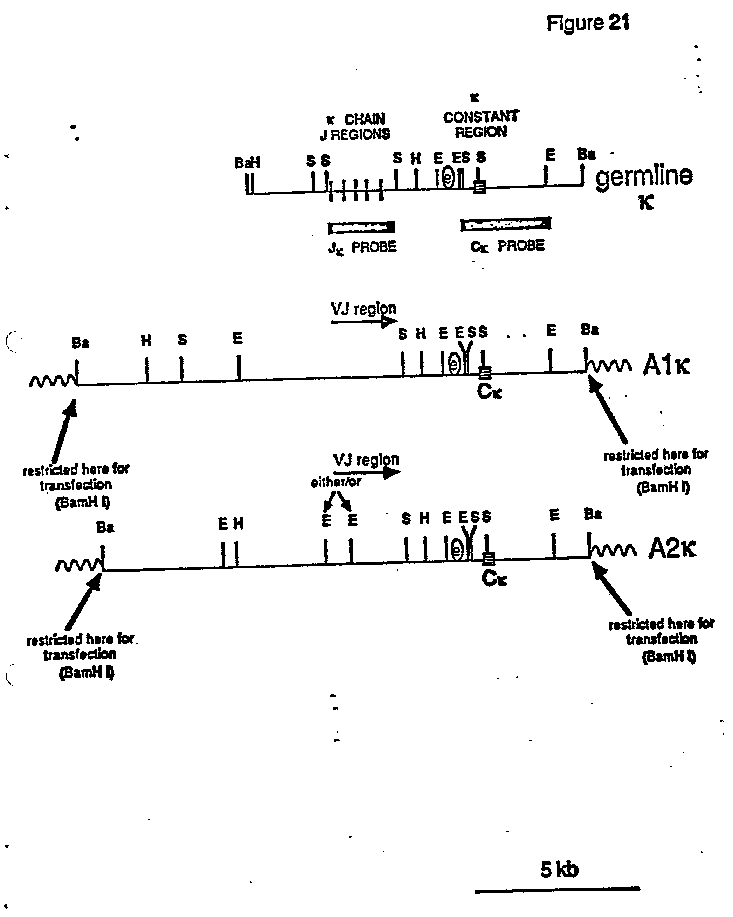

Figure 21 shows maps of the germline $x$ light chain J and C regions and of the $x$ light chain clones derived from cell line 4B9. In the germline map, the J regions are shown as boxes filled with diagonal lines spanning the central line and the constant region is shown as a box filled with horizontal lines. pJ$x$, the probe used to detect the variable region genes, is shown as a thick solid black line below the J regions. pC$x$, the probe used to detect the $x$ constant region, is shown as a thick solid black line below the constant region gene. In the maps of the kappa genes derived from cell line 4B9, the approximate location of the L-VJ genes is shown as an arrow pointing in the direction of transcription. "Either/or" and two arrows indicates that only one of the two indicated EcoR I sites is present. The wavy lines at the ends of the kappa genes indicate bacteriophage vector sequences. Arrows indicate the BamH I sites for restriction of the constructs prior to transfection.

Figure 22 shows the structure of pG$x$A1-HCMVE and of phage A1$x$, from which it is derived. The $x$ constant region gene is shown as an insert into the EcoR I site of pUCG-MCSII-HCMVE; the variable region gene is shown as an insert into the Hind III site of pUCG-MCSII-HCMVE. Arrows above the immunoglobulin genes show the direction of transcription. For Phage A1$x$, two arrows indicate the BamH I sites for restriction of the construct prior to transfection; for pG$x$A1-HCMVE, two arrows indicate the Pvu I sites for restriction of the construct prior to transfection.

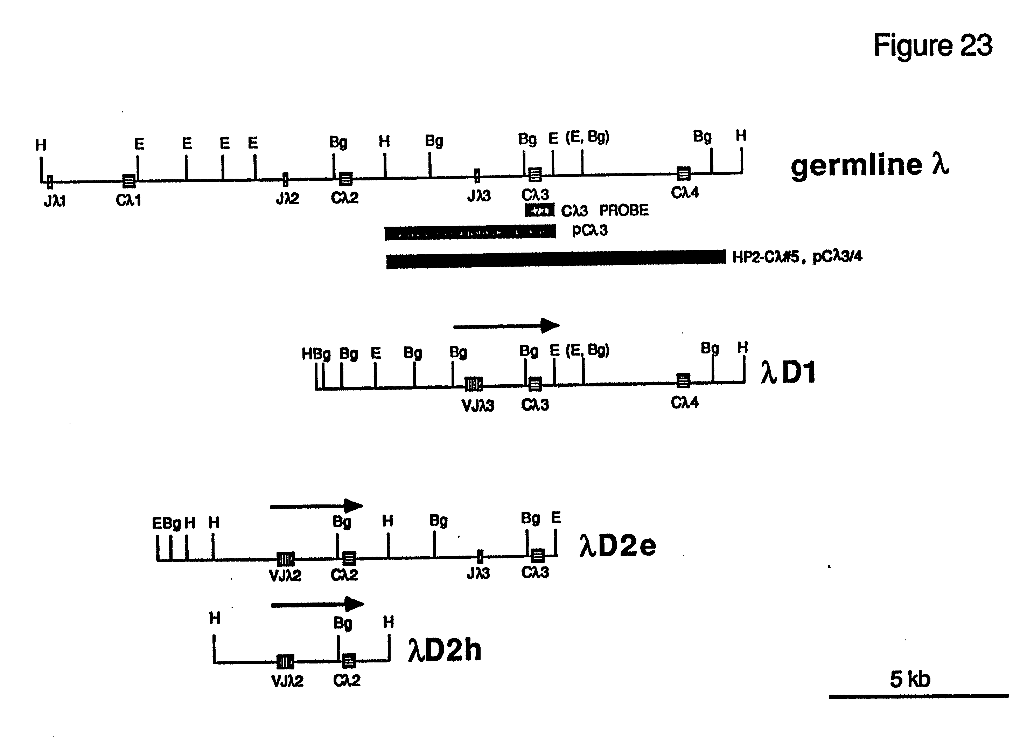

Figure 23 shows maps of the germline $\lambda$ light chain J and C regions and of the $\lambda$ light chain clones derived from cell line 9D1. In the maps, the V region genes are shown as boxes filled with vertical lines, the J regions are shown as boxes containing diagonal lines and the constant region is shown as a box filled with horizontal lines. The regions corresponding to HP2-Cλ#5, pCλ3/4, pCλ3, and the Cλ3 probe are shown as solid black boxes below the germline map. In the maps of the clones derived from cell line 9D1, the direction of transcription is shown by an arrow above the variable and constant region genes.

Figures 24a, 24b, and 24c show the structure of pGλ-D1, pGλ-D2h, and pGλ-D2e, respectively. For pGλD1 and PgλD2h, the immunoglobulin gene is shown as an insert in the Hind III site of pUCG-MCSII-MHE. For pGλ-D2e, the immunoglobulin gene is shown as an insert in the EcoR I site of pUCG-MCSII-MHE. Arrows above the immunoglobulin genes show the direction of transcription. Arrows indicate the sites of restriction prior to transfection: Pvu I for pGλ-D1, EcoR I for pGλ-D2h, and Sal I for pGλ-D2e.

Figure 25 is a histogram showing the range of concentrations of a recombinant IgG1 antibody (in μg/ml) produced according to this invention. The concentrations were determined in an ELISA assay as described herein and represent values from the 40 masterwells producing the most immunoglobulin.

Figure 26 is a histogram showing the range of concentrations of a recombinant IgM produced according to this invention. These concentrations were determined in an ELISA assay as described herein and represent values from the 40 masterwells producing the most immunoglobulin.

Figure 27 depicts the SDS-PAGE (polyacrylamide gel electrophoresis) pattern of various novel immunoglobulins of this invention. Lanes 1 and 2 depict the molecular weights of the heavy and light chains of the parental 4B9 antibody (parIgM) and recombinant 4B9 IgM antibody (rIgM) of this invention, respectively; lanes 3, 4 and 5 depict the molecular weights of the antibody chains of a recombinant IgG1 of the invention; lane 6 depicts a recombinant IgG2 of the invention; lane 7 contains a control IgG1, λ light chain heteromyeloma immunoglobulin; and lane 8 contains molecular weight markers.

Figure 28 shows a comparison of the ability to bind to various Group B Streptococci of the rIgM of the invention (-O-) vs. parIgM (-●-), using a Poly-L-Lysine (PLL)-ELISA method.

Figure 29 depicts the results of an opsonophagocytic assay demonstrating the identical bacteriocidal activities displayed by the rIgM of this invention and ParIgM.

Figure 30 shows in vivo comparative data of the ability of the rIgM of the invention (16/2B) to protect against a Group B Streptococcus infection in a neonatal rat model system.


## V. DESCRIPTION OF THE SPECIFIC EMBODIMENTS


The present invention provides novel recombinant human immunoglobulins and methods for their production using recombinant DNA techniques. Genes encoding human immunoglobulin molecules may be cloned from a cell secreting or capable of secreting an immunoglobulin molecule of a desired antigen binding specificity; these genes may then be transfected into a eucaryotic cell line capable of expressing the immunoglobulin molecules. The transfected host cells, or transfectomas, then secrete functional recombinant human immunoglobulin molecules which possess the desired specificity. By substituting genes which code for constant regions of other isotypes, immunoglobulins of a desired isotype and possessing desired effector functions may be provided. By recombinant is intended those immunoglobulins or portions thereof which are coded for by DNA sequences made through gene cloning techniques. By human is meant immunoglobulins or portions thereof or the genes coding for such molecules which are primarily (at least 80 to 85%) human in origin. The recombinant human immunoglobulins thus produced find use in a wide variety of applications, including prophylaxis or therapy, diagnosis, or as vaccines.

Prior to setting forth the invention in more detail, it may be helpful to an understanding thereof to set forth definitions of certain terms to be used hereinafter.


Expression vector

- A DNA sequence, usually derived from a plasmid or bacteriophage, which contains necessary regulatory signals to drive the transcription of at least one gene carried on the vector and its subsequent translation into its polypeptide product when transfected into a host cell. The expression vector is capable of replicating in bacteria, so that adequate quantities of DNA may be obtained for transfection.


Expression cassette

- A DNA sequence, usually derived from a plasmid or bacteriophage, which has been modified by the addition of at least one element (e.g., enhancer, variable region gene, constant region gene) such that it can serve as an immediate precursor to final constructs encoding immunoglobulin chains.

Operably linked

- DNA sequences joined to produce a continuously transcribable gene sequence which, after RNA splicing, produces sequences in reading frame and can be continuously translated to produce a polypeptide.

Transfection

- The transfer of DNA into eucaryotic cells.

Immunoglobulin

- Assemblies of heavy and light chains into dimers, tetramers, or aggregates thereof, where the assemblies possess specific binding reactivity to an antigenic determinant.

Donor Cell

- Cell which serves as a source of human immunoglobulin genes.

Host Cell

- Cell into which human immunoglobulin genes cloned from donor cells are transfected for ultimate expression.

Generally, in one preferred embodiment, the method for producing recombinant human immunoglobulins comprises the following steps:

(1) construction of an expression cassette containing a human immunoglobulin heavy chain constant region ($C_H$) of the desired isotype;

(2) isolation of genomic DNA encoding the expressed variable region of a human immunoglobulin heavy chain ($V_H$) from a donor cell line capable of secreting the immunoglobulin and inserting the $V_H$ gene in the cassette of step 1 at a location proximal to the 5' end of the $C_H$ segment and in the same orientation;

(3) preparation of a vector containing genomic DNA encoding the expressed kappa or lambda light chain isolated from the donor cell line of step 2;

(4) transfection of the cloned human heavy chain and light chain immunoglobulin genes into a eucaryotic host cell line capable of supporting the expression and secretion of human immunoglobulin molecules; and

(5) identification and isolation of transfected cells secreting desired human immunoglobulin molecules, and culturing the cells for the production thereof.

The invention employs cell lines which produce or are capable of producing human immunoglobulins as a source of genes to code for the immunoglobulin or fragment thereof which is desired to be secreted by a host cell line. Such donor cells will serve as a source of at least the variable region genes of both chains, $V_H$ and/or $V_L$, which genes may be cloned into expression cassettes, which may already contain a human constant region gene ($C_H$ or for example, $C_L$ respectively), obviating the necessity of cloning the constant region gene(s) of the donor cell. Alternatively, genes encoding the variable region of the heavy chain ($V_H$) and the entire light chain (kappa or lambda) may be cloned from the donor cell. The donor cell may also provide genes encoding the entire heavy chain.

Cell lines which secrete human monoclonal antibodies may conveniently serve as a source of human immunoglobulin genes. These cell lines may be produced using conventional hybridoma or transformation technology, such as that described in Methods of Hybridoma Formation, Bartal and Hirshaut (eds.), Humana Press, Clifton, N.H., 1985, or U.S. Patent No. 4,464,465, both of which are incorporated herein by reference.

Other cells which may serve as a source of human immunoglobulin genes are available from a number of depositories, including the American Type Culture Collection (ATCC) of Rockville, MD. The scientific literature contains several descriptions of cell lines secreting human immunoglobulins to specific antigenic targets, such as those reviewed in Carson et al., 1986, Adv. Immunol., 38:275 and James et al., 1987, J. Immunol. Meth., 100:5, both of which are incorporated herein by reference.

The genes encoding the human immunoglobulins or domains thereof which are cloned from the donor cell lines may be transferred into an expression vector prior to transfection into a host cell. A human $V_H$ region gene may be cloned into a cassette already containing a human $C_H$ region gene, such that a complete human heavy chain is encoded and will be expressed by a recipient host cell. The human light chain genes, containing L, V, J, and C region genes, may be cloned in their entirety and used to co-transfect a host cell with the heavy chain constructs for ultimate expression and secretion of a complete immunoglobulin molecule with the desired characteristics. Alternatively, as with the heavy chain genes, a human $V_L$ gene may be cloned into a cassette already containing a human $C_L$ gene, which construct is then used to transfect a host cell for expression.

As referred to herein, for both the heavy and light chain genes the L gene codes for a hydrophobic leader sequence of about 17 to 20 amino acids which is thought to be important in transporting an immunoglobulin through the endoplasmic reticulum, during which process the leader sequence is cleaved away from the immunoglobulin chain. The V gene specifies most of the variable region, the remainder being coded for by the J or joining gene. The DNA coding for the variable region of heavy chains contains an additional gene, the D (diversity) gene, which codes for a short segment between the V and J segments.

The general structure of immunoglobulin heavy chain genes is known. See Joho et al., 1983, Current Topics in Development Biology, 18:15-58; and Calame, 1985, Ann. Rev. Immunol., 3:159-195, which are incorporated herein by reference. Genes coding for a functional heavy chain consist of a rearranged variable region gene separated by an intron from one of any of the constant region genes, $\mu$, $\gamma1$, $\gamma2$, $\gamma3$, $\gamma4$, $\epsilon$, $\alpha1$ or $\alpha2$. In a genetically engineered heavy chain, the variable and constant region genes may be derived from the same or different donor cell line and then spliced together to form a functional immunoglobulin chain. Typically, the heavy chain variable region gene will be derived from a donor B cell line producing a monoclonal antibody of a desired antigen binding specificity, or is capable of producing said antibody having undergone VDJ and VJ recombination.

In the present invention it is desirable to construct cassettes consisting of heavy chain constant region genes in DNA vectors such that a variable region gene can be inserted 5′ of the constant region gene to produce an expression vector containing a complete expressible heavy chain gene. This may be accomplished by inserting the heavy chain constant region gene in the vector along with a short polylinker 5′ of the constant region gene. The variable region gene may then be inserted in the polylinker region. Polylinkers, or regions of multiple cloning sites (MCS), provide sites for desired restriction endonucleases and are well known by those skilled in the art. Expression cassettes may be prepared for the expression of the $\mu$, $\gamma1$, $\gamma2$, $\gamma3$, $\gamma4$, $\epsilon$, $\alpha1$, $\alpha2$, and $\delta$ heavy chain constant regions, into which the desired variable region genes may be cloned in an enzyme site of the polylinker region.

A vector for the construction of an expression cassette may have the following useful properties: 1) a bacterial origin of replication, such as that found in pBR322, to allow propagation as a plasmid, in E. coli, for example; 2) a gene which allows for phenotypic selection in bacteria, such as $\beta$-lactamase; 3) an origin of replication which allows for propagation in mammalian cells; and 4) a gene which allows for phenotypic selection of its uptake in and expression by mammalian cells, such as the Ecogpt genes. In addition, restriction enzyme sites should be provided for the convenient insertion of foreign genes. Preferred vectors include the pSV2-gpt vector described by Mulligan and Berg, 1981, Science, 209:1422-1427, which has a pBR322 origin of replication, a gene encoding $\beta$-lactamase, an SV40 origin of replication which allows it to replicate in mammalian cells, and the Ecogpt gene. Another preferred vector is pSV2-neo, described in Southern and Berg, 1982, J. Mol. Appl. Genet., 1:327-341, which is similar to the pSV2-gp vector except that, in place of the Ecogpt gene, it includes a neomycin-kanamycin resistance gene, which provides for selection in bacterial and mammalian cells. Other vectors, including bovine papilloma virus vectors, polyoma virus vectors, and recombinant retroviruses, have emerged as suitable vectors for stably transferring genes into a variety of cells and are described in Coffin, 1985, RNA Tumor Viruses, Weiss et al. (eds.), Cold Spring Harbor, N.Y., Vol. 2, pp. 36-63, and Kwok et al., 1986, Proc. Natl. Acad. Sci. USA, 83:4552-4552.

In constructing a cassette for expression of human immunoglobulin heavy chain genes it may be necessary to modify the vector so that it does not contain restriction enzyme sites which interfere with later cloning steps. The modification may also be designed to direct higher levels of expression of the gene encoding the selectable marker, thereby facilitating the selection of cells taking up the vector. Other modifications of the vector may be made to increase the yield of plasmid DNA. For instance, plasmid pUC18 replicates to a significantly higher copy number in bacteria than does plasmid pBR322, which has been attributed to differences in their respective origins of replication. Therefore, the pBR322 sequences of a vector may conveniently be replaced with the analogous sequences from pUC18.

In some instances, depending on the vector in which the $C_H$ gene is inserted, it may be convenient to first subclone the $C_H$ gene into a shuttle or intermediate vector, such as pUC18, in an orientation such that the 5′ end is adjacent to a polylinker region, which vector may then be digested with an appropriate restriction endonuclease to release the $C_H$ gene with the polylinker at the 5′ end. The isolated fragment may then be cloned into an expression vector to form a convenient cassette for inserting the desired variable region gene in the polylinker region.

Clones of $C_H$ genes of the various human isotypes have been described. Cloning of the germline human γ1 gene is reported in Flanagan and Rabbitts, 1982, Nature, 300:709-713 and Ellison et al., 1982, Nucleic Acids Research, 10:4071;4079; the γ2 gene is described in Flanagan and Rabbitts, supra, Ellison and Hood, 1982, Proc. Natl. Acad. Sci. USA, 79:1984-1988, and Takahashi et al., 1982, Cell, 29:671-679; the γ3 gene is described in Flanagan and Rabbitts, supra; the γ4 gene is described in Flanagan and Rabbitts, supra, Ellison and Hood, supra, and Ellison et al., 1981, DNA, 1:11-18; the μ gene is described in Ravetch et al., 1981, Cell, 27:583-591; and the ε, α1 and α2 genes are described in Flanagan and Rabbits, supra. All of the aforementioned publications are incorporated herein by reference.

In many instances the constant region of the immunoglobulin molecule in the donor cell line will be replaced by a human constant region from another source. The new C region may be of the same or a different isotype, thereby providing a mechanism for switching the isotype of the original antibody to that more suitable for a particular purpose. For instance, IgG molecules are often felt to be preferred over IgM molecules for therapeutic administration to humans, where IgG has effector functions which allow it to more easily cross the placenta or to participate in antibody-dependent cellular cytotoxicity, IgG has a longer half life in vivo than IgM, and is often preferred for manufacturing purposes, as methods for IgG purification are well known while those for IgM are not. Also IgM is often unstable in vitro. See generally, Shulman, 1987, New Frontiers in the Study of Gene Functions, Poste and Crooke (eds.), Plenum Press, New York, p. 139-158, which is incorporated herein by reference.

To obtain higher levels of expression of human immunoglobulin genes, transcriptional enhancer sequences may be inserted in the expression vehicle. Enhancers are cis-acting elements of DNA that stimulate transcription of adjacent genes by RNA polymerase II. Enhancers are active in either orientation and over distances up to several kilobase pairs, even from a position downstream of the transcribed region. Sequence analysis of several enhancers has shown that they are composed of single or multiple copies of one or more conserved "motifs", relatively short sequences of nucleotides separated by less conserved or non-conserved sequences. It is believed that trans-acting factors bind to these motifs and thereby increase levels of transcription, although the present invention is not intended to be bound by any such theories as to mechanism of action. Different trans-acting factors are believed to bind to different motif sequences. Enhancers may be conveniently inserted into the MCS region, where the immunoglobulin gene of interest may be inserted either 5′ or 3′ of the enhancer. Alternatively, an enhancer may be inserted with an intron of an immunoglobulin gene.

One aspect of the invention involves the use of transcriptional enhancers obtained from murine or human immunoglobulin heavy chain gene sequences, which enhancers increase the expression of DNA sequences coding for human immunoglobulin light chain polypeptides in mammalian cells. The murine or heavy chain enhancer sequences are inserted into DNA constructs which code for the human light chain, which may be either a kappa or lambda chain. Immunoglobulin transcriptional elements are more fully described in Calame, 1985, Ann. Rev. Immunol., 3:159-195, which is incorporated herein by reference.

Another aspect of the present invention contemplates DNA constructs for expressing immunoglobulin polypeptides in mammalian cells and methods for expressing said sequences, wherein the construct contains a cytomegalovirus transcriptional enhancer sequence from the upstream region of the major immediate early genes of a cytomegalovirus which may be selected from the group consisting of human, simian and murine cytomegalovirus. In a preferred embodiment, the DNA construct additionally contains a cytomegalovirus promoter sequence, which itself may be selected from promoters of human, simian and murine cytomegaloviruses, and need not necessarily be from the same species as the enhancer sequence. In another preferred embodiment, the enhancer sequence is inserted in the DNA construct between sequences coding for the constant region and sequences coding for the rearranged variable region. The immunoglobulin sequences may code for a kappa or lambda light chain, or heavy chains of the α, γ, δ, ε, and μ classes and subclasses thereof.

As used herein, a cytomegalovirus enhancer sequence is intended to include any fragment of DNA which contains sequences having at least 40% homology (percentage of similarity of sequences), preferably at least 60% homology, more preferably at least 80% or more homology to the entire cytomegalovirus enhancer sequence, or which contains sequences having at least that homology to any of the enhancer motifs which are found in cytomegalovirus, as listed in Table I. One or more motifs may be repeated one or more times within the sequence of any one enhancer element. Such motifs are identified by sequences which are found within the human, simian or murine cytomegalovirus immediate early enhancer region, but are not present in the known immunoglobulin enhancers or SV40 enhancer at a stringency of at least that listed in Table I. The known immunoglobulin enhancer regions include the human heavy chain enhancer, bp 1 to 583 to Rabbitts et al., 1983, Nature, 306:806-809; the human kappa light chain enhancer, bp 1 to 199, according to Emorine et al., 1983, Nature 304:447-449; the murine heavy chain enhancer, bp 1 to 997, of Ephrussi et al., 1985, Science, 227:134-140; the murine kappa light chain enhancer, bp 1 to 479, of Picard et al., 1984, Nature, 307:80-82; and the SV40 enhancer region, bp 1 to 294, according to the New England Biolabs catalog no. (1986/87), where bp 1 is the last guanosine nucleotide in the Bgl I site. For the purpose of defining cytomegalovirus enhancer motifs, the human cytomegalovirus enhancer is the region bp -737 to bp +193, as defined by Boshart et al., 1985, Cell, 41:521-530; the murine cytomegalovirus enhancer is the region bp -835 to bp +50, according to Dorsch-Hosler et al., 1985, Proc. Natl. Acad. Sci. USA, 82:8325-8329; and the simian cytomegalovirus enhancer is the region from bp -1260 to bp +661, as defined by Jeang et al., 1987, J. Virol., 61:1559-1570, all of which are incorporated herein by reference.

TABLE I. Cytomegalovirus Transcriptional Enhancer Motifs
from the Immediate Early Enhancer Region of Human,
Murine and Simian Cytomegaloviruses

| MOTIF | | SEQUENCE | STRINGENCY | NUMBER OF OCCURRENCES | | |
|---|---|---|---|---|---|---|
| | | | | HUMAN | MURINE | SIMIAN |
| A | a) | CCATTGACGTCAA | 10/13=76% | 5 | 6 | 11 |
| | b) | reverse complement | | 6 | 3 | 13 |
| B | a) | TTGGCAGTACATC | 12/13=92% | 3 | 0 | 1 |
| | b) | reverse complement | | 0 | 0 | 0 |
| C | a) | TGCCCAGTACATA | 12/13=92% | 1 | 5 | 0 |
| | b) | reverse complement | | 0 | 0 | 0 |
| D | a) | ACGGTAAATGGCCCGCCTGGC | 15/21=71% | 3 | 0 | 3 |
| | b) | reverse complement | | 0 | 0 | 0 |
| E | a) | CTAACGGGACTTTCCAA | 14/17=82% | 3 | 0 | 0 |
| | b) | reverse complement | | 0 | 0 | 0 |
| F | a) | TCAATAGGGACTTTCCA | 13/17=76% | 1 | 6 | 0 |
| | b) | reverse complement | | 0 | 2 | 0 |
| G | a) | TCAATAGGGGTGAATCA | 12/17=70% | 1 | 3 | 0 |
| | b) | reverse complement | | 0 | 0 | 0 |
| H | a) | TCAATAGGGGGTGAATCA | 13/18=72% | 0 | 4 | 0 |
| | b) | reverse complement | | 1 | 0 | 1 |
| I | a) | ATGGGTTTTGCC | 10/12=83% | 0 | 7 | 1 |
| | b) | reverse complement | | 0 | 1 | 0 |
| J | a) | AATGACGTATGT | 10/12=83% | 3 | 0 | 0 |
| | b) | reverse complement | | 0 | 0 | 0 |
| K | a) | CAAATGGGCGGT | 10/12=83% | 1 | 0 | 5 |
| | b) | reverse complement | | 1 | 0 | 0 |
| L | a) | CCATATATGGGGTTTCCTA | 14/19=73% | 1 | 0 | 3 |
| | b) | reverse complement | | 0 | 0 | 0 |
| M | a) | GGCACGTACATA | 10/12=83% | 0 | 2 | 1 |
| | b) | reverse complement | | 0 | 0 | 0 |
| N | a) | TATTGGCACGTATAC | 12/15=80% | 0 | 3 | 1 |
| | b) | reverse complement | | 0 | 0 | 0 |
| O | a) | AATCAATATTGGCCATT | 13/17=76% | 3 | 0 | 0 |
| | b) | reverse complement | | 0 | 0 | 0 |
| P | a) | TATTGACTCAT | 10/11=90% | 1 | 0 | 1 |
| | b) | reverse complement | | 0 | 3 | 0 |
| Q | a) | ACTATTGGCACGT | 11/13=84% | 0 | 2 | 1 |
| | b) | reverse complement | | 1 | 0 | 0 |
| R | a) | CTCCTATTGACGTA | 11/14=78% | 2 | 0 | 8 |
| | b) | reverse complement | | 3 | 9 | 3 |
| S | a) | GCCTATAGAATCTATAGGTG | 14/20=70% | 1 | 0 | 2 |
| | b) | reverse complement | | 1 | 0 | 1 |

To secrete an intact human immunoglobulin molecule capable of binding to an antigen, both the light and heavy chain genes must be transcribed, translated, appropriately modified and the resulting polypeptides assembled together in the HL dimer or $H_2L_2$ tetramer structure (where H represents the heavy chain and L represents the light chain). Furthermore, for IgM and IgA, secretion typically involves polymerization of the $H_2L_2$ tetramers and addition of a J chain.

The heavy chain and light chain polypeptides are each obtained by translation of mRNA derived from a rearranged variable region gene located 5′ of a constant region gene, as explained below. The rearranged variable genes are most conveniently obtained from either genomic or cDNA cloning from the cell line producing the immunoglobulin of interest, also known as the donor cell.

The general structure of the heavy chain genes is described in Ellison and Hood, 1983, Adv. Human Genet., 13:113-147. A complete heavy chain variable region gene is composed of $L-V_H$, $D_H$ and $J_H$ elements. In the germline configuration there are loci containing multiple $L-V_H$, $D_H$ and $J_H$ elements, however, in a cell line expressing or capable of expressing the heavy chain, single $L-V_H$, $D_H$ and $J_H$ elements are recombined such that intervening elements are removed. This unit is then known as a VDJ gene or a rearranged heavy chain variable region gene. Although this rearrangement is required for the expression of the variable region, many rearrangements result in genes which are not expressed at the protein level. Either one or both heavy chain alleles can undergo this VDJ rearrangement; if both are rearranged, either one or both can be expressed as mRNA, but only one of the RNA species is translated into a complete heavy chain polypeptide (containing variable and constant region domains). This phenomenon is known as allelic exclusion. Because the expressed allele cannot be conveniently determined at the DNA level and may or may not be determined at the RNA level, it may be necessary to clone both rearranged alleles and then to determine which one is expressed by transfection into cells capable of immunoglobulin expression.

To accomplish this, genomic DNA is restricted with an appropriate endonuclease, DNA fragments of the desired size (determined by Southern transfer and hybridization with a $J_H$ region probe) are enriched for by preparative sucrose gradient centrifugation and used to make a bacteriophage library, which is then screened with a $J_H$ region probe and positive plaques identified, purified and amplified. To facilitate restriction endonuclease site mapping and subcloning into the $C_H$ cassette, the rearranged heavy chain variable region genes (VDJ genes) may be subcloned into pUC18, for example. Plasmid DNA containing the rearranged variable region genes is identified by hybridization with a $J_H$ probe and by the presence of a conserved 0.8 kb Hind III-Bgl II fragment 3' of the J regions. The variable region gene is then transferred into the desired $C_H$ cassette at a position 5' of the $C_H$ gene by restriction of each component with endonucleases producing compatible ends and then ligation of the two components. Bacteria are transformed with the ligation mixture and transformants containing the complete heavy chain gene in proper orientation may be identified using restriction enzyme digestion. Once the entire heavy chain gene has been assembled, it may be linearized and then transfected into a suitable host cell for co-expression with a light chain gene.

There are two classes of human light chains: kappa and lambda. Only a single human light chain, either kappa or lambda, is expressed at the protein level in any particular cell. That light chain combines with the heavy chain to form a HL dimer or a tetramer of the structure $H_2L_2$. The kappa and lambda genes are encoded on different chromosomes and the genes are not believed to normally interact with each other. The general structure of kappa and lambda light chain genes is similar, although somewhat simpler, than the structure of the human heavy chain genes. The complete light chain gene is composed of variable and constant region genes which are separated from each other by an intron. As with the heavy chain, the variable region is actually composed of a few segments which are separated in the germline but are recombined to form a functional light chain variable region gene. The compositions of the kappa and lambda loci differ significantly from each other and are described separately.

The structure of human kappa light chain genes is described in Hieter et al., 1980, Cell, 22:197-207, Hieter et al., 1982, J. Biol. Chem., 257:1516-1522 and in Klobeck et al., 1984, Nucleic Acids Research, 12:6995-7006, all of which are incorporated herein by reference. As shown in Figure 21, in the germline configuration there is a single kappa constant region gene per haploid genome. Upstream of the constant region is a group of 5 Jx region genes; the variable region genes are presumably located an unknown distance upstream of the Jx regions. In a cell line expressing or capable of expressing the kappa light chain, a single L-Vx and Jx element are recombined such that intervening elements are removed. This unit is then known as a VJx gene or a rearranged kappa variable region gene. This rearrangement may occur on one or both alleles. To clone the rearranged x variable region genes, a probe may be used, such as pJx which encompasses the germline Jx genes. With certain restriction enzymes which do not have recognition sequences between the J region and constant region (e.g., BamH I), the Cx probe can also be used to detect rearranged kappa variable region genes.

The structure of human lambda light chain genes is described in Hieter et al., 1981, Nature, 294:536-540 and in Udey and Blomberg, 1987, Immunogenet., 25:63-70, both of which are incorporated herein by reference. In the germline configuration there are at least six lambda constant region genes per haploid genome; however, only Cλ1 through Cλ3 are known to be functional. Each of the lambda constant region genes 1 through 3 has a single Jλ gene 5' of it; the location of the lambda variable region genes is unknown. In a cell line expressing of capable of expressing the lambda light chain, a single L-Vλ and Jλ element are recombined such that intervening elements are removed. This unit is then known as VJλ gene or a rearranged lambda variable region gene. This rearrangement may occur on one or both alleles. To clone the rearranged λ genes, one can take advantage of the close proximity of Jλ and Cλ genes; a Cλ probe can be used to detect rearrangements occuring 5' of it at the respective Jλ locus.

In kappa chain producing cells, one or both kappa alleles may be rearranged, but the lambda locus is rarely or never rearranged. In lambda chain producing cells, one or both lambda alleles may be rearranged and both kappa alleles are usually rearranged, although in a non-productive fashion such that a complete kappa chain cannot be expressed. As with the heavy chains, one cannot determine at the DNA level and may not be able to determine at the RNA level which rearranged light chain is expressed at the protein level; therefore either both rearranged kappa or both rearranged lambda genes need to be cloned from kappa and lambda producing cells, respectively, and the functional gene determined by transfection experiments. As explained in more detail below, the human light chain genes may be cloned using a methodology similar to that used for the human heavy chain genes.

Genomic DNA obtained from a donor cell of interest is restricted with an appropriate endonuclease, DNA fragments of the desired size (determined by Southern transfer using an appropriate probe such as Jx, Cx, or Cλ) are enriched for by preparative sucrose gradient centrifugation and used to make a bacteriophage library; this library is then screened with the appropriate probe and positive plaques identified, purified and amplified. Bacteriophage DNA is then isolated from the plaques.

If the rearranged light chain variable region and constant region genes are found on a single restriction fragment small enough to be cloned into a single vector, they may be cloned together and need not be subcloned into an expression cassette before being transfected into host cells. In other instances, the variable and constant region genes may not fit into one vector and the variable region gene will require subcloning into an expression cassette containing the constant region gene. In still other instances, it will be possible to clone variable and constant region genes on a single vector, but it may be desirable to transfer the variable and constant region genes, either simultaneously or sequentially, into a cassette containing other components which will enhance the level of expression.

To facilitate restriction endonuclease site mapping and subcloning into an expression vector or cassette, the rearranged light chain variable region genes (VJ elements) may be subcloned, for example, into pUC18. Plasmid DNA containing the rearranged variable region genes is identified by hybridization with the appropriate J or C region probe. The variable region gene insert is then transferred into the desired expression vector by restriction of vector and insert with endonucleases producing compatible ends and then ligation of the two components. Bacteria may be transformed with the ligation mixture and transformants containing the complete light chain genes (variable and constant region) in proper orientation may be identified using restriction enzyme digestion. Once the genes coding for the entire light chain have been assembled in an expression vector (either bacteriophage or plasmid), the vector may be linearized and transfected, either simultaneously or sequentially, with genes coding for a human heavy chain into a suitable eucaryotic host cell capable of expressing the genes.

It should be appreciated that in genetically engineered kappa or lambda light chain genes, the variable and constant region genes may be derived from either the same source or from different sources and then spliced together to form a functional gene. In a preferred embodiment, the variable region gene will be derived from a B cell line producing an antibody of desired specificity, while the constant region gene is derived from either a B cell or non-B cell source. It should also be recognized that expression cassettes containing the kappa or lambda constant region genes may be constructed in a manner similar to that described for the heavy chain genes. Preferably, this cassette would utilize a different selectable marker than the heavy chain cassette, permitting selection for uptake of DNA containing both the heavy and light chain genes. In addition, the cassette might also contain enhancers and/or promoters to direct higher levels of expression of the immunoglobulin genes. Alternatively, the cassette might contain the gene control regions such as promoters, enhancers, etc. but not a light chain constant region gene. The variable and constant region genes would then be conveniently added to the cassette as a single unit.

In another aspect of the invention, it is contemplated that the donor cells will supply human variable region genes, which genes may be used with portions of human constant regions to produce recombinant immunoglobulin fragments, such as, for example, Fab, F(ab')₂. An Fab fragment could be produced using recombinant techniques, where the human light chain would be cloned as described above, and the human heavy chain terminated prematurely by inserting stop codons, such as between amino acid residues 221 and 226 of the human γ1 chain (number according to Eu, Edelman et al., 1969, Proc. Natl. Acad. Sci. USA, 63:78-85). This may be accomplished by introducing a translation stop codon in reading frame prior to or replacing the cysteine at residue 226. For example, using site-directed mutagenesis, an adenosine residue could be inserted between the T and G residues of the cysteine triplet TGC, thus producing the stop codon

14

TAG and destroying the cysteine codon. This approach has the advantage of minimally altering the RNA structure, so normal RNA splicing and stability would be preserved. Fab fragments utilizing the $C_HI$ domain of other subclasses of IgG could also be prepared. Unlike IgG1, the other subclasses utilize cysteine residue 131 for disulfide bonding to the light chain. The first cysteine residues involved with inter-heavy chain disulfide bonding occur at residues 221, 226, and 226 for IgG2, IgG3, and IgG4, respectively. Using site-directed mutagenesis, stop codons could be inserted anywhere following residue 215 (end of $C_{HI}$) and the first cysteine residue and most conveniently by replacing the cysteine codon with a stop codon, as described above. F(ab')₂ fragments could be produced using similar techniques to insert stop codons following the cysteine residues responsible for inter-heavy chain disulfide bonding. In a related aspect of the invention, alterations or substitutions in these heavy chain constant region domains (other than $C_HI$) may be made at the DNA level where a second protein or portion thereof is encoded. The second protein should not interfere with the antigen binding activity of the variable region or the assembly of the resulting Fab-, F-(ab')₂-, immunoglobulin-second protein or portions thereof. See PCT patent publication no. WO 86/01533, which is incorporated herein by reference.

While certain substitutions or alterations may be introduced in the L-VJ or L-VDJ and $C_HI$ or $C_L$ sequences, the resulting Fab portion should preferably remain at least 80% human in origin, more preferably 90% or more human.

The DNA sequences coding for the light and heavy chain genes prepared as described above may be inserted into a host cell for ultimate expression. The host cell is a eucaryotic cell capable of expressing the human immunoglobulin genes in a functional form. Yeast cells may serve as hosts, in that they are capable of carrying out post-translational modifications, such as glycosylation, and can secrete polypeptides bearing leader sequences. More preferred hosts are mammalian cells, which may be grown in vitro or in vivo. Mammalian cells are also capable of providing post-translational modifications to immunoglobulin proteins, including leader sequence removal, correct folding and assembly of both heavy and light chains, glycosylation at correct sites, and secretion of functional antibody from the cell. Examples of mammalian cells obtainable from the American Type Culture Collection in Rockville, MD, which may be used for the production of immunoglobulins of the invention include fibroblast cell lines ATCC Nos. CCL 92, CRL 1569, CRL 1650, CCL 75, and CCL 54; lymphoid cell lines ATCC Nos. TIB 53, CCL 167, CRL 1580, CRL 1581, TIB 6, TIB 18, CRL 1631, CRL 1662, CRL 1647, CRL 1648, CCL 86, CRL 8155, CRL 1596, CRL 1432, TIB 161, CCL 119 and CCL 155; and the CHO-K1 ovarian cell line, ATCC No. CCL 61. Lymphocyte lines are preferred hosts, particularly human or murine lymphocytes. More preferred are lymphocytes of a B-cell lineage, which may be a cell selected from the group consisting of a lymphoblastoid cell, a B-cell, and a plasmacytoma. Importantly, the host cell should be compatible with the replicon and control sequences of the expression vector which is used to transfect the immunoglobulin genes of interest. The host cell may also be a myeloma cell line capable of producing immunoglobulin.

The heavy and light chain DNA may be inserted into the host cell by a number of means, such as electroporation, which is described by Potter et al., 1984, Proc. Natl. Acad. Sci. USA, 81:7161-7165. Other methods of transfection which may be used include calcium phosphate coprecipitation of DNA, which is then added directly to the cultured host cells, Graham and Van der Eb, 1973, Virology, 52:456-467; DEAE dextran precipitation, Sompayrac and Danna, 1981, Proc. Natl. Acad. Sci. USA, 78:7575-7578; protoplast fusion, as described in Oi and Morrison, 1987, Biotechniques, 4:214-220; and microinjection, as described in Sullivan et al., 1985, Cancer Cells, 3:293-297. The foregoing articles are incorporated herein by reference in their entirety.

Following transfection, the cells are incubated in nonselective media for about two days, and then are transferred to a selective medium and observed for proliferation. After a sufficient time for cell outgrowth, the supernatants are tested for the presence of human light and/or heavy chains using means described in the examples hereinbelow. If the host cell secreted an immunoglobulin molecule or a portion thereof prior to transfection, the endogenous immunoglobulin should be distinguished from the immunoglobulin whose DNA sequences are being transfected. Cultures positive for human immunoglobulin of the desired isotype should be cloned and expanded (grown) in vitro for functional testing of the immunoglobulin obtained from the culture supernatant. Following the isolation of single cell clones, the cells may be removed from the selective medium by gradually decreasing the concentration of the selecting agent(s), so long as no deleterious effects are observed on cell growth or immunoglobulin production. The transfectoma may be cloned by cultured in vitro for the production of the desired immunoglobulin. Depending on whether the transfected host cell secreted in endogenous immunoglobulin, the novel recombinant immunoglobulin molecules produced may be much like monoclonal antibodies. Functional antibody tests will depend on the nature of the immunoglobulin; for instance, if the genes coding for the variable regions are cloned from a donor cell producing an antibody capable of specifically binding to a particular antigen, the immunoglobulin produced by the transfectoma should be able to specifically bind to the same antigen. Desirably, the recombinant immunoglobulin will have an affinity for the antigen of at least $10^{-6}$ M, more often at least $10^{-8}$ M, and preferably at least $10^{-9}$ M. Desirably, the recombinant immunoglobulin should also possess effector functions associated with the isotype of the constant region. It is to be understood that the present invention encompasses the novel immunoglobulins of this invention as well as any derivatives thereof, e.g., obtained by deletion, insertion, or substitution of amino acids or by altering the degree or presence of glycosylation. For example, certain amino acid changes may be introduced into the novel recombinant immunoglobulins of this invention to decrease their immunogenicity and thus increase their therapeutic potential.

In examples described hereinbelow, the light chain gene and heavy chain variable region gene were cloned from a lymphoblastoid cell line producing a monoclonal antibody that recognized several bacterial species. The heavy chain variable region gene was inserted into an expression cassette containing a heavy chain constant region gene coding for an isotype (according to one embodiment, IgG1 and according to another embodiment, IgG2) different from that of the donor cell line (IgM). The heavy chain constructs and the light chain genes were transfected into a mouse myeloma cell line. The antibodies produced by respective transfectomas were of the IgG isotype and were able to bind the same bacterial species in vitro as the monoclonal antibody of the donor cell line.

The transfected cell line producing the human immunoglobulin molecule may then be propagated for optimal antibody production, either in vitro or in vivo for the production of ascites, depending on the cell line. One advantage of the present invention is that it enables entirely human immunoglobulins to be produced in non-human cell lines, such as murine cells, which may be conveniently and economically propagated in large scale cultures. The use of non-human lines also results in the production of human immunoglobulins substantially free of other human proteins.

Having both human variable and constant regions, the recombinant immunoglobulins of the invention may find utility in administration to humans, whether as therapeutic or diagnostic agents, or as immunogens. The particular utility chosen will depend much on the antigen binding specificity of the immunoglobulin, the effector functions of the constant regions, etc. For therapeutic or prophylactic purposes, the immunoglobulins may be administered separately or in conjunction with other therapeutic or prophylactic agents well known to those skilled in the art. The immunoglobulins and pharmaceutical compositions of this invention are particularly useful for parenteral administration, such as subcutaneously, intramuscularly, or intravenously. Compositions of this invention for parenteral administration comprise a solution of the immunoglobulin or a cocktail thereof dissolved in a pharmaceutically acceptable excipient or carrier well known to those skilled in the art. Actual methods for preparing parenterally administrable compositions are described in more detail in, for example, Remington's Pharmaceutical Science, 15th Ed., Mack Publishing Co., Easton, PA (1980), which is incorporated herein by reference. In embodiments exemplified below, recombinant immunoglobulins capable of specifically binding to a preselected bacterial antigen are described, where the immunoglobulins possess effector functions, in that bacteria bound by the immunoglobulins are phagocytized by human neutrophils in vitro (see Examples V and VI, infra).

The recombinant human immunoglobulins may be used as carrier molecules for the targeting of a compound or compounds for delivery to specific cells, tissues, organs or any other site in vivo or in vitro. Recombinant human antibodies against any desired target (e.g., antigenic determinants or tumor cells, bacteria, viruses, fungi or parasites) may be used as carrier molecules. The compounds are selected according to the purpose of the intended application (e.g., cytotoxicity, preventing proliferation, target imaging) and may include, for example, pharmaceutical agents, toxins, enzymes, antibiotics, anti-metabolites, hormones, radioactive isotopes, radioopaque dyes, etc. It is desirable to attach the compound to the recombinant human antibody molecule without interfering with either the antigen binding capacity of the antibody or with effector functions such as the ability to activate complement. Once the antibody-compound conjugate is administered in vivo, the carrier antibody molecule will attach to the antigenic determinant of the target site and may subsequently release the linked compound. Such methods are described in further detail in U.S. Patent No. 4,671,958, which is incorporated herein by reference.

Another application of the recombinant human immunoglobulins of the invention is as antigen for use in humans as an antiidiotype vaccine. The recombinant human immunoglobulin which binds specifically to the relevant antigen is used, or the Fv, Fab, or F(ab')$_2$ portion thereof, to immunize a human to make antibodies specific for the variable region (antiidiotypic antibodies). Some of the antiidiotypic antibodies may share structural features with the original antigen (which itself could not be used as an immunogen), and may be used for immunization in place of the original antigen. See generally, Bona et al., 1985, Ann. Inst. Pasteur Immunol., 136:299-312 and Kohler et al., 1985, Proc. Soc. Exp. Biol. Med., 178:189-195, which are incorporated herein by reference.

For diagnostic purposes, the recombinant human immunoglobulins may be either labeled or unlabeled. Typically, diagnostic assays entail detecting the formation of a complex through the binding of the immunoglobulin to the antigenic determinant of the organism, cell or antigen desired to be detected. When unlabeled, the immunoglobulin may find use in agglutination assays or may be used in combination with other labeled antibodies that are reactive with the human immunoglobulin, such as antibodies specific for immunoglobulin. Alternatively, the immunoglobulins of the invention may be directly labeled, and a wide variety of labels may be employed. Numerous types of immunoassays are available and, by way of example, include those described in U.S. Patents Nos. 3,817,827; 3,850,752; 3,901,654; 3,935,074; 4,034,074; 4,098,876; and 4,376,110, all of which are incorporated herein by reference.

By virtue of the present invention, human immunoglobulins may be class switched to an isotype preferred for a particular utility. As many of the monoclonal antibodies produced by viral transformation are of the IgM class, conversion to the IgG class is of great benefit.

Other features and advantages of the present invention will become apparent from the following experimental description, which describes the invention by way of example. The examples are offered by way of illustration and not by way of limitation.


## EXAMPLE I


## CONSTRUCTION OF EXPRESSION CASSETTES AND IMMUNOGLOBULIN GENE CLONING


This Example describes methods for constructing recombinant DNA molecules encoding human antibodies of desired binding specificities and isotypes. The construction of cassettes for the expression of immunoglobulin genes is first described, followed by the cloning of the heavy chain variable region genes and light chain genes from two antibody secreting cell lines and the introduction of these genes into the expression cassettes.

In the experiments described below, the variable region genes from two established human lymphoblastoid cell lines (LCL) were used: 4B9 (ATCC CRL 9008), which secretes a human IgM kappa antibody reactive with Group B Streptococcus, Serratia marcescens, Enterobacter cloacae, Escherichia coli and Pseudomonas aeruginosa; and 9D1 (ATCC CRL 9489), which secretes a human IgG lambda antibody that binds to Serratia marcescens, Klebsiella pneumoniae, Enterobacter aerogenes, and Pseudomonas aeruginosa.


### A. Construction of Expression Cassettes

The vectors pSV2-gpt and pSV2-neo form the basis of the expression cassettes. The vectors were first modified to increase their usefulness; cassettes were then formed by the addition of constant region genes (heavy or light chain) and/or enhancers. In Sections B and C, adding the variable region genes to these cassettes is described.


### 1. Vector Modifications


### a. Modifications of pSV2-gpt Vector


### i. pSV2-gpt to pG (Fig. 1a)

In this Example, the pSV2-gpt shuttle vector described by Mulligan and Berg, 1980, Science, 209:1422-27, was modified to construct cassettes for the expression of Ig heavy and light chain genes. As will become clear later in the Example, it was desirable to modify the original pSV2-gpt vector so that 1) it did not contain a Hind III site which would interfere with later cloning steps; and 2) it would direct higher levels of expression of the Ecogpt gene in mammalian lymphocytes, thus facilitating selection of cells taking up this vector. Both of these goals could be accomplished by the removal of a 121 bp Hind III-Bgl II fragment located at the 5′ end of the Ecogpt gene. This 121 bp fragment contains two initiation codons upstream of the functional initiation codon (which is not on the 121 bp fragment). Removal of these upstream sites should increase levels of expression of the Ecogpt gene. Briefly, this was accomplished by digesting pSV2-gpt with Hind III and Bgl II, making the termini blunt ended with the Klenow fragment of DNA polymerase, self-ligating the DNA and transforming competent bacteria. The experimental details follow.

Ten micrograms of pSV2-gpt DNA (ATCC No. 37145) were digested to completion with Hind III (80 units for 1 hour at 37° C). The Hind III-digested material was then digested to completion with Bgl II (24 units for 30 minutes followed by 16 units for 1 hour at 37° C). The digested material was fractionated on a 1% agarose gel in TAE buffer (0.04 M Tris-acetate, 0.001 M EDTA). After staining with ethidium bromide at 0.25 μg/ml, DNA fragments of 5.4 kb and 121 bp were visualized. The higher molecular weight band was electrophoresed into NA45 DEAE paper (Schleicher and Schuell) and then eluted from the paper according to the manufacturer's instructions. The DNA was precipitated with ethanol and resuspended to approximately 1 μg/μl in TE buffer (10 mM Tris, pH 7.8, 1 mM EDTA).

The termini created by the restriction endonucleases were made to be blunt ended using the Klenow fragment of DNA polymerase (2 units enzyme per 1 μg of DNA in 25 μl buffer (50 mM Tris, pH 7.2, 10mM MgSO4, 0.08 mM dATP, 0.08 mM dCTP, 0.08 mM dTTP, 0.08 mM dGTP, 0.1 mM DTT, 50 μg/ml BSA) for 10 minutes at room temperature). The sample was immediately phenol and chloroform extracted and then precipitated with ethanol. The DNA pellet was resuspended in TE to 1 μg/μl. One microgram of DNA was self-ligated in a final volume of 20 μl at 15° C for 7 hours using 1 unit T4 DNA ligase (Bethesda Research Laboratories) in "ligation buffer" (50 mM Tris, pH 7.8, 10 mM MgCl$_2$, 1 mM DTT, 6 mM KCl, 1 mM ATP, 1 mM spermidine, and 5% polyethylene glycol (Sigma PEG 8000)).

The ligation reaction was diluted 1:5 in Te and 1 μl was used to transform 100 μl of competent E. coli DH5α cells (Bethesda Research Labs) according to manufacturer's instructions. Transformed bacteria were plated on LB agar containing 100 μg/ml ampicillin. Plasmid DNA from ampicillin resistant colonies was screened for its ability to be cut with EcoR I, but not with Hind III or Bgl. II. The resultant vector was termed pG.

### ii. pG to pUCG (Fig. 1a)

The pBR322 sequences of pG were replaced with the analogous sequences from pUC18 to increase yields of plasmid DNA. Briefly, this was accomplished by digesting pG with EcoR I and Pvu II, recovering the 3 kb DNA fragment containing the Ecogpt gene and SV40-derived DNA, and ligating it to the Pvu II fragment from pUC18 containing the origin of replication and ampicillin resistance gene.

The details were as follows: Ten micrograms of pUC18 (Bethesda Research Laboratories) were digested to completion with Pvu II (50 units for 2 hours at 37° C) and 10 μg of pG were digested to completion with EcoR I (50 units for 2 hours at 37° C), and then with Pvu II (50 units for 2 hours at 37° C). Both digests were electrophoresed through 1.2% agarose and the desired fragments (3 kb for pG and 2.4 kb for pUC18) were electrophoresed into a NA45 DEAE membrane. The DNA was then eluted from the DEAE paper according to the manufacturer's instructions, ethanol precipitated and resuspended to 1 μg/μl in TE buffer.

The EcoR I ends from the digested pG were filled in using the Klenow fragment of DNA polymerase (2 units of enzyme and 1 μg DNA for 10 minutes at room temperature in a 25 μl reaction volume). The sample was then phenol and chloroform extracted, ethanol precipitated and resuspended to 200 ng/μl in TE buffer. Two micrograms of the pUC18 fragment were treated with calf intestinal alkaline phosphatase (CIP; Boehringer-Mannheim) suspended in 50 mM Tris-Cl, pH 9.5, 1 mM MgCl$_2$m, 0.1 mM ZnCl$_2$, 1 mM spermidine, (10 units for 15 minutes at 37° C followed by 15 minutes at 56° C), phenol and chloroform extracted, ethanol precipitated and resuspended to 250 ng/μl in TE buffer.

Twenty nanograms of the pG fragment and 25 ng of the pUC18 fragment were ligated overnight at 15° C in a 20 μl volume. One hundred microliters of competent DH5α cells were transformed with a portion of the ligation mixture. Colonies were screened for the presence of a single Pst I site, which is within the SV40 region of pG. There is an additional Pst I site in the amp$^r$ gene of pBR322 but not in the amp$^r$ gene of pUC18. The plasmids were also screened for the regeneration of the EcoR I site upon ligation of Pvu II ends with filled-in EcoR I ends. The resultant plasmid was termed pUCG.

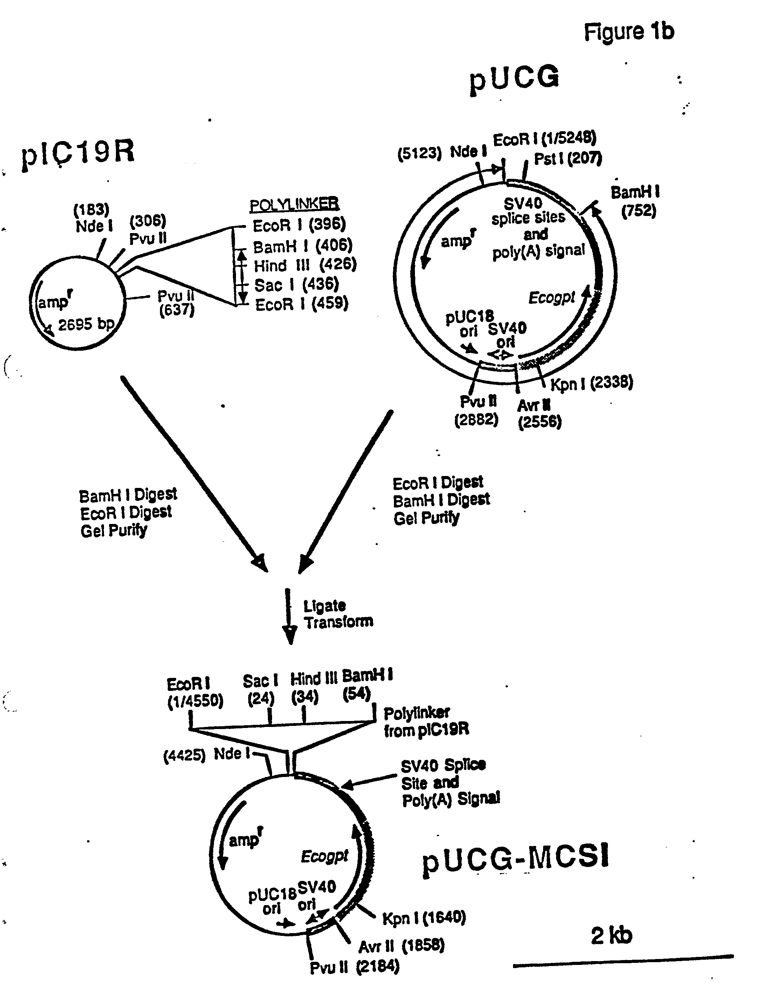

### iii. pUCG to pUCG-MCSII (Figs. 1b and 1c)

It was desirable to have as many restriction sites as possible available for cloning variable and constant region genes into the pUCG vector. Therefore the EcoR I to BamH I region of pUCG was replaced with the polylinker or multiple cloning sites (MCS) region derived from two sources: pIC19R (obtained from J. Lawrence Marsh, Department of Developmental and Cell Biology, School of Biological Sciences, University of California at Irvine; see also, Marsh et al., 1984, Gene, 32:481-485) and pUC18. This was accomplished in two stages: first the EcoR I-BamH I fragment of pUCG was replaced with the EcoR I-BamH I polylinker fragment of pIC19R to form pUCG-MCSI (where MCSI indicates Multiple Cloning Site I). Then, the Hind III-BamH I fragment of pUCG-MCSI was replaced with the Hind III-BamH I polylinker fragment of pUC18. This vector was designated pUCG-MCSII and contains 14 unique restriction sites in the multiple cloning site region.

The details of the construction were as follows. Ten micrograms of pUCG were digested to completion with EcoR I (100 units for 2 hours at 37°C), extracted with PCI (phenol:chloroform: isoamyl-alcohol in a ratio of 25:24:1), ethanol precipitated, and resuspended in 100 μl TE buffer. The vector was then digested to completion with BamH I (200 units for 2 hours at 37°C), PCI extracted, ethanol precipitated, and resuspended in 80 μl TE buffer. The DNA was then electrophoresed through 1% agarose and the desired higher molecular weight fragment then electrophoresed into NA45 DEAE paper and eluted. The DNA was then PCI extracted, ethanol precipitated, resuspended and centrifuged through a "spun-column," as described by Maniatis et al., Molecular Cloning, A Laboratory Manual, 1982, pp. 466-7. This latter procedure removed particulate material, such as agarose, which might have contaminated the sample. The DNA was ethanol precipitated and resuspended in 60 μl TE buffer.

Twenty micrograms of pIC19R were digested to completion with BamH I (400 units for 2 hours at 37°C), PCI extracted, ethanol precipitated, and resuspended in 200 μl TE buffer. The DNA was then digested to completion with EcoR I (200 units for 2 hours at 37°C), PCI extracted, ethanol precipitated, and resuspended in 80 μl TE. The DNA was electrophoresed through 4% NuSieve agarose (FMC Corporation) in TBE buffer (89 mM Tris-borate, 89 mM boric acid, and 8 mM EDTA). The small polylinker fragment from pIC19R was electrophoresed into NA45 DEAE paper, eluted and further purified as described in the previous paragraph. The DNA was ethanol precipitated and resuspended in 20 μl TE buffer.

Ten nanograms of vector (pUCG fragment) were then ligated with 250 ng polylinker fragment (from pIC19R) (1 unit BRL T4 DNA ligase and ligase buffer lacking spermidine to a final volume of 25 μl, for 30 minutes at 37°C). The ligation was diluted 1:5 in TCM (10 mM Tris, pH 7.9, 10 mM MgCl$_2$, and 10 mM CaCl$_2$) and 25 μl was used to transform 200 μl of competent DH5α cells. Plasmid DNA was prepared from the resulting colonies and the DNA screened for the presence of both a Sac I (from the polylinker) and a Kpn I (derived from the gpt gene in pUCG) site. This construct was termed pUCG-MCSI.

pUCG-MCSI was then digested to completion with BamH I and Hind III (200 units each per 2.5 μg DNA), precipitated with ethanol, resuspended in 50 μl TE buffer, and electrophoresed through a 1% agarose gel. The higher molecular weight band was electrophoresed into NA45 DEAE paper, purified as described above, and resuspended in 50 μl TE buffer.

Sixty micrograms of pUC18 were digested to completion with BamH I and Hind III (400 units each), ethanol precipitated, redissolved in 50 μl TE buffer and electrophoresed through 4% NuSieve agarose (FMC Corporation) in TBE. The small BamH I-Hind III fragment was collected into NA45 DEAE paper, purified as described above and resuspended in 20 μl TE buffer.

Fifty nanograms of vector (pUCG-MCSI) and 300 ng pUC18 polylinker were ligated in a 50 μl reaction volume (in ligase buffer) for 30 minutes as previously described, the mixture diluted 1:5 in TCM, and 25 μl used to transform 200 μl competent DH5α cells. Plasmid DNA was prepared from the transformants and screened for the presence of an Xba I site derived from the polylinker of pUC18 (the XbaI site in the polylinker of pIC19R and pUCG-MCSI is only restricted with XbaI when the plasmid is grown in methylase-(dam-) cells) and a Kpn I site derived from pUCG-MCSI. The resultant vector was designated pUCG-MCSII. The sequence of the polylinker region was as follows:

5' GAATT CATCG ATATC TAGAT CTCGA GCTCG CGAAA GCTTG

CATGC CTGCA GGTCG ACTCT AGAGG ATCC 3'

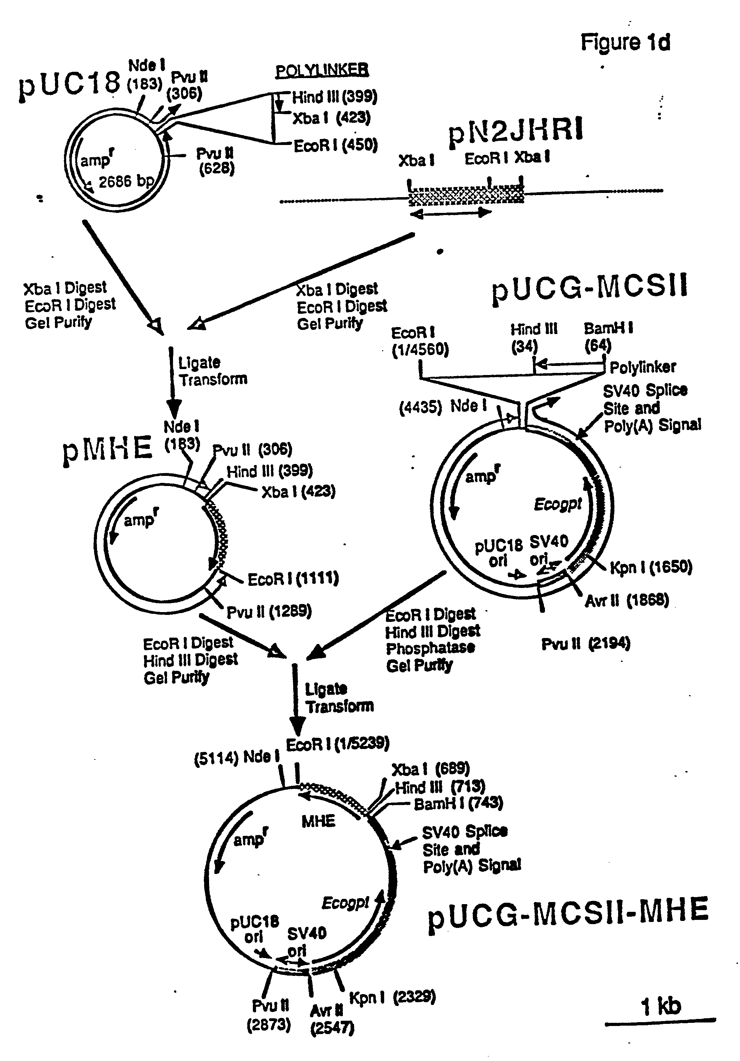

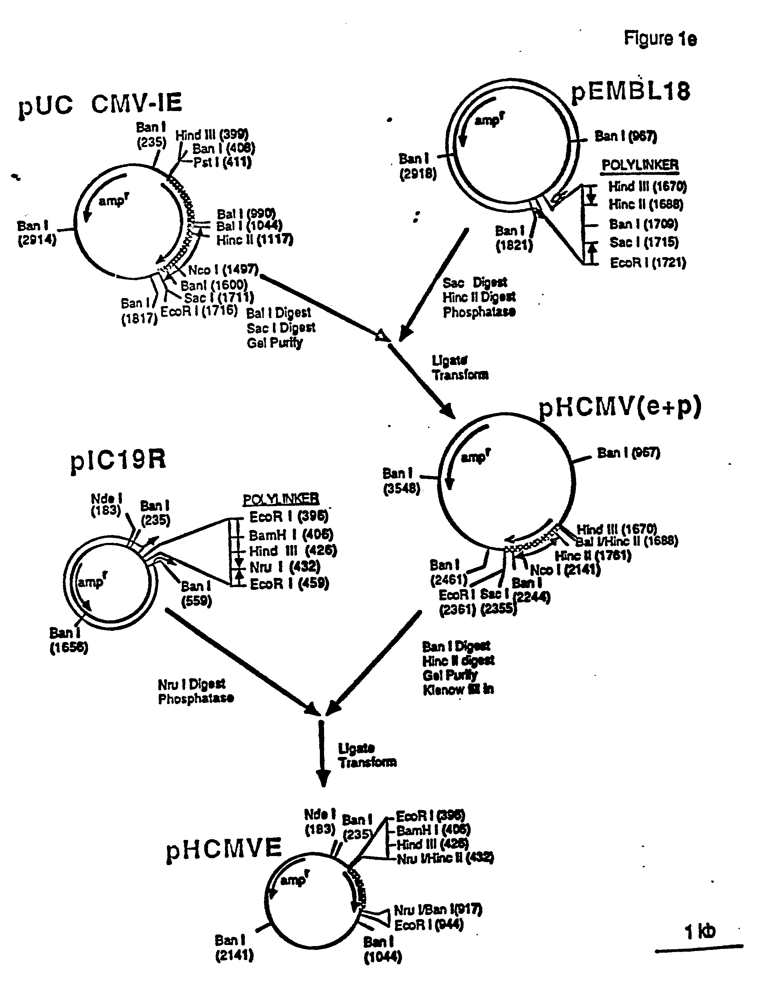

### iv. pUCG-MCSII to pUCG-MCSII-MHE (Fig. 1d)

To obtain higher levels of expression of light chain immunoglobulin genes, the mouse heavy chain enhancer (MHE) was inserted into the pUCG-MCSII vector to form pUCG-MCSII-MHE. The enhancer was positioned in the MCS region such that the immunoglobulin gene of interest could be inserted 5' or 3' of the enhancer or the enhancer could be located within an intron in the gene.

The mouse heavy chain enhancer (MHE) was obtained from a plasmid, pN2JHR1, which is an EcoR I subclone of the bacteriophage B122 (obtained from Phillip Tucker, Department of Microbiology, Southwestern Medical School, Dallas, TX) which is derived from NZB liver DNA. Alternatively, the mouse heavy chain enhancer can be obtained from most cloned mouse heavy chain genes, e.g., bacteriophage M23 described in Marcu et al., 1981, Cold Spring Harbor Symposia on Quantitative Biology, 45:899-911. The MHE was subcloned as a 688 bp Xba I-EcoR I fragment from pN2JHR1 using the following protocol.

Fifty micrograms of pN2JHR1 were digested to completion with EcoR I (150 units) and Xba I (200 units), phenol and chloroform extracted, ethanol precipitated, resuspended in 50 μl TE buffer, and electrophoresed through 1% agarose. The 688 bp fragment containing the MHE was electrophoresed into NA45 DEAE paper, eluted and purified as described above and resuspended in 50 μl TE buffer. pUC18, 2.6 μg, was then digested to completion with EcoR I (25 units) and Xba I (50 units), phenol and chloroform extracted, ethanol precipitated, resuspended in 50 μl TE, and electrophoresed through 1% agarose. The higher molecular weight fragment was electrophoresed into NA45 DEAE paper and further purified as described above. Following ethanol precipitation, it was resuspended in 20 μl TE buffer. 5 μl of the 688 bp fragment from pN2JHR1 were then ligated with 2 μl pUC18 (final volume of 25 μl) overnight at 15°C. The ligation mixture was then diluted 1:5 in TCM buffer and used to transform 200 μl competent E. coli JM109 cells, a strain described in Yanisch-Perron et al., 1985, Gene, 33:103-119. Transformants were screened with EcoR I and Xba I for the desired 688 bp insert. The resultant plasmid was termed pMHE.

The MHE was then inserted into pUCG-MCSII as an EcoR I-Hind III fragment. The Hind III site was derived from the polylinker in pUC18. pUCG-MCSII was digested to completion with EcoR I and Hind III, treated with CIP (1 μg DNA, 40 units CIP, for 30 minutes at 37°C) and 100 ng was electrophoresed through SeaPlaque low melt agarose (FMC Corporation). About 20 to 30 μl of agarose containing the higher molecular weight fragment was transferred to a microfuge tube. pMHE was then digested to completion with EcoR I and Hind III, and 100 ng was electrophoresed through low melt agarose. The agarose (about 20 to 30 μl) containing the 0.7 kb fragment was transferred to a microfuge tube. The two agarose plugs, containing digested pUCG-MCSII and pMHE, respectively, were melted at 70°C for 5 minutes and 5 μl of each were then combined and cooled to 37°C. The samples were ligated for 20 minutes at 37°C in a final volume of 25 μl in ligation buffer, then heated at 70°C for 5 minutes and diluted with 60 μl TCM buffer. One hundred microliters of competent DH5α cells were transformed with a portion of the ligation reaction. DNA was prepared from transformants and screened for the presence of a 0.7 kb Hind III-EcoR I fragment containing MHE. The resulting construct was designated pUCG-MCSII-MHE.

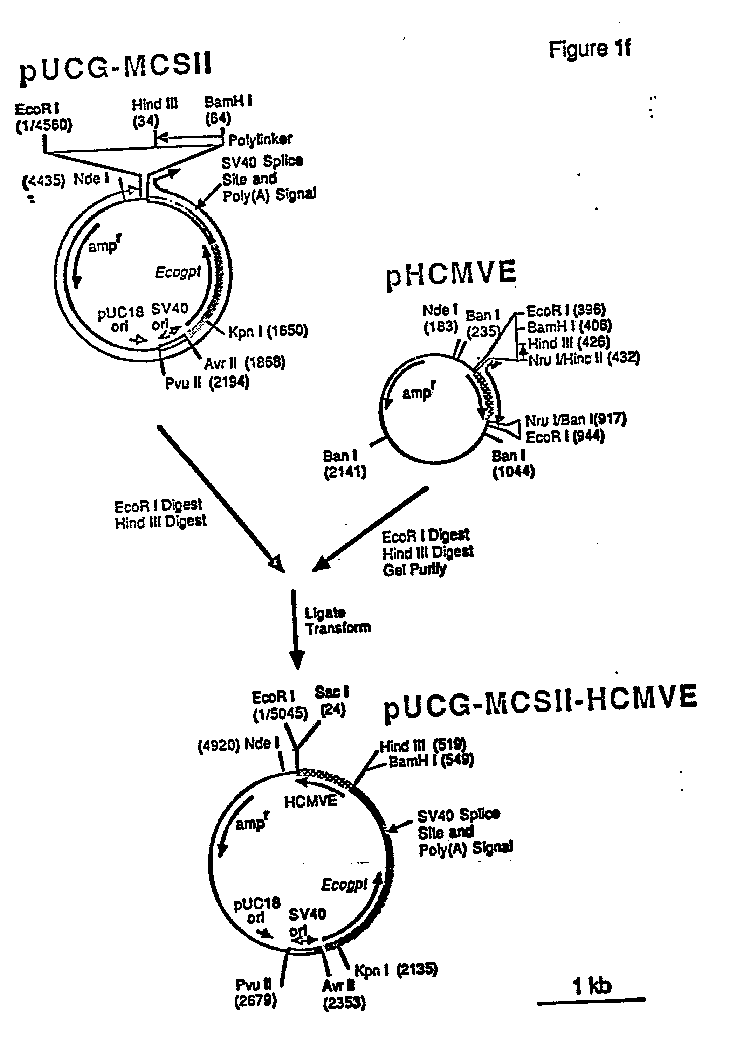

v. pUCG-MCSII to pUCG-MCSII-HCMVE (Figs. 1e and 1f)

The enhancer from the Immediate Early region of human cytomegalovirus (HCMV) was inserted into the vector pUCG-MCSII. The resulting plasmid, pUCG-MCSII-HCMVE, formed the basis for a cassette (described in Section A.3) for the expression of kappa immunoglobulin genes. This vector can be modified similarly to form a cassette for the expression of heavy chain or lambda light chain genes.

The plasmid pUC CMV-IE was obtained from Dr. Denise Galloway (Fred Hutchinson Cancer Research Center, Seattle, WA). It consists of a 1.3 kb Sac I-Pst I insert, derived from the Pst I m fragment (Greenway, P. J. et al., 1982, Gene, 18:355-360) of the AD169 strain of human cytomegalovirus, in pUC18 . The sequence of a portion of this fragment can be found in Boshart et al., Cell, 41:521-530 (1985). This fragment contains both enhancer and promoter sequences. For the purpose of this Example, it was desirable to remove the known promoter sequences from the enhancer and then to insert the enhancer sequences into the pUCG-MCSII vector. Briefly, a 655 bp Bal I-Sac I fragment from pUC-CMV-IE was subcloned into pEMBL18 (Dente et al., 1983, Nucleic Acids Research, 11:1645-1655). This fragment contained both enhancer and promoter sequences and was designated pHCMV(e + p) (see Figure 1e). Following a Klenow fill-in reaction, the Ban I-Hinc II fragment from the resulting plasmid was inserted into the Nru I site of pIC19R vector to form pHCMVE. This plasmid contains only the enhancer sequences. These sequences were then transferred into pUCG-MCSII as an EcoR I-Hind III fragment to form pUCG-MCSII-HCMVE. The details are provided below.

Five micrograms of pEMBL18 + strand were digested with Sac I (25 μl volume, 50 units for 2 hours), and were then digested with Hinc II (100 μl volume, 20 units for 2 hours). The DNA was PCI extracted, ethanol precipitated, and resuspended in 20 μl TE buffer. The DNA was then treated with CIP. The reaction was stopped with nitrilotriacetic acid, PCI extracted, and run over a "spun column." The DNA was ethanol precipitated and resuspended in 75 μl TE buffer to approximately 50 ng/μl.

Ten micrograms of pUC CMV-IE were simultaneously digested with Bal I and Sac I (10 units Bal I and 30 units Sac I in Bal I buffer, overnight digestion). The entire sample was electrophoresed through agarose. Following separation of the fragments, the 655 bp Bal I-Sac I fragment was electrophoresed into NA45 DEAE paper and eluted.

Approximately 100 ng of the 655 bp Bal I-Sac I HCMV insert were ligated with 10 ng of the pEMBL18 vector (400 units T4 DNA ligase (New England Biolabs) in ligase buffer lacking spermidine, 25 μl reaction volume, overnight at 15° C). The DNA was diluted 1:5 in TCM buffer and a portion was used to transform competent DH5α cells. Plasmid DNA was prepared from ampicillin resistant colonies and the DNA was digested with Hind III and EcoR I. The desired colonies showed a plasmid insert size of approximately 650 bp. This DNA was designated pHCMV(e + p).

Ban I was then used to separate the promoter from the enhancer. The subcloning of the Hinc II-Ban I fragment of pHCMV(e + p) into the Nru I site of pIC19R was accomplished as follows. Five micrograms of. pIC19R was digested to completion with Nru I (12.5 units, 2 hours at 37° C). The DNA was PCI extracted, ethanol precipitated and resuspended in 25 μl TE buffer. The DNA was treated with CIP, further purified as described above, ethanol precipitated and resuspended to approximately 50 ng/μl in TE buffer. pHCMV-(e + p) was then digested with Ban I followed by Hinc II, PCI extracted, ethanol precipitated and resuspended in TE buffer. The DNA was electrophoresed through agarose and the desired 484 bp Ban I-Hinc II fragment was electrophoresed into NA45 DEAE paper. The DNA was recovered as previously described. The sticky ends of the Ban I site were then filled in using the Klenow fragment of DBA polymerase (in 10X BRL nick translation buffer containing dATP, dCTP, dGTP supplemented with 10μCi $^{32}$P-dCTP, 5 minutes at room temperature, followed by a chase with unlabelled dCTP for an additional 5 minutes at room temperature). Unincorporated nucleotides were removed by separation on a "spun column." The DNA was ethanol precipitated and resuspended in TE buffer.

Approximately 50 ng Nru I digested pIC19R vector was then ligated with approximately 250 ng purified 484 bp Ban I-Hinc II fragment (5 minutes at room temperature with 200 units T4 DNA ligase (New England Biolabs) in ligation buffer lacking spermidine in a volume of 10 μl, followed by overnight incubation at 15° C with an additional 400 units of ligase in a final volume of 25 μl). A portion of the ligation was then diluted 1:5 in TCM and used to transform 200 μl of competent DH5α cells. Ampicillin resistant colonies were screened for plasmid containing an approximately 500 bp EcoR I-Hind III insert in pIC19R. This plasmid was designated pHCMVE. The identity of the insert was further confirmed by linearization with Nco I, which cuts within the enhancer fragment but not within pIC19R, and by digestion with Hinf I which cuts once within the enhancer and multiple times within pIC19R.

The HCMV enhancer was then inserted into pUCG-MCSII as an EcoR I-Hind III fragment. Five micrograms of pUCG-MCSII was digested with Hind III and EcoR I (30 units Hind III, 7.5 units EcoR I in EcoR I buffer for 2 hours). The DNA was PCI extracted, ethanol precipitated and resuspended in TE buffer. pHCMVE was digested to completion with EcoR I and Hind III. The DNA was ethanol precipitated, resuspended in TE buffer, and electrophoresed through agarose. The 500 bp fragment was collected into NA45 DEAE paper and then eluted. Approximately 10 ng pUCG-MCSII (digested with EcoR I and Hind III) were ligated with approximately 250 ng HCMVE EcoR I-Hind III insert as described above. A portion of the ligation reaction was diluted 1:5 in TCM and used to transform competent DH5α cells. Plasmid DNA from ampicillin resistant colonies was digested with EcoR I and Hind III. A transformant yielding bands of 500 bp and approximately 4.6 kb was designated pUCG-MCSII-HCMVE. The orientation of the enhancer was not determined. Subsequent experiments have indicated that the enhancer is in the same orientation as the gpt gene (see Figure 1f).

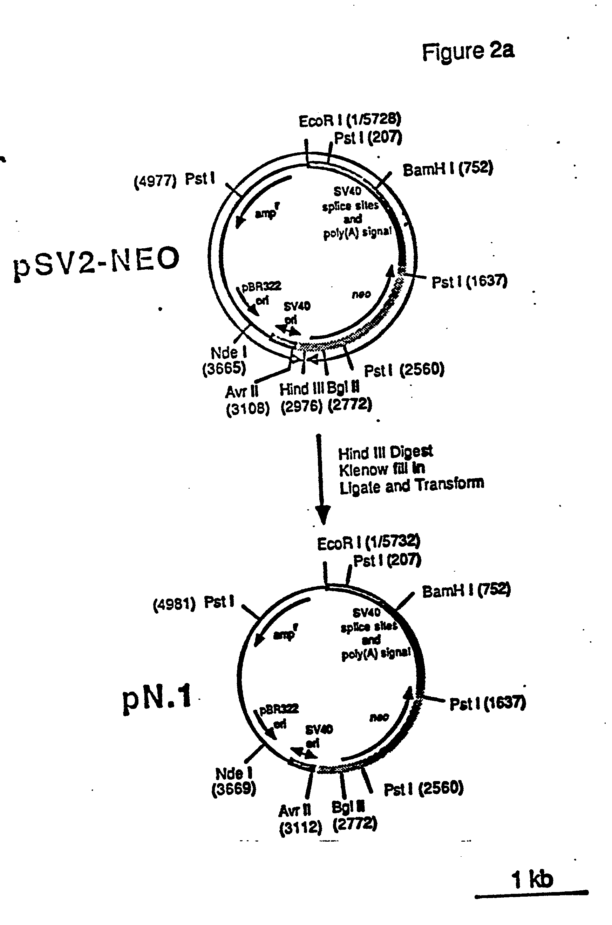

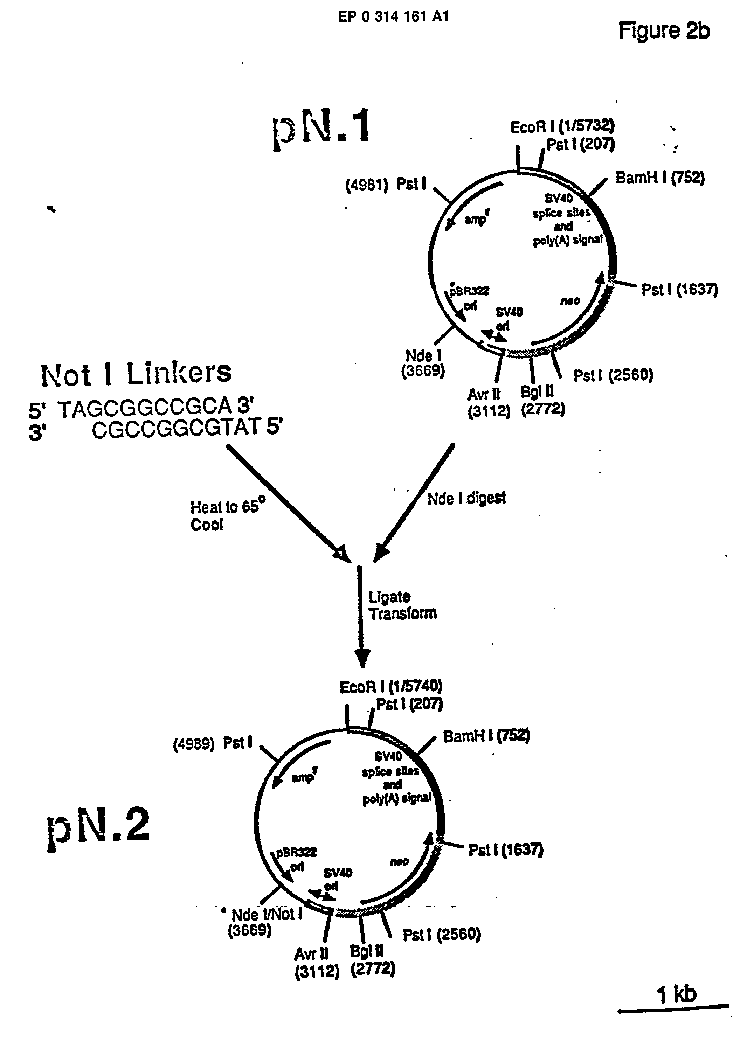

### b. Modifications of pSV2-neo Vector (figs. 2a and 2b)

In this embodiment of the invention, the pSV2-neo vector (ATCC No. 37149; described by Southern and Berg, 1982, J. Mol. Appl. Genet., 1:327-341) was modified to construct a cassette for the expression of immunoglobulin heavy chain genes. The pSV2-neo vector was modified so that it did not contain a Hind III site which would interfere with later cloning steps. Briefly, this was accomplished by digesting pSV2-neo with Hind III, making the termini blunt ended with the Klenow fragment of DNA polymerase, self-ligating the DNA and using it to transform competent bacteria. The procedure was similar to that used for modifying the pSV2-gpt vector, described above, except that pSV2-neo was digested only with Hind III and the gel purification step was omitted and replaced with a phenol and chloroform extraction. The resulting vector was designated pN.1 (also referred to in copending U.S. parent application, Serial No. 114,632, as pN).

A second vector was then constructed from pN.1, in which a Not I restriction site was added at the Nde I site. Not I restricts mammalian DNA only rarely and does not cut pUC18, pBR322, pSV2-gpt, pSV2-neo or any of the heavy or light chain genes tested (see infra). Thus, inclusion of this restriction site in pN.1 facilitates the ability to linearize, at a predetermined site, the vectors described infra. As depicted in Figure 2b, 1.25 μg of pN.1 was digested to completion with Nde I (40 units for 2 hours at 37° C). The following two linkers, each at 0.56 mg/ml, were combined and heated to 65° C fcr 10 minutes and then cooled:

$$\text{5' TAGCGGCCGCA 3'}$$
$$\text{3' CGCCGGCGTAT 5'}$$

When annealed, these linkers form a Not I site. 50 ng of the linearized pN.1 DNA were ligated with 2.8 μg of the linker oligonucleotide mixture in ligation buffer, using 1 unit of T4 DNA ligase (Boehringer-Mannheim) in a reaction volume of 20 μl overnight at 14° C. The ligation reaction was then ethanol precipitated and resuspended in 50 μl TE. 10 μl of the resulting mixture was used to transform 250 μl of competent E. coli JM83 cells (ATCC 35607). Plasmid DNA was prepared from transformed colonies and those which contained a Not I site were selected. The resulting vector was designated pN.2.

## 2. Heavy Chain Cassettes

It was desirable to construct cassettes comprising heavy chain constant region genes in the vector such that the variable region gene could easily be inserted 5' of the constant region gene to produce an expression vector containing a complete and expressible heavy chain gene. This was accomplished by inserting the heavy chain constant region gene into a modified vector along with a short polylinker 5' of the constant region gene. The variable region genes were then inserted into the polylinker region.

### a. γ2 Heavy Chain Cassettes

In this Example, a phage clone, Phage 5A (Ellison and Hood, 1982, Proc. Natl. Acad. Sci., 79:1984-1988) was used as a source of the germline γ2 gene. This clone had been obtained by screening a human fetal liver genomic DNA library with a γ3 cDNA constant region probe (pOMMC, described in Alexander et al., 1982, Proc. Natl. Acad. Sci. USA, 79:3260-3264).

Briefly, the cassette was prepared as follows. A Hind III fragment from Phage 5A, containing the γ2 gene, was subcloned into pUC18 in the orientation such that the 5' end was adjacent to the polylinker region. This construct was then digested with BamH I, thus releasing the γ2 gene with a polylinker at the 5' end. The isolated fragment was then cloned into the BamH I sites of pG and pN.1, forming the cassettes pGγ2 and pNγ2, respectively.

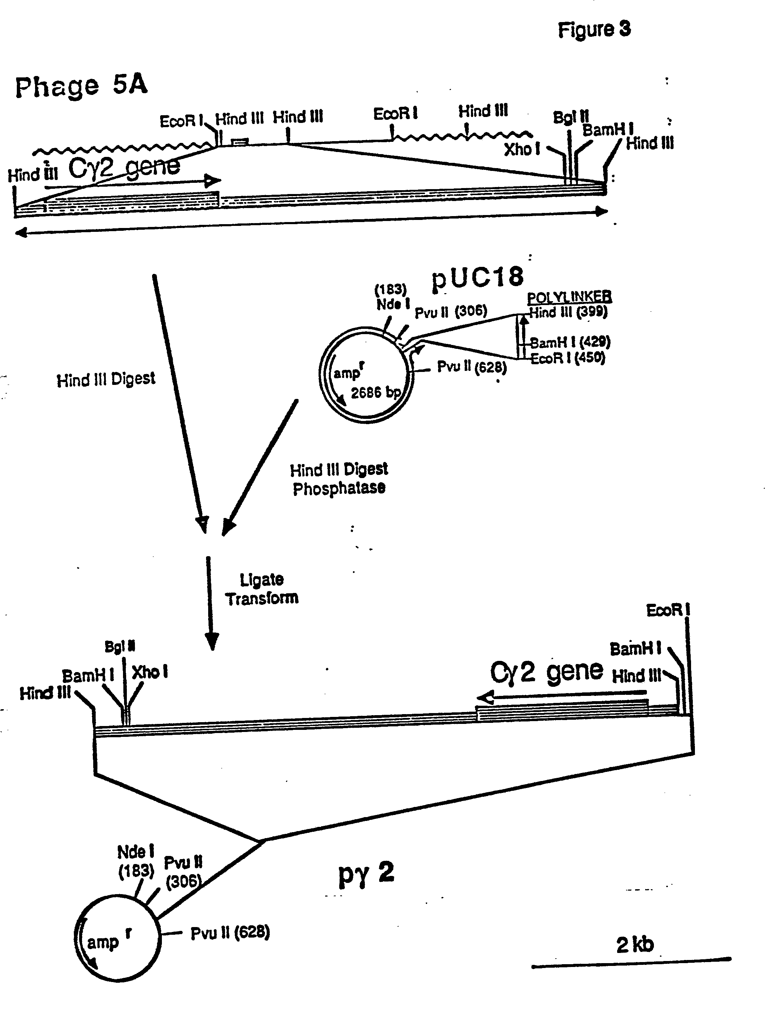

### i. Phage 5A to pγ2 (Fig. 3)

The DNA containing the γ2 gene was prepared by digesting 1 μg of Phage 5A with Hind III (20 units), extracting with phenol and chloroform, ethanol precipitating and resuspending to 20 ng/μl in TE buffer. pUC18 was then digested with Hind III, treated with CIP, phenol and chloroform extracted, ethanol precipitated and resuspended to 50 ng/μl in TE. One hundred nanograms of digested Phage 5A were ligated to 50 ng pUC18 with 100 units T4 DNA ligase (New England Biolabs) in ligation buffer. The ligation mixture was used to transform competent DH5α E. coli and the bacteria were plated on agar containing 100 μg/ml ampicillin and X-gal as a color indicator.

Plasmid DNA was prepared from the white colonies which resulted from the transformation. The plasmids were first screened for those containing the correct insert, and then screened for those containing the insert in the desired orientation. In the selected colonies, digestion of plasmid DNA with Hind III produced fragments of 2.7 kb (derived from pUC18) and 6.0 kb (containing the γ2 gene). The latter fragment hybridized with a γ3 probe (p3.6RH4.2, described in Krawinkel and Rabbitts, 1982, EMBO J., 1:403-407). BamH I digestion of plasmid DNA produced fragments of 2.9 kb and 5.8 kb, while EcoR I and Xho I double digestion produced fragments of 5.8 kb and 2.9 kb. The resulting plasmid was termed pγ2.

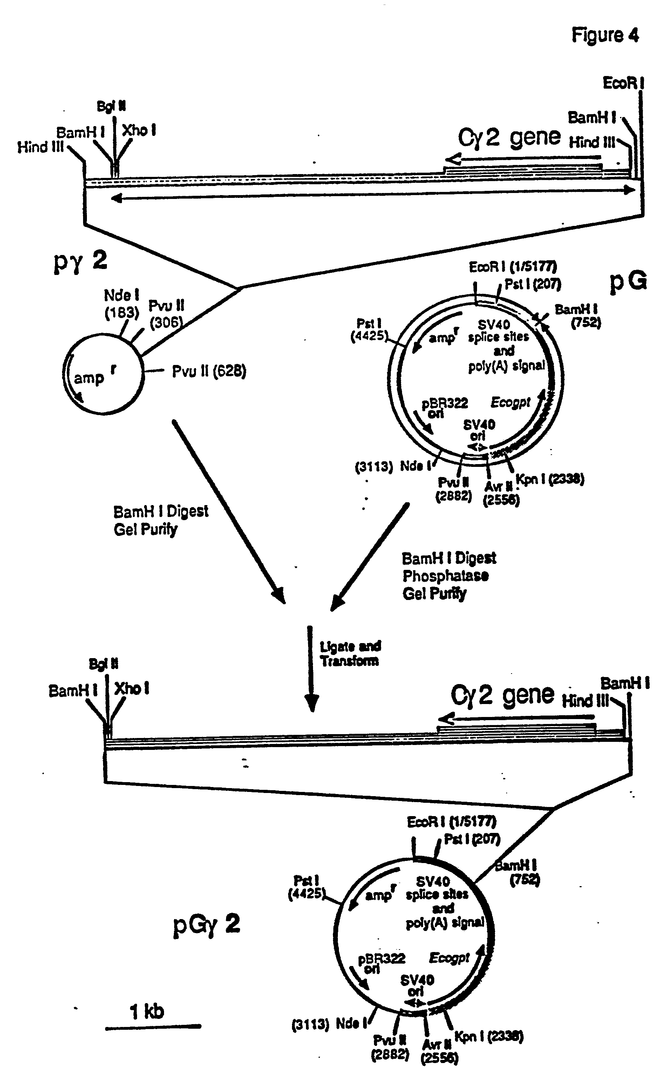

### ii. Gpt system: pγ2 to pGγ2 (Fig. 4)

pγ2 was digested with BamH I to release the γ2 insert. The DNA was then ethanol precipitated and resuspended to 1 μg/μl in TE buffer. Ten micrograms of the BamH I digest were then digested with EcoR I to minimize the religation of the pUC18 portion of the molecule. The sample of digested pγ2 DNA was then fractionated on 0.7% low melt agarose gel (SeaPlaque, FMC Corp.) in TAE buffer (40 mM Tris-acetate and 1 mM EDTA) containing 0.25 μg/ml ethidium bromide. The band containing the γ2 gene was excised (20 to 30 μl) and melted in a 1.5 ml microfuge tube at 70° C.

The vector, pG, was then digested to completion with BamH I, phenol and chloroform extracted, ethanol precipitated and resuspended to 1 μg/μl in TE buffer. One microgram of the digested pG DNA was treated with 19 units CIP and 5 μl (0.05 μg) was fractionated on a 0.7% low melt agarose gel in TAE buffer with 0.25 μg/ml ethidium bromide. An agarose slice (20 to 30 μl) containing the linearized DNA was excised and melted at 70° C in a 1.5 ml microfuge tube.

To insert the γ2 gene into the pG vector, 2 μl of the melted agarose containing the BamH I-digested pG vector were combined with 2 μl of the melted agarose containing the γ2 gene and ligated as described above. The ligation mixture was heated to 70° C for 5 minutes to remelt the agarose, diluted 1:5 in TCM and 40 μl used to transform 200 μl competent DH5α cells. The bacteria were plated on agar containing 100 μg/ml ampicillin. DNA was prepared from 2 ml overnight cultures of each of the resulting colonies and the DNA was screened for the γ2 gene in the pG vector. This DNA was termed pGγ2. The γ2 gene was found to be in the same orientation as the gpt gene. This was determined by double digestion with EcoR I and Hind III, which produced bands of 4.45 kb and 6.75 kb.

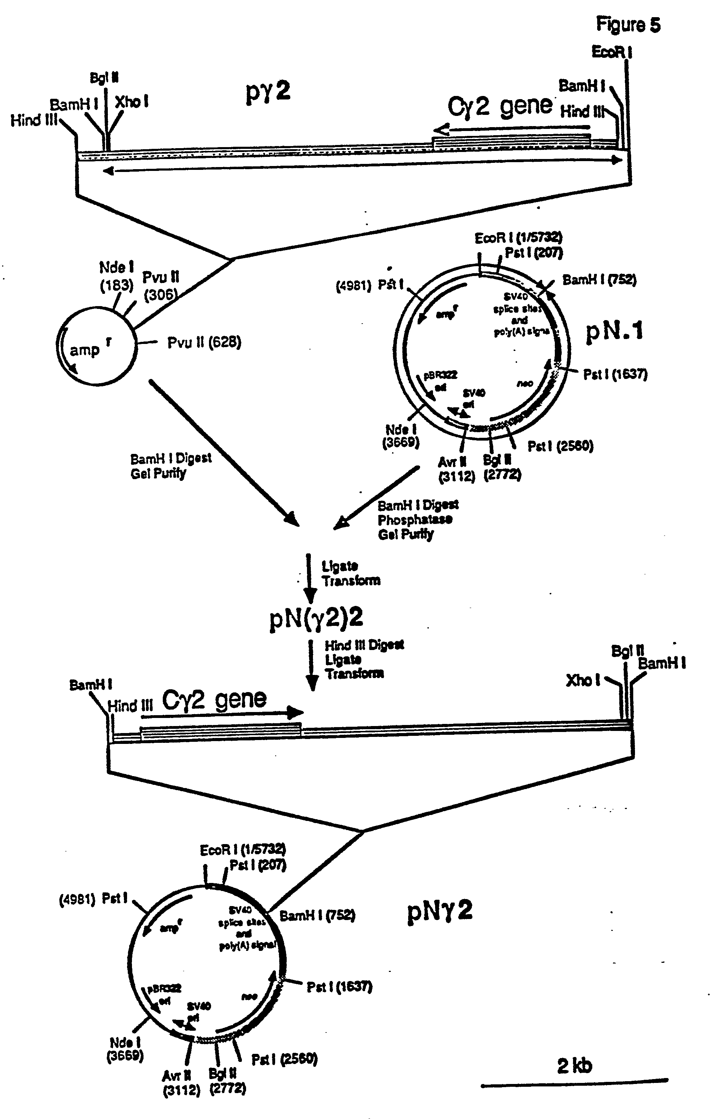

### iii. Neo system: pγ2 to pNγ2 (Fig. 5)

The γ2 gene was inserted into the pN.1 vector essentially as described above for its insertion into the pG vector. The insert containing the γ2 gene was prepared from pγ2 as described above. The pN.1 vector was prepared as follows: pN.1 was digested to completion with BamH I, then phenol and chloroform extracted, ethanol precipitated and resuspended to 0.1 μg/μl in TE. The BamH I digested DNA was treated with CIP (11 units per 0.5 μg DNA), and 0.1 μg of the DNA was fractionated on a 0.7% low melt agarose gel in TAE buffer with 0.25 μg/ml ethidium bromide. An agarose slice (20 to 30 μl) containing the linearized DNA was excised and melted at 70° C in a 1.5 ml microfuge tube.

To ligate the pγ2 insert with the pN.1 vector, 2 μl of the melted agarose containing pN.1 digested with BamH I were combined with 10 μl of the melted agarose containing the γ2 gene and ligated as described above. The ligation mixture was then heated to 70° C for 5 minutes to remelt the agarose, diluted 1:2 in TCM and 50 μl was used to transform 200 μl competent DH5α cells. The bacteria were plated on agar containing 100 μg/ml ampicillin and 0.5% glucose.

DNA was prepared from overnight cultures of each of the resultant colonies and the DNA was screened for the γ2 gene in the pN.1 vector. A combination of Bgl II and BamH I double digests, Bgl II single digest, and EcoR I and Xho I double digests showed that two copies of the γ2 gene had been inserted in a head to tail configuration, but in the opposite orientation as the neo gene. This DNA is termed pN(γ2)2. As explained below, digestion of pN(γ2)2 with Hind III removed the second copy of the γ2 gene and allowed insertion of the variable region gene, so this vector was used without further modification. However, as shown in Figure 5, for future use, pN(γ2)2 was also digested with Hind III and religated so that only one copy of the γ2 gene would be present. This new vector was called pNγ2.

### b. γ1 Heavy Chain Cassettes

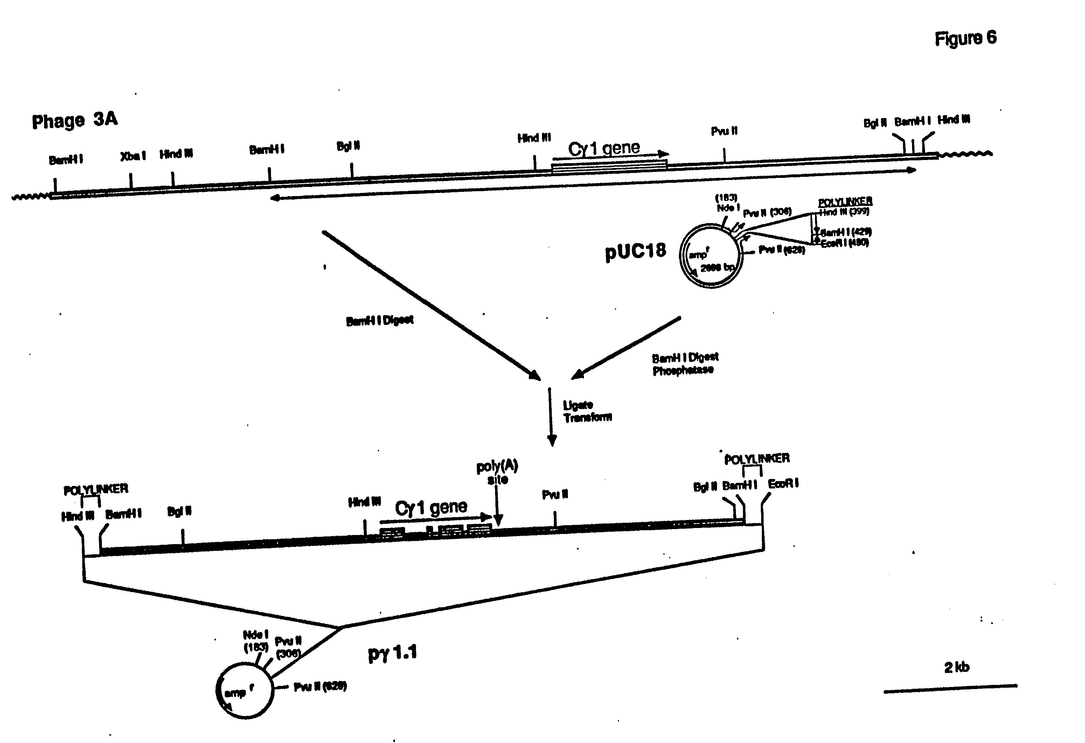

Other heavy chain cassettes containing a human γ1 heavy chain constant region gene were also constructed. According to this embodiment, a phage clone, Phage 3A (Ellison et al., 1982, Nucleic Acids Research, 10: 4071-4079) was used as a source of the germline Cγ1 gene (see Figure 6). This clone was obtained in an identical manner to Phage 5A, described above.

Three different γ1 cassettes were made: a) pNγ1.1, which contains extra sequences both 5′ and 3′ of the coding region of the γ1 constant region gene, b) pNγ1.2, which lacks the extra sequences 5′ of the gene and c) pNγ1.3, which lacks the sequences both 5′ and 3′ of the gene (see Figures 10-12). Briefly, the γ1 cassettes were made by first subcloning the appropriate sequences of the Cγ1 gene into pUC and pIC vectors adjacent to the polylinker regions of those vectors to produce: pγ1.1, pγ1.3 and pγ1.4, respectively (see Figures 6-9). (pγ1.2 was produced from pγ1.1 as an intermediate vector and was used in the production of pγ1.3 and pγ1.4). The Cγ1 sequences were then removed from these vectors along with the desired polylinker sequences and inserted into pN.1 (described above) to produce pNγ1.1 or into pN.2 (described above) to produce pNγ1.2 or pNγ1.3. The detailed procedures used to construct these cassettes were as follows:

### i. pγ1.1 (Fig. 6)

2 μg of Phage 3A DNA were digested to completion with BamH I, PCI extracted, ethanol precipitated and resuspended in 20 μl TE (0.1 mg/ml). 10 μg of pUC18 were digested to completion with BamH I, PCI extracted, ethanol precipitated and resuspended in 44 μl $H_2O$. The DNA was then treated with CIP (28 units from Boehringer-Mannheim), PCI extracted, ethanol precipitated and resuspended in TE to 50 ng/μl. 200 ng of the BamH I digested phage DNA was then ligated to 200 ng of the BamH I digested and phosphatased pUC18 with 400 units of T4 DNA ligase (New England Biolabs) for 15 min. at room temperature in ligation buffer in a final volume of 20 μl. The reaction was then diluted to 80 μl with ligation buffer lacking PEG. 160 units of T4 DNA ligase (New England Biolabs) was added and the mixture incubated overnight at 15° C. 5 μl of the ligation mixture was then used to transform 200 μl of competent JM83 cells. The transformed bacteria were plated in LB-agar containing ampicillin (100 μg/ml) and X-gal as a color indicator. White colonies were picked and streaked onto a plate containing LB-agar and ampicillin. These colonies were grown and then screened by colony hybridization, using a nick-translated γ3 probe, an EcoR I-Hind III insert derived from p3.6RH4.2 described by Krawinkel, supra and Flanagan and Rabbits, 1982, Nature, 300:709-713.

DNA was prepared from those colonies which hybridized with the probe. The DNA was digested with BamH I, electrophoresed, photographed and transferred to nitrocellulose and hybridized with the same γ3 probe. DNA containing a 10.5 kb insert was detected and the resulting vector was designated pγ1.1.

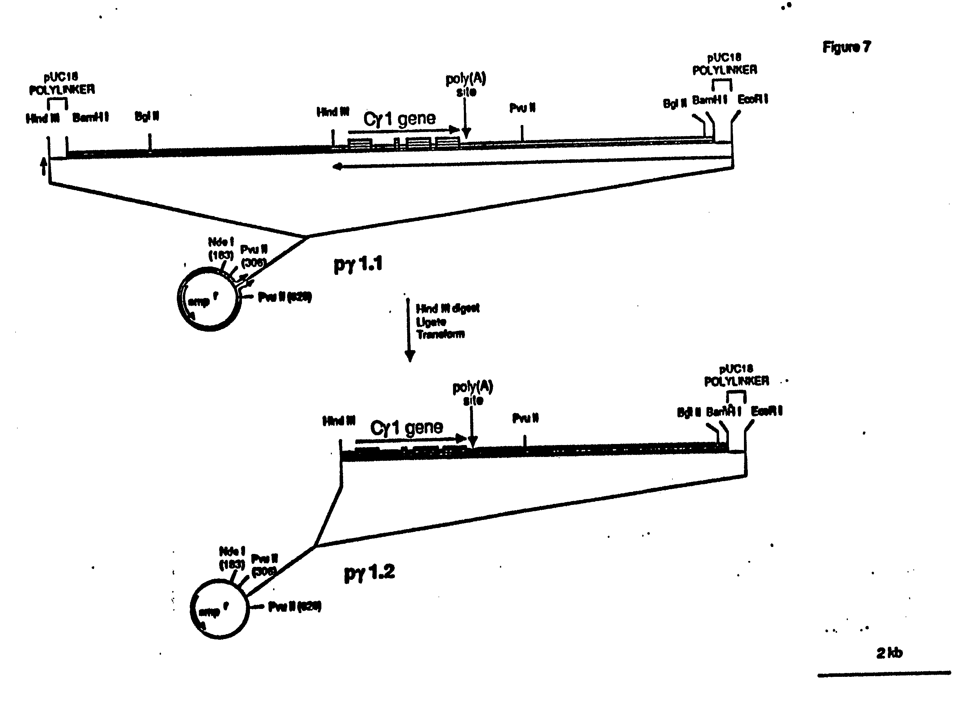

### ii. pγ1.2 (Fig. 7)

pγ1.1 was digested to completion with Hind III and 250 ng of the DNA was self-ligated using 1 μl of T4 DNA ligase (Boehringer-Mannheim) for 30 min. at 37° C. The volume was brought to 400 μl with TE and 5 μl was used to transform 50 μl of competent DH5α cells. The transformed bacteria were plated on LB-agar plates containing ampicillin at 100 μg/ml. 12 colonies were grown up overnight in LB broth containing 1% glucose and 100 μg/ml ampicillin. DNA was prepared from the overnight growth and digested with Hind III to produce a linearized fragment of about 8 to 9 kb (2.7 kb pUC18 plus 5.3 to 6.3 kb γ1 sequences). The identity of the construct was further confirmed by digestion with Hind III and BamH I which produced fragments of about 5.8 and 2.7 kb and by digestion with Hind III and Pvu II which produced fragments of approximately 3.0, 2.8, and 2.2 kb and a smaller undetectable fragment.

This step served to delete from the pγ1.1 DNA 4 kb 5' of the coding region of the Cγ1 gene and containing the switch region. This intermediate vector was called pγ1.2.

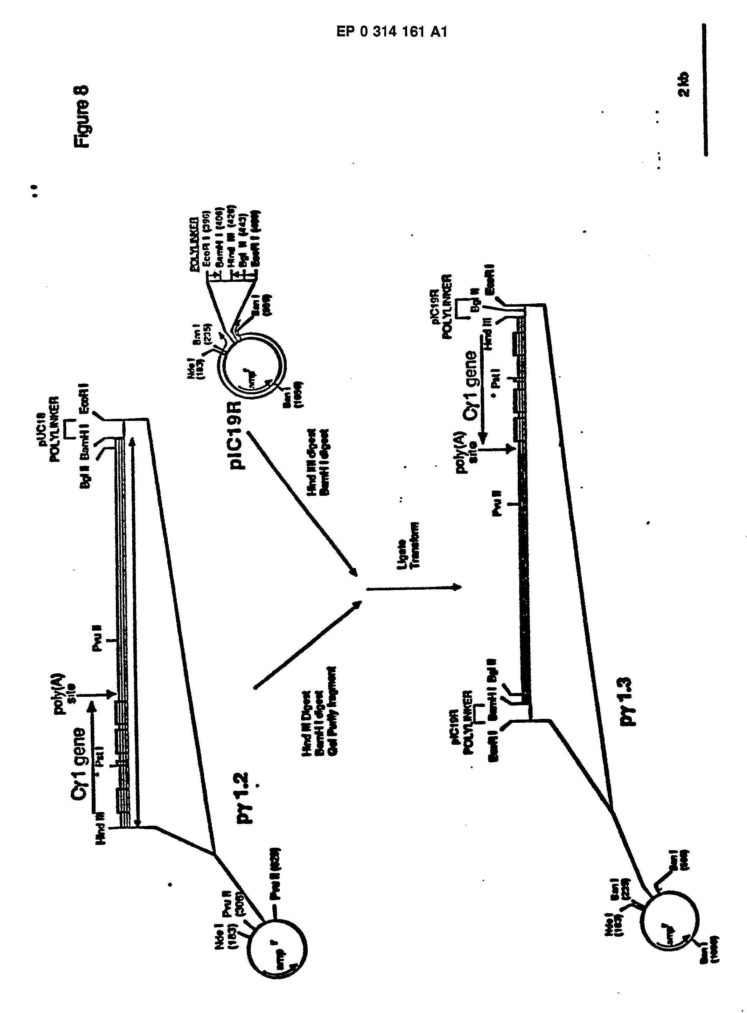

### iii. pγ1.3 (Fig. 8)

75 μg of pγ1.2 was next digested to completion with Hind III (300 units, 200 μl reaction volume). 3.75 μg of this DNA was then digested with 29 units of BamH I and the fragments were separated on a 0.8% low melt agarose gel as described. A 5.8 kb fragment was purified using GENECLEAN (5 μl Glassmilk) (BIO 101, CA) according to the manufacturer's instructions and resuspended in 50 μl TE. Gel electrophoresis showed the concentration to be approximately 50 ng/μl, giving a total recovery of approximately 2.5 μg DNA.

9 μg of pIC19R was digested with 30 units of BamH I and then 30 units of Hind III in a final volume of 60 μl. 250 ng of the γ1 insert were ligated with 50 ng of pIC19R DNA in a reaction volume of 20 μl using ligation buffer and 1 unit of T4 DNA ligase (Boehringer-Mannheim) for 20 min. at 37° C. 3 μl of the ligation mixture was then used to transform 100 μl of competent JM83 cells. The transformed bacteria were plated on LB-agar containing ampicillin (100 μg.ml). The presence of the Cγ1 insert in selected colonies was determined by digestion of the DNA with EcoR I, which released a 6 kb fragment. The orientation of the insert was then confirmed by digestion with Hind III and Pst I, which produced a 0.85 kb fragment showing that the 5' end of the Cγ1 gene was closest to the Bgl II site in the polylinker. This construct was named pγ1.3.

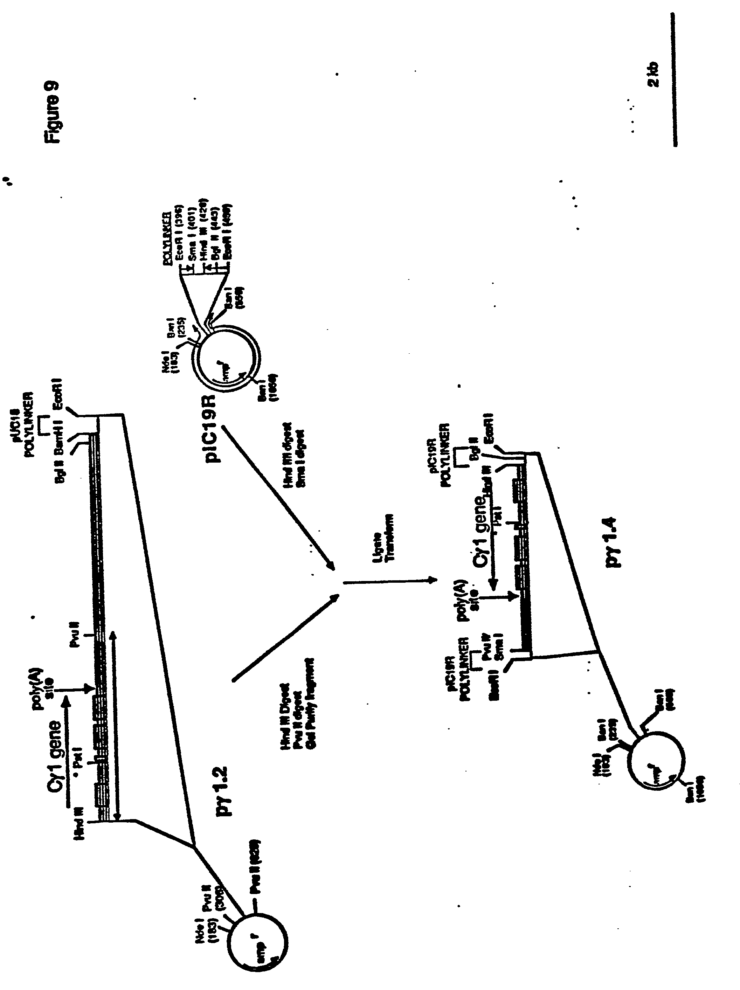

### iv. pγ1.4 (Fig. 9)

According to another embodiment, 75 μg of pγ1.2 was digested with Hind III as described in the section above. About 40 μg was further digested with Pvu II. The DNA was ethanol precipitated and resuspended in 200 μl TE. 2 μg of the DNA was further treated with CIP (1 μl from Boehringer-Mannheim) for 20 min. at 37°C in a reaction volume of 25 μl. The volume was then increased to 50 μl, an additional 1 μl of CIP was added and the reaction was incubated for 30 min. at 56°C. 50 μl of TE was added and the reaction was phenol extracted and ethanol precipitated. The DNA was resuspended in 25 μl TE, giving a concentration of between 0.05 and 0.1 μg/μl. The DNA was then separated on a 0.8% low melt agarose gel as described. Two upper bands of approximately 3.0 and 2.8 kb were separately excised and then purified using GENECLEAN (5 μl Glassmilk) according to the manufacturer's instructions. The DNA was resuspended in 50 μl TE to a final concentration of approximately 30 ng/μl.

9 μg of pIC19R was digested first with 30 units of Sma I and then with 30 units of Hind III in a final volume of 60 μl. 210 ng of the Hind III-Pvu II γ1 insert isolated as described in the paragraph above was then ligated with 50 ng pIC19R in a 20 μl reaction volume containing ligation buffer with PEG and 1 unit of T4 DNA ligase (Boehringer-Mannheim) for 20 min. at 37°C. 3 μl of the ligation mixture was used to transform 100 μl of competent JM83 cells and the transformed bacteria were plated on LB-agar containing 100 μg/ml ampicillin.

DNA was prepared from overnight growths of transformants and digested with EcoR I. Inserts of both 3.0 and 2.8 kb were seen, corresponding to the two bands excised from the low melt agarose gel. In certain transformants, the insert was shown to be γ1 by digestion with Pst I and with Hind III plus Pst I. The transformants containing the γ1 gene produced Pst I fragments of 3.7 and 1.85 kb, while Pst I plus Hind III digestion produced fragments of 2.85, 1.85, and 0.87 kb. The DNA construct of these transformants was named pγ1.4.

The next step in the construction of the Cγ1 heavy chain cassettes of this embodiment involved removal of the Cγ1 sequences from the vectors just described and their insertion into vectors, pN.1 or pN.2, described earlier in Example I, A.1.b, to form the heavy chain cassettes: pNγ1.1, pNγ1.2, and pNγ1.3.

### v. pNγ1.1 (Fig. 10)

2.4 μg of pN.1 was digested with 99 units of BamH I, phenol extracted, ethanol precipitated and resuspended in 50 μl Te (giving 0.05 μg/μl). pγ1.1 was also digested with BamH I. Approximately 1 μg of the pγ1.1 digest was combined with 100 ng of the pN.1 digest in ligation buffer in a volume of 98 μl and heated to 65°C for 5 min. 2 μl of T4 DNA ligase (New England Biolabs) was added and the sample was incubated for 30 min. at room temperature followed by overnight incubation at 14°C. 10-50 μl of the ligation reaction were used to transform 200 μl of competent DH5α cells. The bacteria were plated on LB-agar containing 100 μg/ml ampicillin and 200 μg/ml kanamycin. Transformants were screened for the presence of plasmid DNA containing a 9-11 kb BamH I insert. Transformants containing an approximately 9.6 kb insert in pN.1 were selected. The DNA was also digested with Hind III and Bgl II to confirm the presence of the γ1 insert and to determine its orientation. As expected, Hind III linearized the DNA. Bgl II digestion produced fragments of 2.1 kb and approximately 10 kb. This is consistent with the γ1 gene being in the opposite orientation to the neo gene if we postulate a deletion including the Bgl II site 5' of the γ1 coding region (see Figure 10, wherein the deleted Bgl II site is shown in parentheses). Hind III plus Bgl II digestion produced bands of 2.1 and a doublet at about 6 kb. This is consistent with the orientation and deletion which we believe occurred. The vector isolated from these transformants was designated pNγ1.1.

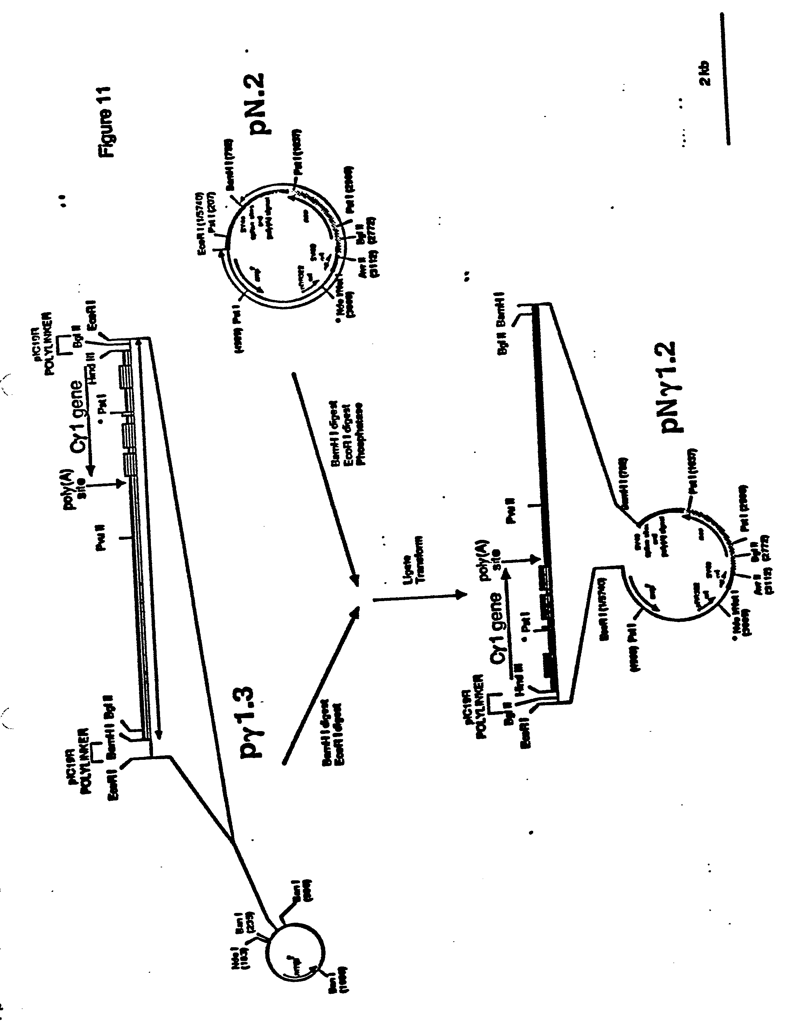

### vi. pNγ1.2 (Fig. 11)

2 μg of pγ1.3 was digested with 44 units of BamH I and then 20 units of EcoR I. 10 μg of pN.2 was digested with 20 units of BamH I and 20 units of EcoR I, ethanol precipitated, and resuspended in 22 μl TE. The DNA was phosphatased using 1 μl CIP (Boehringer-Mannheim) at 56°C for 30 min. The DNA was phenol extracted, ethanol precipitated and resuspended in 100 μl TE.

50 ng of pN.2 was combined with 250 ng of pγ1.3 and ligated with 1 unit T4 DNA ligase (Boehringer-Mannheim) in ligation buffer to a final reaction volume of 20 μl for 25 min. at 37 °C. 15 μl of the ligation mixture was used to transform 100 μl competent DH5α cells. The bacteria were plated on LB-agar containing both ampicillin (100 μg/ml) and kanamycin (200 μg/ml). Transformants were screened by digesting the plasmid DNA with EcoR I and screening for a linearized DNA of approximately 11 kb. The γ1 gene could only have been inserted in an orientation opposite to that of the neo gene. The vector isolated from such transformants was termed pNγ1.2.

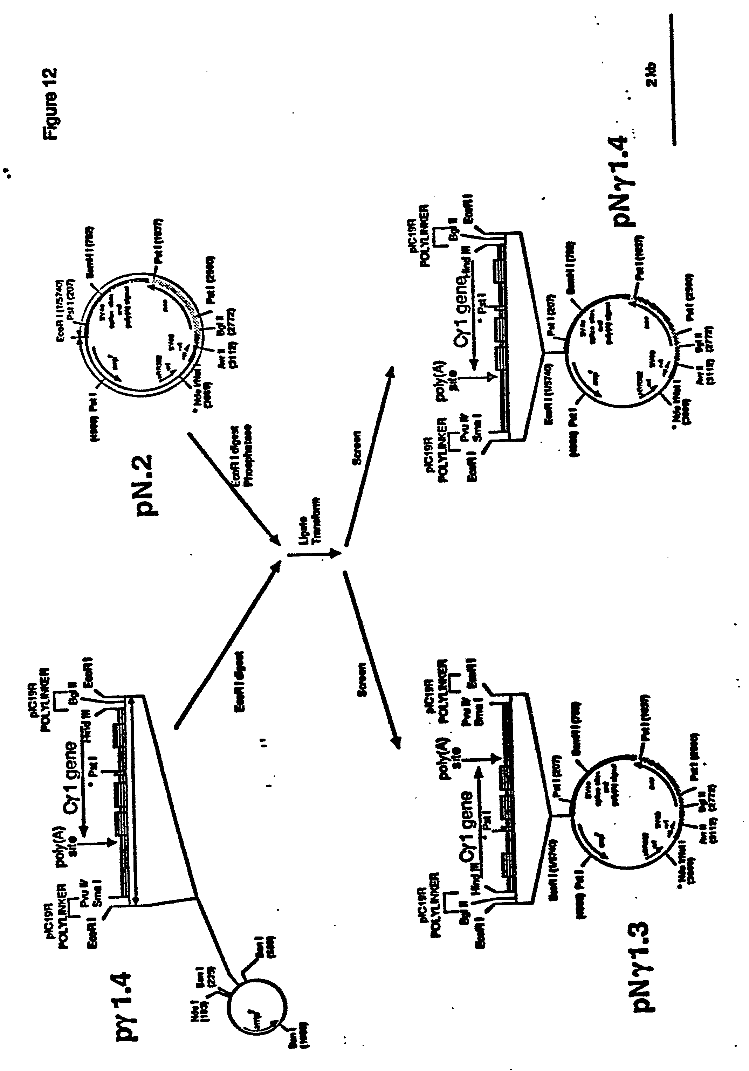

vii. pNγ1.3 (Fig. 12)

2 μg of pγ1.4 and 10 μg of pN.2 were each digested with 20 units of EcoR I. The pN.2 digest was ethanol precipitated, resuspended in 2 μl TE and phosphatased with 1 μl CIP (Boehringer-Mannheim) for 30 min. at 56 °C. The DNA was then phenol extracted, ethanol precipitated and resuspended in 100 μl TE. 50 ng of digested pN.2 was combined with 250 ng of digested pγ1.4 and ligated with 1 unit T4 DNA ligase (Boehringer-Mannheim) in ligation buffer to a final reaction volume of 20 μl for 25 min. at 37 °C. 15 μl of the ligation mixture was used to transform competent DH5α cells. The bacteria were plated on LB-agar containing both ampicillin (100 μg/ml) and kanamycin (200 μg/ml). Transformants were screened by digesting the plasmid DNA with EcoR I for the presence of an approximately 3 kb insert. The orientation of the insert was then determined by digestion with Pst I. The vector containing the γ1 gene in the opposite orientation to the neo gene was called pNγ1.3. (The vector containing the γ1 gene in the same orientation as the neo gene was called pNγ1.4).

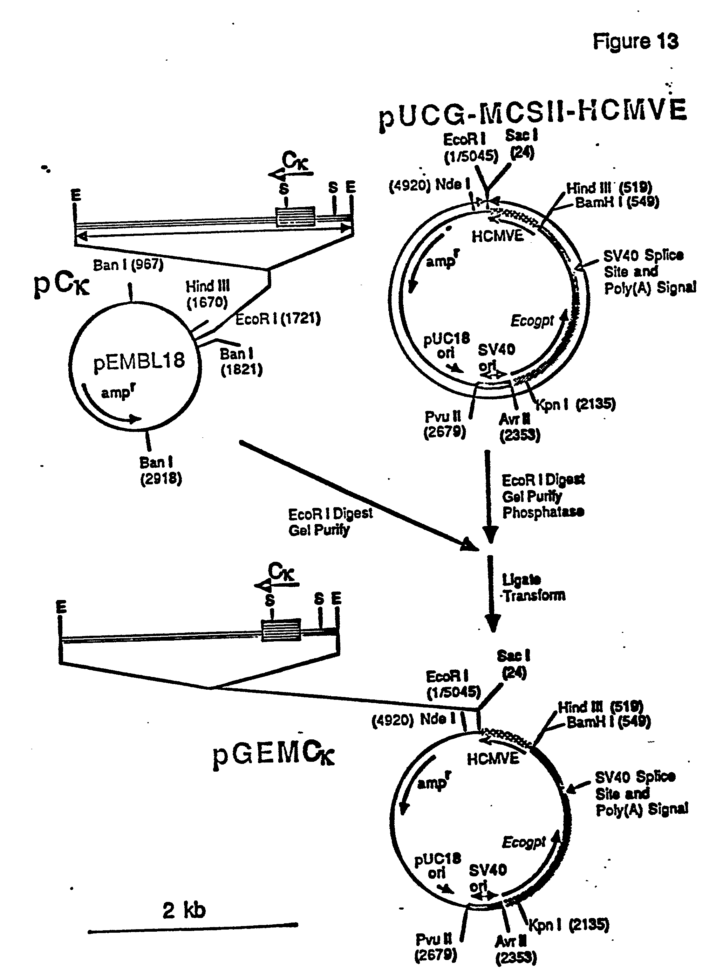

3. Kappa Light Chain Cassettes (Fig. 13)

The human kappa constant region gene Cx was inserted into the pUCG-MCSII-HCMVE vector as an EcoR I fragment into the vector's EcoR I site, and in the same orientation as the gpt gene.

Approximately 1.5 μg pUCG-MCSII-HCMVE were digested with EcoR I (15 units, overnight digestion). The DNA was electrophoresed through agarose and the linearized fragment collected into NA45 DEAE paper, then eluted as previously described. Following ethanol precipitation, the DNA was resuspended in 20 μl TE buffer and treated with CIP as described above.

pCx is a pEMBL 18 based plasmid containing the 2.7 kb Eco RI fragment (containing the Cx gene) derived from the x phage clone described by Hieter, 1980, Cell, 22:197-207. pCx was digested with EcoR I, the DNA electrophoresed through agarose, and the Cx insert collected into NA45 DEAE paper. The DNA was eluted and further purified by passage through a "spun-column." Approximately 120 ng Cx EcoR I insert was ligated with approximately 10 ng pUCG-MCSII-HCMVE linearized with EcoR I (ligation buffer lacking spermidine). A portion of the ligation mixture was diluted 1:5 with TCM and used to transform 100 μl competent DH5α cells. Plasmid DNA was prepared from ampicillin resistant colonies. The DNA was screened for the desired insert (Cx) by EcoR I digestion. A single colony was identified as containing the desired insert; however, Sac I digestion showed that the insert was not in the desired orientation. The plasmid DNA (200 ng) was therefore digested with EcoR I and ligated (25 μl reaction volume, 70 units T4 DNA ligase (New England Biolabs) in ligation buffer lacking both PEG and spermidine, 15 °C overnight). A portion of the ligation mixture was diluted with TCM and used to transform competent cells as described above. Due to the high background of transformants containing vector without the Cx insert, the ampicillin resistant colonies were screened by colony hybridization with purified Cx insert (2 hr. hybridization with 5 X 10⁶ cpm/ml; filters washed twice for 30 minutes at 65 °C in 0.1XSSC, 0.1% SDS; film exposed 2 hours). DNA from 24 of the hybridizing colonies was screened by Sac I digestion for the desired orientation. The desired plasmid was designated pGEMCx.

B. Heavy Chain Gene Constructs

1. Heavy Chain Variable Region Gene Cloning

Both rearranged heavy chain alleles from each cell line were cloned and each inserted into a heavy chain cassette (e.g., pGγ2, pNγ2 or pNγ1.1-.3) in the appropriate orientation; it was then determined by

26

transfection which one was translated into a polypeptide chain which combined with the appropriate light chain to ultimately bind antigen.

## a. Library Constructions and Screening

In this Example, Southern Transfer analysis with the human $J_H$ region probe $pJ_H$ (Figure 14), was used to determine the size of Hind III fragments of DNA from cell lines 4B9 and 9D1 that would contain rearranged heavy chain VDJ genes. Furthermore, it was important to determine that the Hind III fragments were of sufficient size to contain the complete variable region gene as well as sufficient upstream sequences to promote adequate levels of transcription. This was determined by measuring the distance from the Hind III site in the $J_H$-$C\mu$ intron to the 5' most $J_H$ element, and adding approximately 2kb for L-V and promoter sequences.

Bacteriophage libraries were made from Hind III digested and size-fractionated genomic DNA from cell lines 4B9 and 9D1. The libraries were screened with the J region probe, DNA was prepared from the positive plaques and the variable region genes were then subcloned into pUC18 to facilitate mapping of restriction sites and identification of duplicate clones. The variable region genes of 4B9 were then inserted into the pG$\gamma$2 cassette or the pN$\gamma$1.1-.3 cassettes and the variable region genes of 9D1 inserted into the pN$\gamma$2 cassette, both described above, to form expressible heavy chain genes.

### i. Vector Construction ($\lambda$L47.1)

In this experiment, the bacteriophage $\lambda$L47.1 was used as a vector. E. coli strains LE 392 (ATCC number 33572) and 803 supF (obtained from Dr. N. Hozumi, Mt. Sinai Hospital, Toronto, Ontario) were used as hosts for bacteriophage growth. $\lambda$L47.1 is described in Maniatis et al., Molecular Cloning, A Laboratory Manual, p. 41 and Loenen and Brammer, 1980, Gene, 10:249. To use $\lambda$L47.1 as a vector the middle "stuffer" fragment was removed, thereby allowing for the incorporation of foreign DNA. Briefly, this was accomplished by ligating the cohesive ends of the vector, digesting the vector with an appropriate restriction endonuclease that removed the stuffer, and then separating the bacteriophage arms from the stuffer piece by centrifugation through a sucrose gradient.

The experimental procedure was as follows. The cohesive ends of 100 $\mu$g $\lambda$L47.1 DNA were annealed by incubation in buffer containing 10 mM $MgCl_2$ and 150 mM Tris, pH 8.0, for 1 hour at 42°C. The annealed ends were then ligated and the ligase then heat inactivated. For the preparation of Hind III arms, the vector DNA was then digested to completion with both Hind III and BamH I. The latter enzyme served to further digest the stuffer piece such that its ends (BamH I) were no longer compatible with those of the vector arms (Hind III). The vector was then treated with bovine alkaline phosphatase so that the bacteriophage arms could not ligate to each other. The DNA was then phenol and chloroform extracted, ethanol precipitated and resuspended in TE buffer.

Fifty micrograms of the digested bacteriophage DNA were then layered onto a 40 ml linear 10-40% sucrose gradient in 20 mM Tris, pH 8.0, 1.0 M NaCl, 2.5 mM EDTA. The tubes were centrifuged at 26,000 rpm for 20 hours at 20°C in a SW28 rotor (Beckman). The gradient was tapped from the bottom and fractions containing vector arms, but no stuffer piece, were identified by electrophoresing a small sample through an agarose gel. DNA was recovered from these fractions by ethanol precipitation and resuspension in TE buffer.

In some instances an alternate but less efficient procedure was utilized. Briefly, bacteriophage arms were prepared by digesting $\lambda$L47.1 DNA with either EcoR I or Hind III, annealing of the cos sites, separating by sucrose gradient centrifugation, and pooling of fractions which contained phage arms but not the stuffer fragment. The phage arms gave a high background, so the DNA was further digested with BamH I to cut any contaminating stuffer fragment. This significantly reduced the background seen during ligation and packaging without the addition of any insert DNA.

### ii. Genomic DNA Extractions

Total high molecular weight DNA was purified from 1-2 x $10^8$ cells of 4B9 or 9D1 essentially according to the procedure of Perucho et al., 1981, Cell, 27:467-76. Briefly, the cells were washed by centrifugation in PBS (phosphate buffered saline) or TBS (Tris buffered saline) and then lysed in a Tris buffer containing SDS, EDTA and proteinase K. Following proteinase digestion, the DNA was extracted with phenol and chloroform, ethanol precipitated, and resuspended in TE. The sample was then digested with RNAse A, and again extracted with phenol and chloroform, ethanol precipitated and resuspended in TE. The procedure is described below in more detail.

Approximately $1\text{-}2 \times 10^8$ 4B9 or 9D1 cells were obtained from cultures and centrifuged at 1,000 x g for 10 min. The cells were resuspended in 50 ml TBS (8 g NaCl, 0.38 g KCl, 3.0 g Tris base per liter, and 1 N HCl to pH 7.4), or PBS, centrifuged, resuspended, and centrifuged again. After the final centrifugation, the cells were resuspended in a small amount of PBS or TBS. In some cases, they were stored at -20°C. For each $10^8$ cells, 5 ml of lysis buffer (10 mM Tris, pH 8, 50 mM EDTA, 150 mM NaCl) containing 200 $\mu$g/ml Proteinase K (Boehringer Manheim) was added. This volume is henceforth considered to be one volume. A one-fortieth volume of 20% SDS was added, the tube quickly mixed and then incubated overnight at 37°C. One volume of 2X phenol extraction buffer (10 mM Tris, pH 8, 12 mM EDTA, 650 mM NaCl) was then added, followed by two volumes of phenol (equilibrated with Tris to neutral pH) containing 0.1% 8-OH quinoline. The tubes were covered, mixed gently for 5 to 10 minutes, then centrifuged at 2500 rpm for 20 minutes. The aqueous phase was transferred to a fresh tube and the phenol extraction repeated 1 to 3 times until the interface had disappeared. The aqueous phase was again transferred to a fresh tube and two volumes of PCI added. The tube was mixed, centrifuged, and the aqueous phase transferred to a fresh tube. The PCI extraction was repeated 1 to 3 times until the interface had disappeared. The aqueous phase was again transferred to a fresh tube and extracted twice with chloroform, isoamyl alcohol (24:1). Finally, the aqueous phase was transferred to a fresh tube and gently overlaid with four volumes of ethanol. The tube was gently mixed by inversion until the DNA had formed a tight clot. The DNA was removed with a glass rod and rinsed sequentially with 70% ethanol in ethanol dilution buffer (10 mM Tris, pH 8, 10 mM EDTA, 150 mM NaCl), 85% ethanol and 100% ethanol. The DNA was dried by absorption of the ethanol on a paper towel and resuspended in a few ml TE buffer overnight at 37°C. This volume is henceforth considered to be one volume.

One volume of 2X RNAse A buffer (100 mM Tris, pH 8, 20 mM EDTA, 20 mM NaCl) was added, followed by RNAse A (Sigma, #R-5125) to 100 $\mu$g/ml (the RNAse A had previously been boiled for 30 minutes to remove trace DNAse activity). This mixture was incubated for 3 hours at 37°C. One-fifth volume of 3 M sodium acetate was added, the DNA extracted twice with phenol/chloroform, precipitated with ethanol and resuspended in TE as described above.

As a source of germline DNA, high molecular weight DNA was prepared from cells of human placenta. Connective tissue was removed from about 5 grams of the placental tissue; the tissue was minced with a scalpel and then rinsed three times by centrifugation and resuspension in TBS, each at 1,000 x g at 4°C for 10 minutes each. The tissue (3.5 g) was resuspended in 20 ml TBS, homogenized in a motor driven glass-teflon homogenizer, centrifuged again for 10 minutes at 1,000 x g, and resuspended in 3.5 ml TE. This formed a very thick suspension. Lysis buffer was added (0.5 M EDTA, 0.5% Sarkosyl, and 100 $\mu$g/ml Proteinase K) and incubated at 50°C for 1 hour, then overnight at 37°C. The remainder of the procedure was as described by Maniatis et al., 1982, Molecular Cloning, A Laboratory Manual, page 280, which is incorporated by reference herein, except that following RNAse A digestion and prior to extraction with phenol, the sample was again digested with Proteinase K (100 $\mu$g/ml) overnight at 37°C. The final concentration of placental DNA was 0.15 mg/ml.

### iii. DNA Hybridization Probes

All DNA hybridization probes described herein were used as inserts isolated from either phage or plasmid vector DNA and were labeled by nick translation using a kit provided by Bethesda Research Labs (Nick Translation Reagent Kit, cat. no. 8160SB) and $^{32}$P-dCTP from New England Nuclear or Amersham. Typically, the probes had specific activities of $1\text{-}2 \times 10^8$ cpm/$\mu$g. Prior to hybridization, the probe was denatured for 10 minutes at about 95°C, quickly chilled on ice, then added to the hybridization mix. Unless otherwise noted, hybridizations were carried out as follows: filters were prehybridized in a basic mix (50% formamide, 5X SSC, 5X Denhardt's solution, 50 $\mu$g/ml sheared salmon sperm DNA, 50 mM Hepes, pH 6.8, 5 mM EDTA, and 10 $\mu$g/ml poly (A)) supplemented with 1% glycine and 0.1% SDS for at least 2 hours in sealed plastic bags in a 42°C water bath. The hybridization buffer varied according to the type of hybridization; for genomic Southern Transfers, the basic mix was supplemented with 10% dextran sulfate, 0.2% SDS, and $10^6$ cpm/ml probe; for Southern transfer of cloned DNA, the basic mix was supplemented with 0.2% SDS and $10^5$ cpm/ml probe; for first screens of phage libraries, the basic mix was supplemented with 0.2% SDS and $10^6$ cpm/ml probe, while subsequent screens frequently had less probe. Hybridizations were normally performed overnight at 42°C. Following the hybridization, filters were first washed in 2X SSC, 0.1% SDS at room temperature and then in 0.1X SSC, 0.1% SDS at 65°C. Filters were exposed to X-ray film with intensifying screens at -70°C for varying times. When the signal was obviously very strong the intensifying screens were omitted and the film was exposed at room temperature.

### iv. Preparation of 4B9 and 9D1 Genomic DNA Inserts

One hundred-twenty micrograms of DNA from LCL 4B9 or 9D1 prepared as described above and 10 $\mu$g of human placental DNA were separately digested with Hind III and 7.5 $\mu$g of each were fractionated by electrophoresis through a 1.1% agarose gel in TAE buffer. The DNA was transferred to nitrocellulose paper using standard procedures, baked overnight at 80°C in a non-vacuum oven and hybridized with a $^{32}$P-labeled human heavy chain J region probe (pJ$_H$ insert). The probe insert (in pBR322) extends from an artificial EcoR I site near the 5' end of the J region to the Hind III site in the major intron between the J region and the mu constant region. The DNA sequence of the J region and part of the intron from a similar clone is described in Ravetch et al., 1981, Cell, 27:583-591 and in Rabbitts et al., 1983, Nature, 306: 806-809. As discussed above, since immunoglobulin genes must undergo a rearrangement in order to be expressed, DNA fragments were sought which were present in the 4B9 or 9D1 DNA but absent in the germline (placental) DNA. Either one or two rearranged bands were expected, depending on whether one or both alleles had undergone rearrangement, respectively. The pJ$_H$ probe hybridized to a 9 kb germline fragment in placental DNA, to 10.6 and 7.8 kb rearranged fragments of 4B9 DNA, and to 7.5 and 6.9 kb rearranged fragments of 9D1 DNA.

Fifty micrograms of the Hind III-digested 4B9 or 9D1 DNA was fractionated on a 10-40% sucrose gradient as described above for preparation of vector DNA arms. Fractions containing DNA of the desired size were screened by Southern transfer analysis with the $^{32}$P-labeled pJ$_H$ insert probe. In the case of 4B9, since the 10.6 and 7.8 kb fragments were easily separable, those fractions hybridizing most strongly were used for the library construction. In the case of 9D1, since the molecular weights of the two fragments were so close, the sucrose gradient fractionation did not completely separate the two fragments. However, fractions were chosen which were enriched for one fragment relative to the other.

### v. Combining Vector and Insert and Library Screening

Approximately 5-10% of the DNA in the peak fractions was ligated with the λL47.1 phage arms, prepared as described above, using T4 DNA ligase and ligation buffer. The ligation reaction was then packaged in vitro. Packaging extracts were prepared according to the procedure of Grosveld et al., 1981, Gene, 13:227-237, using a freeze-thaw lysate prepared from E. coli strain BHB 2688 (ATCC No. 35131) and a sonic extract prepared from strain BHB 2690 (ATCC No. 35132). Other procedures or commercial packaging extracts may be used. Titers of the bacteriophage were determined using the 803 supF strain of E. coli and were usually between 0.5 and 1.5 x 10$^5$ total plaque forming units (pfu) from each ligation reaction (corresponding to 5-10% of a sucrose gradient fraction).

Each library corresponding to a particular band seen on Southern transfer analysis consisted of at least 10$^5$ plaques (derived from one to three sucrose gradient fractions). The libraries were screened using $^{32}$P-labeled pJ$_H$ insert. Positive plaques were picked, diluted, plated, and screened using the same probe. This process was repeated (usually a total of 3 to 5 times) until all plaques on a plate were positive.

### 2. Subcloning V$_H$ Genes into Expression Cassettes

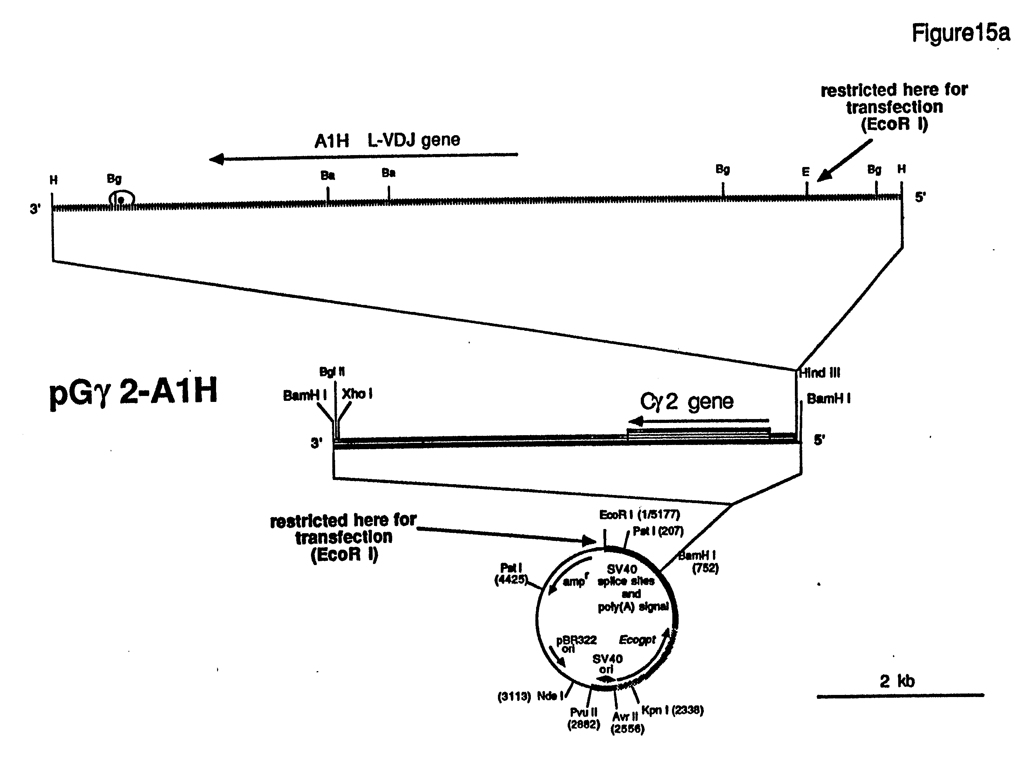

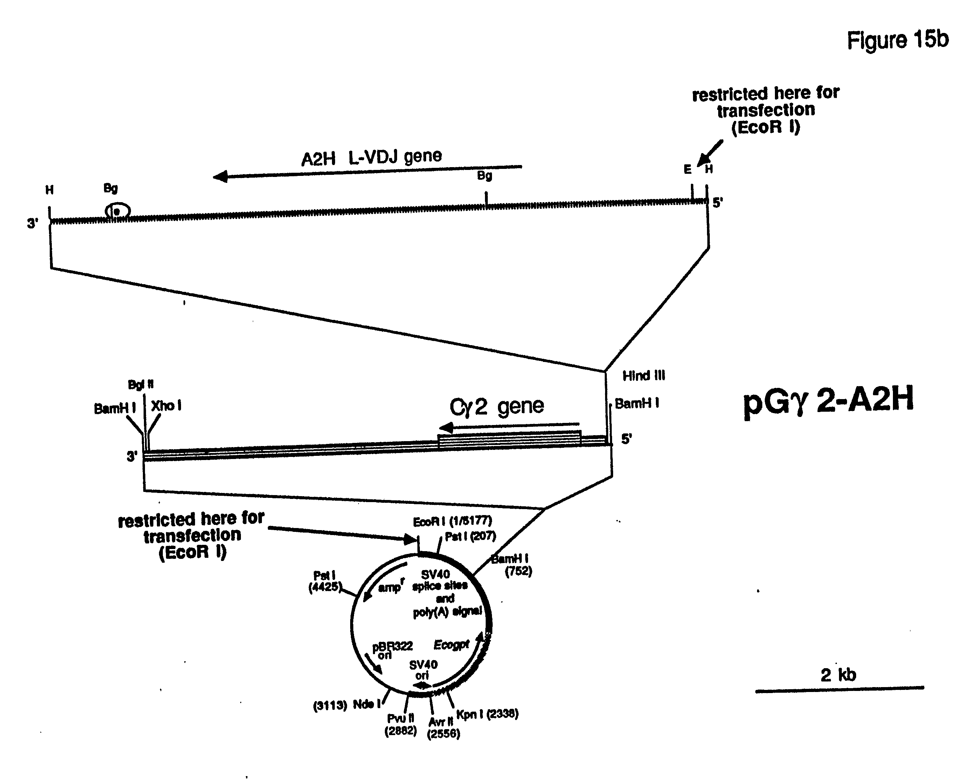

### a. 4B9 V$_H$ Genes into pG$\gamma$2 (Fig. 14 and 15a and b)

Plaques hybridizing to the pJ$_H$ probe were purified as described above. A single plaque was picked from the plaque pure plate, amplified, and used to inoculate a large phage culture. Phage DNA was purified according to the procedure of Maniatis et al., supra, pp. 77-85. Many of the phage clones contained multiple Hind III inserts. To facilitate mapping and subcloning into the pG$\gamma$2 vector, the rearranged variable region genes were subcloned into pUC18. To identify duplicate clones, the plasmid DNA was digested with Hind III and Bgl II and separated on agarose gels. Ethidium bromide staining and hybridization patterns (with pJ$_H$

probe) were compared. Two patterns were seen (Fig. 14): One corresponded to the 10.5 kb Hind III band and the other to the 7.8 kb band originally seen on the genomic Southern transfers. The former pattern was designated A1H and the latter A2H. Both patterns showed a characteristic 0.8 kb Hind III-Bgl II fragment hybridizing with the $pJ_H$ probe. This fragment corresponds to the 3' end of the clone, which is expected to be conserved within the human genome and not to undergo rearrangements.

The heavy chain variable region genes A1H and A2H were then purified from Hind III digests of the plasmids described above by electrophoresis through low melt agarose, as more fully explained below. The $pG\gamma2$ cassette was also digested with Hind III, treated with CIP, and then separated by electrophoresis through low melt agarose. The melted agarose slices containing the linearized cassette and variable region gene insert were combined, the DNA ligated, and then used to transform competent bacteria. Transformants were screened for those containing the V gene inserted in the same orientation as the $\gamma2$ gene in the $pG\gamma2$ vector. The details are as follows.

The $pG\gamma2$ vector was first prepared by digesting $pG\gamma2$ to completion with Hind III. Five micrograms of the DNA were then treated with 100 units of CIP and the reaction stopped by the addition of 1/10 volume of 0.5 M nitrilotriacetic acid, pH 8. The DNA was extracted with phenol and chloroform and precipitated with ethanol, then resuspended in 25 $\mu l$ TE buffer. 640 ng of DNA was separated on a 0.7% low melt agarose gel and subsequently recovered in a 400 $\mu l$ agarose slice, yielding a concentration of approximately 1.6 ng/$\mu l$.

To prepare the variable region gene insert, DNA obtained from subclones pA1H and pA2H was digested to completion with Hind III and approximately 2.5 $\mu g$ of each was separated on 0.7% low melt agarose gel in TAE buffer. The inserts containing the rearranged variable region genes were recovered in 400 $\mu l$ agarose slices. The concentrations of the DNA fragments were approximately 5 ng/$\mu l$ and 3.6 ng/$\mu l$ for A1H and A2H, respectively.

The vector and insert were combined as follows. Agarose slices containing the $pG\gamma2$ vector and the variable region insert (A1H and A2H) were melted at 70° C. Melted agarose containing 20 ng vector and 50 ng of insert were mixed at 37° C. Ligation buffer and T4 DNA ligase were added, bringing the final volume to 35 $\mu l$. The reaction was incubated for 30 minutes at 37° C, diluted 1:5 in TE buffer and 5 $\mu l$ was used to transform competent DH5$\alpha$ cells. The transformed bacteria were plated on LB agar containing 100 $\mu g/ml$ ampicillin.

To identify transformants containing the desired insert, the resulting colonies were inoculated into 2 ml of LB broth containing 1% glucose and 100 $\mu g/ml$ ampicillin and grown overnight at 37° C. DNA was prepared from the overnight growths and digested with Hind III to determine which samples contained the variable region insert in the $pG\gamma2$ vector. Orientation of the insert within the vector was determined by digestion with Bgl II and BamH I. Samples containing the variable and constant region genes in the same orientation were selected and designated $pG\gamma2$-A1H and $pG\gamma2$-A2H (Figs. 15a and 15b). Preparation of DNA for transfections is described in Example II.


### b. 4B9 $V_H$ Genes Into pN$\gamma$1 Cassettes

The 4B9 heavy chain variable region gene was inserted into the pN$\gamma$1 cassettes described in Example I,A.2.b above. Specifically, each vector (i.e., pN$\gamma$1.1, pN$\gamma$1.2, and pN$\gamma$1.3) was digested with Hind III and then treated with CIP. Hind III-digested phage A2H (as described above in section 2.a) was used as the source of the 4B9 variable region gene. Vector DNA was then mixed and ligated with phage DNA and the ligation mixture was used to transform competent DH5$\alpha$ cells. Transformants were screened for the presence of the variable region gene and the orientation of the gene was determined by various restriction enzyme digestions. Thus, constructs containing the 4B9 variable region gene adjacent to a $\gamma$1 constant region gene were prepared and designated: pN$\gamma$1A2.1, pN$\gamma$1A2.2 and pN$\gamma$1A2.3 (see Figures 16-18). The experimental procedures for the production of these constructs were as follows:


### i. pN$\gamma$1A2.1 (Fig. 16)

7.5 $\mu g$ of A2H phage DNA was digested with 80 units of Hind III, ethanol precipitated and resuspended in 150 $\mu l$ TE. 50 $\mu g$ of pN$\gamma$1.1 was digested with 200 units of Hind III, treated with CIP, ethanol precipitated and resuspended in 100 $\mu l$ TE (0.5 ug/$\mu l$). 500 ng of the A2H DNA was then ligated with 25 ng of pN$\gamma$1.1 DNA using 1 unit T4 DNA ligase (Boehringer-Mannheim) in a reaction volume of 20 $\mu l$ for 30 min. at 37° C. 5 $\mu l$ of the ligation mixutre was diluted with 30 $\mu l$ TE and used to transform 60 $\mu l$ competent DH5$\alpha$ cells.

The transformed cells were plated on LB-agar containing ampicillin at 100 μg/ml.

12 colonies were picked and DNA was prepared from them. Digestion of the DNA with Hind III showed the presence of an insert of the appropriate size (about 8.4 kb). Orientation was then determined by digestion with BamH I plus Bgl II. Transformants which produced bands of 2.0, 3.7, 6.1, 4.8 and 7.8 kb were selected. This is based on the absence of the Bgl II site 5′ of the γ1 gene. The resulting construct was termed pNγ1A2.1.

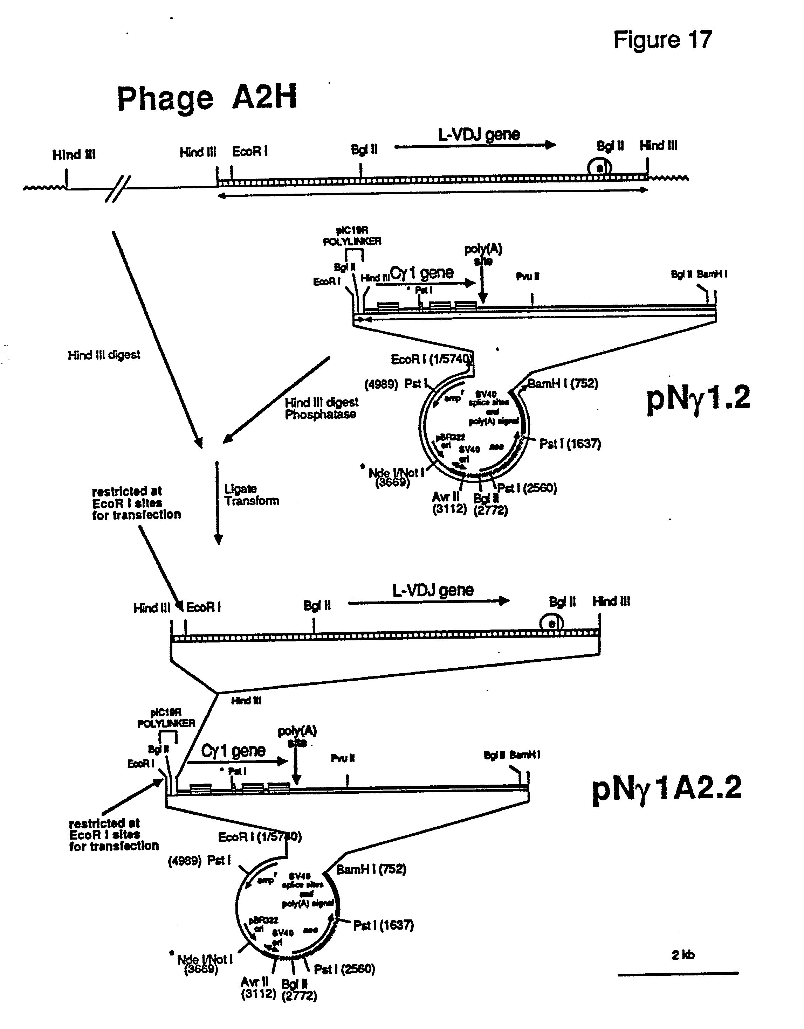

### ii. pNγ1A2.2 (Fig. 17)

15 μg of pNγ1.2 was digested with 22 units of Hind III and then phosphatased with 1 μl CIP for 20 min. at 56° C in a 30 μl reaction volume. The DNA was phenol extracted, ethanol precipitated and resuspended in 100 μl TE. A2H DNA was restricted with Hind III and treated as described in the section above. 50 ng of the restricted pNγ1.2 was ligated with 250 ng of the A2H DNA using 1 unit T4 DNA ligase (Boehringer-Mannheim) in ligation buffer in a reaction volume of 20 μl for 30 min. at 37° C or additionally, overnight at 4° C. 75 μl TCM was added to 15 μl of the ligation reaction and used to transform 200 μl of competent DH5α cells. The cells were plated on agar containing ampicillin and kanamycin as described earlier.

DNA was prepared from transformed colonies and digested with Hind III. Those samples showing the presence of the expected 8.4 kb Hind III insert (containing VDJ gene) were then screened for the orientation of the insert by digestions with EcoR I and Bgl II. As expected, Bgl II digestion produced bands of 2.8, 4.8, 7.2, 2.0, and 3.0 kb and EcoR I digestion produced bands of less than 0.5 kb and approximately 20 kb. The resulting construct was termed pNγ1A2.2.

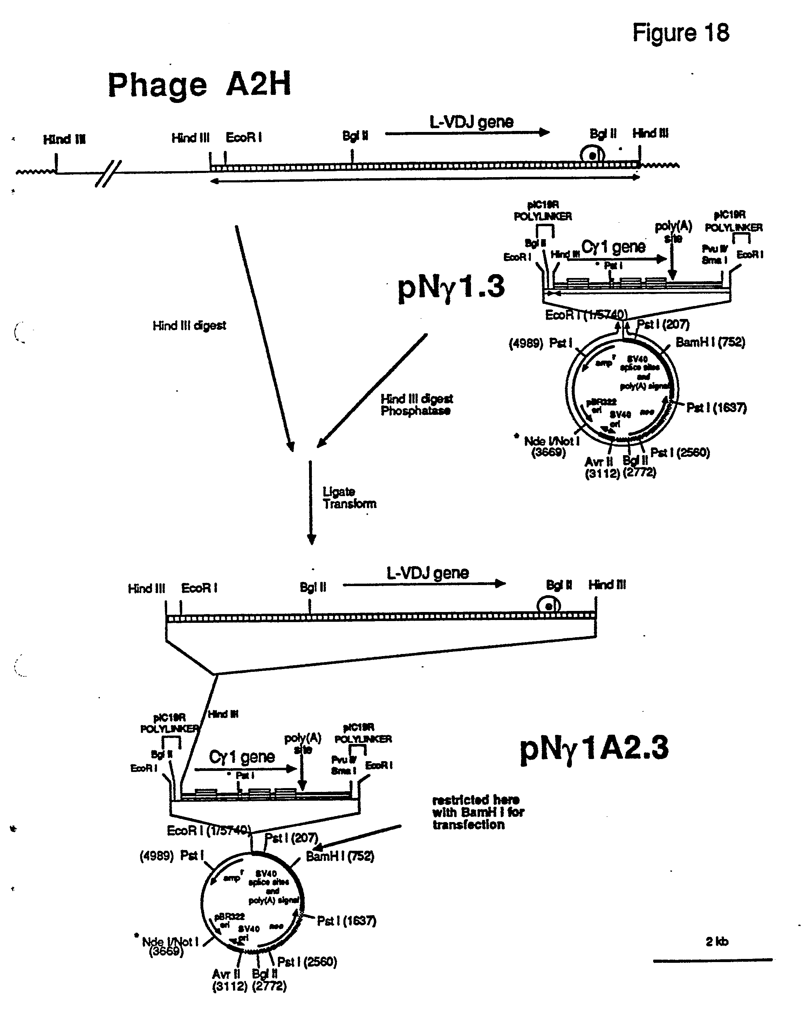

### iii. pNγ1A2.3 (Fig. 18)

40 μg of pNγ1.3 was digested with 33 units Hind III, ethanol precipitated and resuspended in 22 μl TE. The DNA was then phosphatased with 1 μl CIP for 30 min. at 56° C in a 25 μl reaction volume. The DNA was phenol extracted, ethanol precipitated and resuspended in 200 μl TE. A2H DNA was restricted with Hind III as described in the sections above. 100-200 ng of the restricted pNγ1.3 was ligated with 20 ng A2H DNA using 1 unit T4 DNA ligase (Boehringer-Mannheim) in ligation buffer to a reaction volume of 20 μl for 30 min. at 37° C or additionally, overnight at 4° C. 75 μl TCM was added to 15 μl of the ligation reaction and used to transform 200 μl of competent DH5α cells. The cells were plated on agar containing ampicillin and kanamycin as described.

DNA was prepared from transformed colonies and digested with Hind III. Those samples showing the presence of the expected 8.4 kb Hind III insert (containing VDJ gene) were then screened for the orientation of the insert by digestions with EcoR I and with Bgl II. As expected, Bgl II digestion produced bands of 2.8, 4.8, 6.6 and 3.0 kb and EcoR I digestion produced bands of 11 kb, 5.7 kb and less than 0.5 kb. The resulting construct was termed pNγ1A2.3.

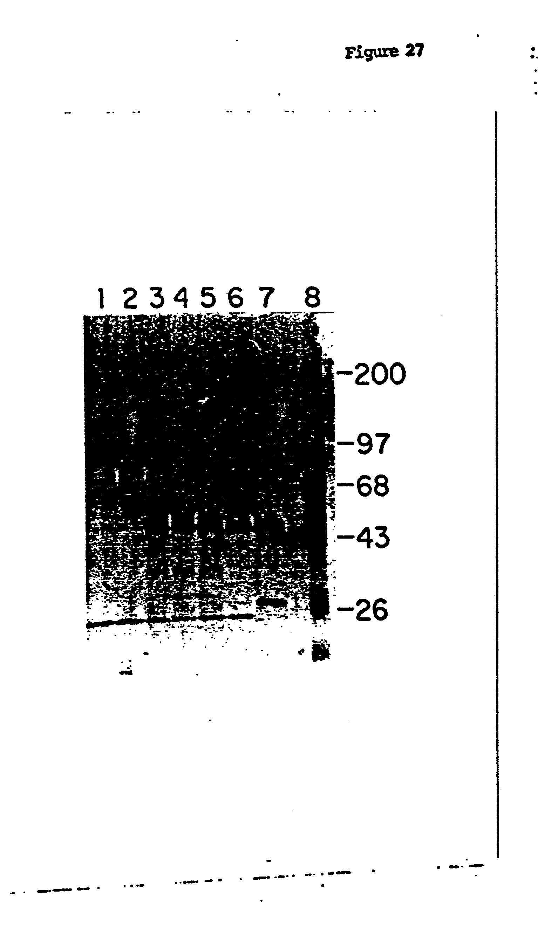

All three of these 4B9/γ1 constructs were used to transfect 4B9 light chain-producing mouse cells as described in Example IV, infra, which cells then expressed a heavy chain of the correct molecular weight, indicating that all three γ1 constructs were functional (see lanes 3-5 of Figure 27). Preparation of DNA for transfection is described in Example IV.

### c. 9D1 V Genes into pNγ2 (Figs. 19a and 19b)

Plaques hybridizing to the pJ_H probe were purified as described above. A single plaque was picked from each plaque pure plate, amplified, and DNA was extracted according to the procedures of Porteous et al., 1986, Analytical Biochem., 159:17-23 and Helms et al., 1985, DNA, 4:39-49. The variable region genes were then subcloned into pUC18 to facilitate mapping of restriction sites and identification of duplicate clones. Unexpectedly, Hind III and Bgl II digests showed that there were three rather than two patterns of restriction sites (see Figure 14). They were designated D1H (8 kb Hind III band), D2H (7.5 kb Hind III band), and DaH (8.4 kb Hind III band). The DaH pattern did not correspond to a genomic pattern and was therefore considered to have arisen as an artifact during the cloning procedures. All three patterns showed the characteristic 0.8 kb Hind III-Bgl II fragment. The Hind III fragments from the phage DNA were then inserted into the pNγ2 cassette, described above, to form expressible heavy chain genes, pNγ2-D1H and pNγ2-D2H. The experimental details are set forth below.

31

pNγ2 was digested to completion with Hind III and 2 μg of DNA were treated with 10-15 units CIP. The reaction was extracted with PCI, ethanol precipitated, and the DNA resuspended to 50 ng/μl in TE buffer. Separately, DNA from clones D1H and D2H was digested to completion with Hind III. The concentration of the DNA of each was approximately 50 ng/μl. The pNγ2 vector (50 ng) digest and phage DNA digests (D1H and D2H) (250 ng) containing the variable region insert were ligated using T4 DNA ligase in 20 μl ligation buffer for 30 min. at 37°C. The ligation reaction was diluted 1:19 in TE buffer and 95 μl was used to transform competent DH5α cells.

The transformed bacteria were plated on LB agar containing ampicillin at 100 μg/ml and kanamycin at 200 μg/ml. The resulting colonies were inoculated into 2 ml of LB broth containing 0.5% glucose and 100 μg/ml ampicillin, and grown overnight at 37°C. DNA was prepared from the overnight growths and digested with Hind III to determine which samples contained the variable region insert in the pNγ2 vector. Orientation of the insert within the vector was determined by digestion with Bgl II. Samples containing the variable and constant region genes in the same orientation were selected and were designated pNγ2-D1H and pNγ2-D2H (Fig. 19a and 19b). Preparation of DNA for transfections is described in Example VI.

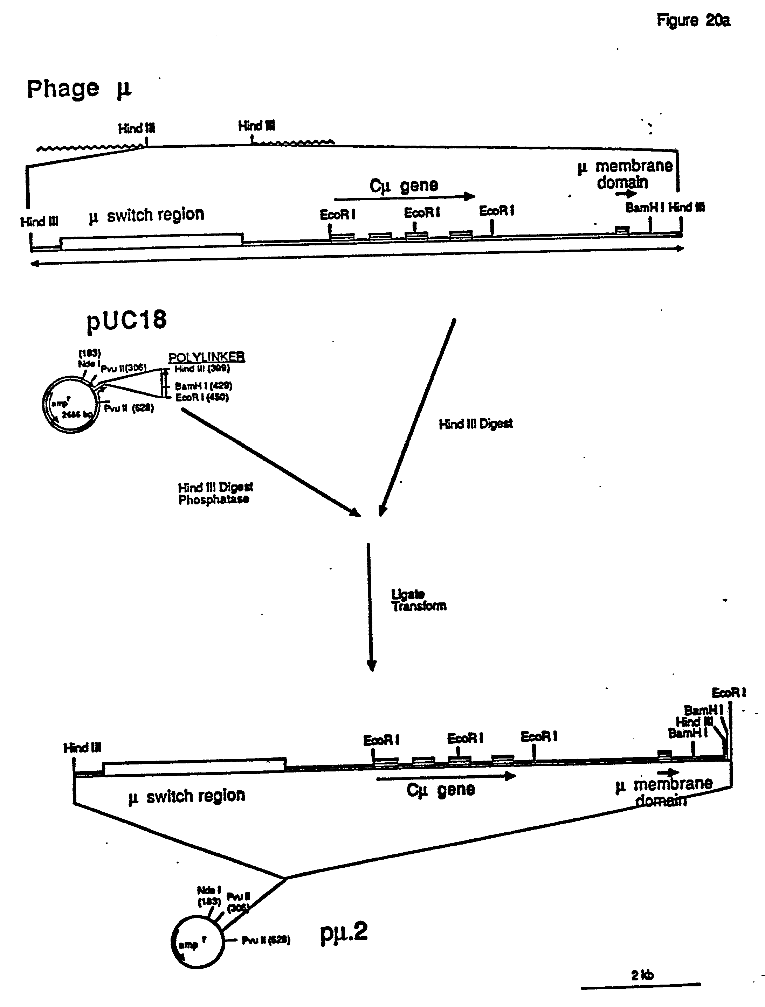

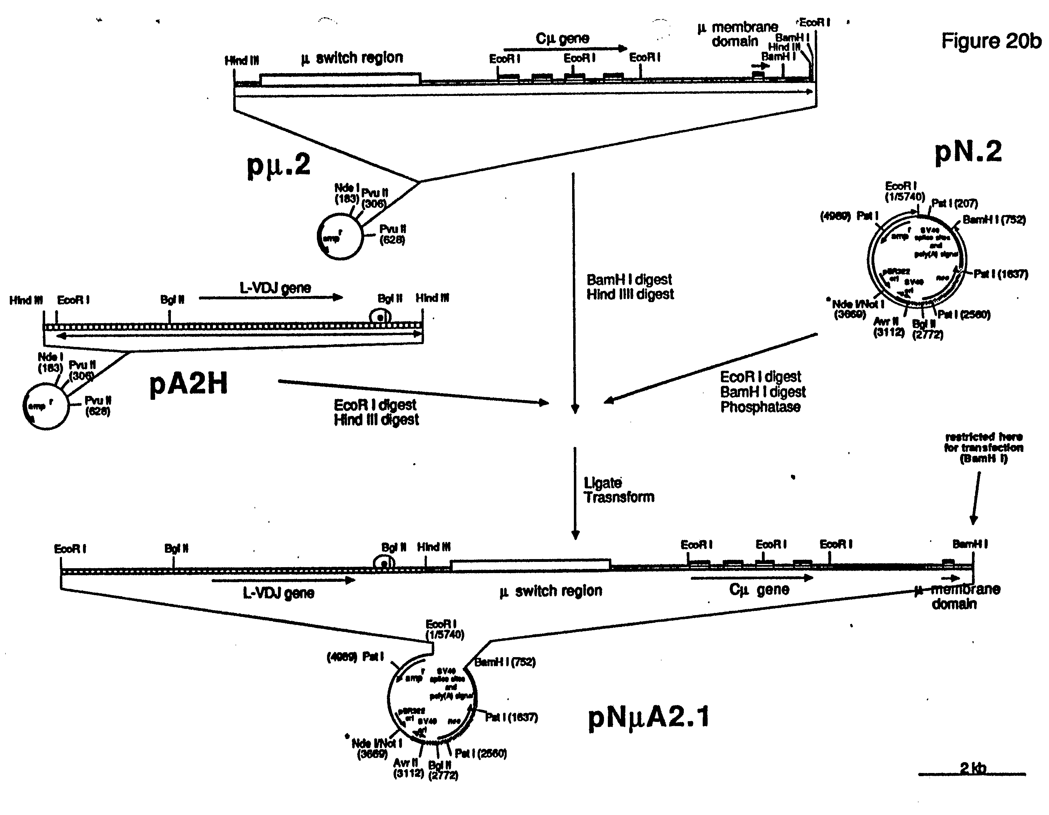

### 3. Preparation Of A 4B9 μ Heavy Chain Construct (Figs. 20a and 20b)

A μ heavy chain construct containing the variable region gene of the 4B9 antibody and a human μ constant region gene was also constructed according to this invention. A first step in this construction was the screening of a genomic library from human placental DNA with murine μ switch region and cDNA probes to obtain the germline μ constant region (Cμ) gene in a phage vector; the μ insert was then transferred to a plasmid vector to form pμ.2. The Cμ gene was then combined with the variable region gene of 4B9 contained in the pA2H vector (described earlier) by a 3-way ligation of pμ.2, pA2H and pN.2. The μ heavy chain construct that resulted was designated pNμA2.1.

The details were as follows: Human placental DNA prepared as described above in Example I, B.1.a.ii, was restricted with Hind III and fractionated by centrifugation through a sucrose gradient. Those fractions containing DNA of approximately 11-13 kb and hybridizing with the murine μ switch region probe, M2-20 (Marcu et al., 1980, Cold Spring Harbor Symposia On Quantitative Biology, 45: 899-911), were ligated with Hind III-digested λL47.1 phage arms prepared as described above in Example I, B.1.a.i, and the ligation mixture was packaged using extracts prepared as described above in Example I, B.1.a.v. This phage library was then plated and screened using a nick-translated insert prepared from M2-20. Hybridizations were performed as described above in Example I, B.1.a.iii. Positive plaques were picked, diluted and replated. These plaques were then screened with both the M2-20 probe and with a murine Cμ cDNA probe (pμ[3741], also described in Marcu et al. (1980), supra). The M2-20-hybridized filters were washed as in the first screen, while the pμ-hybridized filters were washed under less stringent conditions (0.2X SSC, 0.1% SDS at 50°C) since a lower degree of homology between mouse and human sequences was expected. Clones hybridizing with both probes were purified by repeated rounds of plating and DNA was prepared from these clones. The clones were confirmed as containing the human germline μ constant region gene by: 1) the fact that the Hind III insert size of the clones was approximately 12 kb, 2) hybridization with a human μ switch region probe, and 3) comparison with the Hind III and EcoR I restriction pattern described by Ravetch et al., 1981, Cell, 27: 583-591. These clones were designated Phage μ.

The Hind III insert from Phage μ was subcloned into Hind III restricted pUC18 and used to transform competent DH5α cells as follows: 5 μg of Phage μ was digested with 20 units Hind III, PCI extracted, ethanol precipitated, and resuspended to 20 ng/μl in TE. 10 μg of pUC18 was restricted with 40 units of Hind III, PCI extracted, ethanol precipitated and resuspended in 45 μl H₂O. The DNA was phosphatased using 56 units of CIP for 1 hour at 37°C at a total volume of 60μl. 2.5 μl of 0.5 M EDTA, pH 8.0 was added, heated to 68°C for 10 min., cooled to room temperature, PCI extracted, ethanol precipitated and resuspended in 150 μl TE to a concentration of about 50 ng/μl.

100 to 250 ng restricted phage DNA was ligated with 50 ng restricted and phosphatased pUC18 in a 15 μl reaction volume containing ligation buffer and 100 units T4 DNA ligase (New England Biolabs). The samples were ligated for 15 min. at room temperature. The samples were then diluted to 30 μl with ligation buffer lacking PEG and 40 units of T4 DNA ligase. The samples were incubated overnight at 15°C and then stored at -20°C. 20 μl of the ligation mixture was diluted to 100 μl with TE and 1 to 2 μl was used to transform 50 μl of competent DH5α cells. The cells were plated on LB-agar containing ampicillin and X-gal as a color indicator.

DNA was prepared from the white colonies and restricted with Hind III. Those samples showing an insert of appropriate size (about 11 to 12 kb) were digested with BamH I to determine orientation of the insert. Clones were obtained with the $C\mu$ gene in both orientations with respect to pUC18 and were termed $p\mu.1$ (BamH I site in polylinker near 5' end of gene) and $p\mu.2$ (BamH I site in polylinker near 3' end of gene), respectively (see Fig. 20a).

Next, $pN\mu A2.1$, a construct containing the expressed heavy chain variable region gene from 4B9 and the $\mu$ constant region gene in the pN.2 vector, was obtained by a 3-way ligation of DNA fragments containing each of these components as follows: 5 $\mu$g of pN.2 (described earlier in Example I,A.1.b) was digested with 50 units EcoR I and 27 units BamH I, ethanol precipitated, and resuspended in 22 $\mu$l TE. The DNA was then phosphatased using 1 $\mu$l CIP (Boehringer-Mannheim) at 56°C for 30 min. The DNA was then phenol extracted and ethanol precipitated and resuspended with 100 $\mu$l TE. The final concentration was about 50 ng/$\mu$l. Approximately 20 $\mu$g pA2H (described earlier in Example I,B.2.a) was digested with 60 units EcoR I and 40 units Hind III. 0.75 $\mu$g $p\mu.2$ was digested with 27 units BamH I and 60 units Hind III and then ethanol precipitated. All three samples were separated on 0.8% low melt agarose and the desired bands (8 kb for pA2H, 5 kb for pN.2, and 11 kb for $p\mu.2$) were recovered. The low melt agarose fractions were then melted at 70°C. 10 $\mu$l of each was combined and ligated with 1.5 units T4 DNA ligase (Boehringer-Mannheim) for 15 min. at room temperature and then at 15°C overnight in a reaction volume of 50 $\mu$l.

1-12.5 $\mu$l of the ligation reaction were used to transform 200 $\mu$l of competent DH5$\alpha$ cells. The bacteria were plated on LB-agar containing ampicillin. The resulting colonies were screened by hybridization with $pJ_H$ as described and the DNA from the low melt agarose fraction containing the $\mu$ gene of $p\mu.2$ as described above. DNA was prepared from those colonies hybridizing with both probes. The DNA was restricted with EcoR I, BamH I, Hind III, and Bgl II to find those with the desired pattern: variable and constant region genes in the same orientation but opposite to that of the neo gene and the variable region gene 5' of the constant region gene. The desired construct was designated $pN\mu A2.1$ (see Figure 20b). Preparation of this construct DNA for transfection is described in Example V.

## C. Light Chain Gene Constructs

### 1. Kappa Light Chain

Since both rearranged kappa light chain alleles from cell line 4B9 can be expressed at the RNA level, it is difficult to determine which of the RNA species will be translated into protein. We therefore cloned both rearranged kappa light chain alleles and then determined by transfection which one was translated into a protein capable of combining with the appropriate heavy chain to bind antigen.

### a. Kappa Gene Cloning (Fig. 21)

In the following protocol, Southern Transfer analysis with the $J_x$ and $C_x$ probes was used to determine the size of BamH I fragments of DNA from cell line 4B9 that would contain rearranged kappa chain VJ genes. Furthermore, it was important to determine that the BamH I fragments were of sufficient size to contain the complete variable region gene as well as sufficient upstream sequences to promote adequate levels of transcription. This size was determined by measuring the distance (in kb) from the BamH I site 3' of $C_x$ to the 5'-most $J_x$ gene, and adding approximately 2 kb for L-V gene and promoter sequences. (In many $V_x$ genes, there is a BamH I site within the L-$V_x$ complex; in such cases, alternative enzymes such as Hind III or EcoR I can be used.) In the case of LCL 4B9, the $V_x$ and $C_x$ genes are found on the same BamH I fragments of less than 19 kb and are therefore easily cloned into a single phage vector, $\lambda$L47.1. The $V_x$ and $C_x$ genes were therefore cloned together on a single restriction fragment. For transfection experiments, DNA from the kappa phage clones was either digested with BamH I and transfected directly or was subcloned into an expression cassette described in Section A.3.

Briefly, a bacteriophage library was made from BamH I digested and size fractionated genomic DNA from LCL 4B9. The library was screened with $J_x$ and $C_x$ probes, DNA was prepared from positive plaques, and digestions were performed on phage DNA to confirm the identity of the clones as kappa genes. The phage DNA was then either digested with BamH I and used for transfections or subcloned into an expression cassette. The experimental details are more fully set forth below.

One hundred twenty micrograms of DNA from cell line 4B9 and 10 $\mu$g of human placental DNA were digested with BamH I and then 7.5 $\mu$g of each fractionated by electrophoresis through a 1.1% agarose gel in TAE buffer. Southern transfers were performed as described above for heavy chain genes, except that the DNA was digested with BamH I and probed with a pJ$x$ (1.8 kb Sac I fragment in Figure 21) probe which spans the J$x$ region. The J$x$ probe was obtained from a plasmid containing the 1.8 kb Sac I fragment of a germline human kappa clone described by Hieter et al., 1980, Cell, 22:197-207 and 1982, J. Biol. Chem., 257:1516-1522. This probe hybridized to an 11 kb germline fragment in placental DNA and to a 16 kb rearranged fragment of 4B9 DNA. Southern transfer experiments using the same probe, but either EcoR I or Hind III digests, showed that there were two rearranged kappa genes in 4B9, suggesting that BamH I digestion had produced two fragments of similar molecular weight such that they were not distinguishable from each other.

Fifty micrograms of BamH I digested 4B9 DNA were fractionated on a 10-40% sucrose gradient and fractions containing DNA of the desired size (16 kb) were screened by Southern transfer analysis with the pJ$x$ probe. Those hybridizing most strongly were used for the library construction.

Approximately 5% of the DNA in the peak fractions was ligated with the λL47.1 phage arms (prepared as described above for the heavy chain variable region cloning, except that the DNA was digested only with BamH I), using T4 DNA ligase and ligation buffer. The ligation reaction was then packaged in vitro, as described above. Titers of the bacteriophage were determined using the 803 supF or the LE 392 strain of E. coli and were usually between 0.5 and 1.5 x 10$^5$ total plaque forming units (pfu) from each ligation reaction (corresponding to 5% of a sucrose gradient fraction). Approximately 10$^5$ pfu were plated out. Duplicate plaque lifts were taken from each plate using nitrocellulose paper (Schleicher and Schuell, BA85). One set of filters was hybridized with the J$x$ probe, the other was hybridized with a C$x$ probe (a 2.7 kb EcoR I fragment derived from the same germline human kappa clone as previously described). Only plaques hybridizing with both probes were picked and further purified as described for the heavy chain variable region gene cloning. Figure 21 shows maps of the two kappa clones, designated A1$x$ and A2$x$. Both clones have approximately 16 kb BamH I inserts. Alternatively as described in the following section, the variable region genes could be inserted into a cassette containing C$x$ and enhancer elements. Preparation of DNA for transfections is described in Examples II and III.

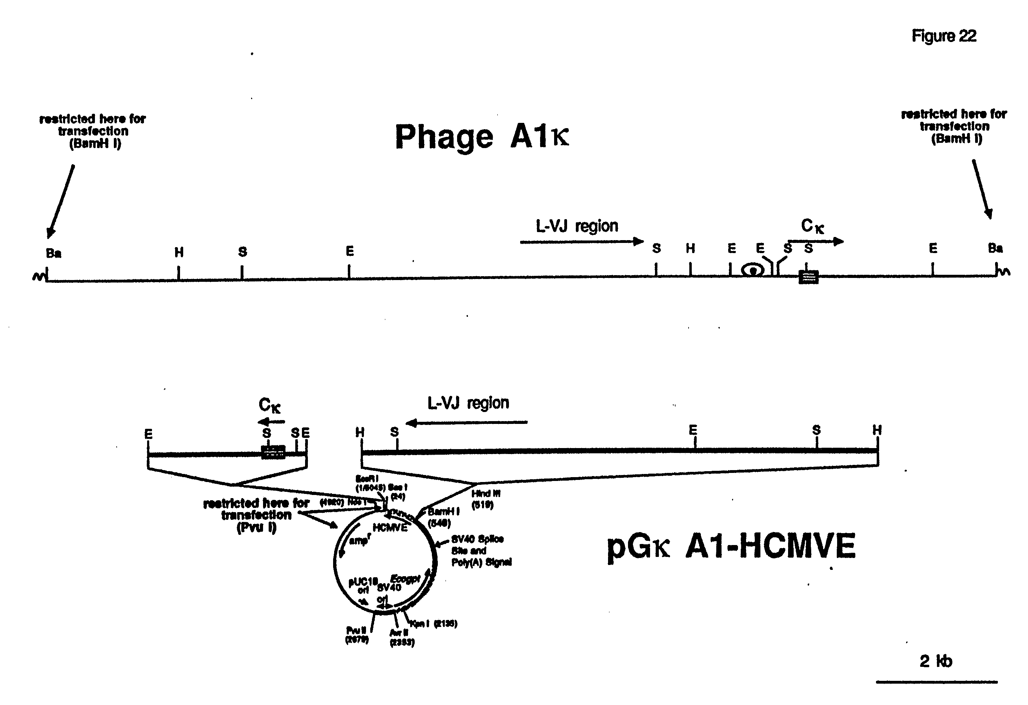

## b. Subcloning 4B9 Kappa V Genes into pGEMC$x$ (Fig. 22)

The variable region from A1$x$ was inserted into the expression cassette pGEMC$x$ as follows. Briefly, PGEMC$x$ was digested with Hind III, treated with CIP, PCI extracted and ethanol precipitated and resuspended to 10 ng/µl.

The entire BamH I fragment of bacteriophage DNA A1$x$ was subcloned into the BamH I site of the pN.1 vector to form pN$x$A1. Fifty micrograms of pN$x$A1 were then digested with Hind III (150 units, overnight). The DNA was electrophoresed through agarose and the 8.7 kb band containing the variable region was excised. DNA was eluted from the agarose by electrophoresis into dialysis tubing. The DNA was extracted with PCI, precipitated with ethanol, and then redissolved in 50 µl TE. The DNA was further purified using an Elutip$^{(R)}$ column, according to the manufacturer's instructions (Schleicher and Schuell). Following ethanol precipitation, the DNA was dissolved in 50 µl TE to a concentration of 270 ng/µl.

The 8.7 kb Hind III fragment of pN$x$A1 containing the 4B9 kappa chain variable region gene (270 ng) was ligated with 10 ng pGEMC$x$ vector, prepared as described above in a 50 µl reaction volume using ligation buffer lacking PEG and spermidine at 15°C overnight. A portion of the ligation mixture was diluted 1:5 with TCM and used to transform competent DH5$\alpha$ cells. Ampicillin resistant colonies were screened by colony hybridization using purified Hind III fragment containing the variable region gene. Plasmid DNA was prepared from selected colonies and the orientation of the insert was determined by digestions with EcoR I and Sac I (separately). The final construct was called pG$x$A1-HCMVE and is depicted in Figure 22.

## 2. Lambda Light Chain

Since both rearranged lambda light chain alleles can be expressed at the RNA level, it was difficult to determine which of the RNA species was translated into protein. We therefore cloned both rearranged lambda light chain alleles, subcloned them into an expression cassette and then determined by transfection which one was translated into protein which ultimately combined with the appropriate heavy chain to bind antigen.

In this Example, Southern Transfer analysis with a Cλ3 probe was used to determine the size of Hind III and EcoR I fragments of DNA from cell line 9D1 that would contain rearranged lambda light chain VJ genes. However, since our probe hybridizes with Cλ1-3 and rearrangements could occur into any of the Jλ genes, it was difficult to determine minimum size restriction fragments necessary to contain promoter and L-V sequences. The rearranged λ genes were therefore cloned as both EcoR I and Hind III fragments.

Briefly, bacteriophage libraries were made from Hind III and from EcoR I digested and size fractionated genomic DNA from LCL 9D1. The libraries were screened with the Cλ3 probe, prepared as described in the following section. The variable region genes were then subcloned into pUC18 to facilitate mapping of restriction sites and identification of duplicate clones. The rearranged λ genes were then inserted into the pUCG-MCSII-MHE cassette, described above, to form expressible lambda light chain genes. The details are set forth below.

## a. Preparation of lambda constant region probe, Cλ3

An oligonucleotide probe was used to clone a germline lambda constant region gene, and a portion of this clone was used as a probe for detecting rearranged lambda genes in human lymphoblastoid cell lines. Briefly, a human germline DNA library containing Hind III fragments of approximately 9 kb was prepared. A synthetic oligonucleotide probe homologous to a region of identity among Cλ 1-3 was prepared and used to screen the library. Hybridization and washing conditions were utilized such that only a few plaques hybridized. These plaques were purified and the DNA was prepared and analyzed to determine whether any of them corresponded to the published map of the lambda locus. Clone HP2-Cλ 5 corresponded to the Hind III fragment containing the Cλ 3 and 4 genes. An EcoR I-Hind III fragment containing Cλ3 was subcloned into pUC18 and a 0.8 kb EcoR I-Bgl II fragment derived from it, referred to as the Cλ3 probe, was used to screen for rearranged lambda genes in a library derived from cell line 9D1.

The details are as follows: Human placenta was used as a source of germline DNA. Genomic DNA was prepared as described above. According to Hieter et al., 1981, supra, the human λ 1 and λ 2 genes are found on an 8.8 kb Hind III fragment and the human λ 3 and λ 4 genes are found on a contiguous 9.2 kb Hind III fragment. Therefore, to prepare a library containing the lambda genes, human placental DNA was digested to completion with Hind III, phenol and chloroform extracted, ethanol precipitated and resuspended in TE buffer. Fifty micrograms of the digested DNA was fractionated on a 10-40% sucrose gradient as described above and fractions containing DNA of 8.2 to 11.5 kb were each ligated with λL47.1-Hind III treated phage arms (prepared as described above; approximately 5% of each fraction was ligated with 100 ng of phage arms).

To screen the bacteriophage library, a 26 base oligonucleotide of the following sequence was synthesized on an Applied Biosystems 380A DNA Synthesizer: 5′ GCTGC CAGGT CACGC ATGAA GGGAG C 3′. This sequence corresponds precisely to a region of identical nucleotide sequence among Cλ 1 through 3 (nucleotides 251-276, according to the numbering of Hieter et al., ibid). The lambda probe was radiolabeled with $^{32}$P-γATP by a T4 DNA kinase reaction (Maniatis et al., supra, p. 125) to a specific activity of approximately $10^8$ cpm/μg.

The library was plated and phage DNA lifted onto nitrocellulose paper (in duplicate) as described by Maniatis et al., supra, p. 320. The filters were prehybridized at 37°C for 4 hours in a solution containing 6X SSC, 5X Denhardt's solution, 0.15% pyrophosphate, and 0.1 mg/ml tRNA. To decrease the non-specific binding of the probe to nitrocellulose, the probe was prehybridized in the above solution to a piece of nitrocellulose. The filters containing the phage were then hybridized with the "prehybridized" probe at $10^6$ cpm/ml overnight at 37°C. Following hybridization, the fluid was stored at -20°C and used again for the next round of hybridization. The filters were washed under progressively more stringent conditions until only a few positive plaques remained. Washing one of the sets of filters in 0.25X SSC at 45°C produced only two positive hybridization signals. Both of these signals, in addition to many more, were also present in the duplicate set of filters which had been washed in 1X SSC at 45°C.

A single plaque (HP2-Cλ5) was picked, diluted and plated out again. Filters were prehybridized as described above, and then hybridized in the fluid remaining from the first library screen. Filters were washed in 1X SSC at 45°C. Many dark hybridization signals were seen against a background of light signals, suggesting that there had been some enrichment for the positive plaque and that most of the hybridization seen after washing in 1X SSC at 45°C was non-specific. Two of the plaques hybridizing most strongly were picked and replated. This process was continued until the plaques were pure.

Phage DNA was then prepared as described in Maniatis et al., supra, p. 77. Hind III and EcoR I digests of the phage DNA gave fragment sizes suggesting that clone HP2-Cλ5 contained the human Cλ3 and Cλ4 genes: the total Hind III insert size was 9.5-10 kb, including two 4.5 kb EcoR I-Hind III fragments and a 1 kb EcoR I fragment. The oligonucleotide probe hybridized with the 4.5 kb EcoR I-Hind III bands.

The 10 kb Hind III insert from clone HP2-Cλ5 was subcloned into pUC18 to form pCλ3/4. The 4.5 kb EcoR I-Hind III fragment containing CX3 was then further subcloned into pUC18. This final subclone, pCX3, was distinguishable from the alternative 4.5 kb insert containing CX4 by the size of the Bgl II fragments generated. A Bgl II digest of pCX3 produced a 2.8 kb fragment. The final probe used to screen libraries containing rearranged lambda DNA was a 0.8 kb EcoR I-Bgl II insert containing the CX3 gene from pCX3 (see Figure 23).

b. Library Construction and Screening (Fig. 23)

Southern blots of genomic DNA from cell line 9D1 and germline DNA, previously probed with $pJ_H$ and $pJx$ probes, were washed by adding boiling water to the nitrocellulose filter and incubating them at room temperature for 15 minutes. An overnight exposure of X-ray film to the filter showed that no probe remained. The filters were then probed with nick translated (specific activity $3.5 \times 10^8$ cpm/$\mu$g) CX3 probe in a hybridization buffer lacking dextran sulfate.

The Southern transfer of Hind III digested 9D1 DNA showed rearranged bands at approximately 10.4 and 4.5 kb and loss of the 8.8 kb germline band containing CX1 and 2 sequences. The Southern transfer of EcoR I digested 9D1 DNA showed rearranged bands at 12 kb and 5 kb and loss of the 8 kb and probably 14 kb germline bands. The two Hind III rearranged fragments and the 12 kb EcoR1 fragment were selected for cloning.

The Hind III sucrose gradient fractions of the appropriate size (10-13 kb and 4-6 kb), obtained during the heavy chain cloning, were ethanol precipitated and resuspended in 50 $\mu$l TE buffer. The EcoR I digest was fractionated on a sucrose gradient, as previously described, and the fractions containing DNA of the appropriate size were determined by electrophoresis on an agarose gel. T4 DNA ligase was then used to ligate 10 $\mu$l of the gradient fractions with 100 ng phage arms (20 $\mu$l reaction volume, 30 minutes at 37° C). The ligations were packaged as previously described and the titers of the libraries, determined using 803 supF strain of E. coli, were between 2 and 5 x $10^5$ pfu. In each case, nearly the entire library was plated. Duplicate plaque lifts were taken from each plate using nitrocellulose paper (Schleicher and Schuell, BA85). Both sets of filters were hybridized overnight with the CX3 probe. Only plaques hybridizing in duplicate were picked and further purified as described for the heavy chain variable region gene cloning. DNA was prepared from an amplified plate using the protocols of Porteous et al. and Helms et al., supra.

The Hind III and EcoR I inserts were subcloned into pUC18. The subclones were confirmed as containing lambda constant regions by hybridization with the CX3 probe. Germline and rearranged lambda genes were distinguished by comparing Hind III, EcoR I, Hind III + EcoR I, and Hind III + EcoR I + Bgl II digests with the known germline patterns. At least one clone containing rearranged lambda DNA was obtained from each library: a 12 kb EcoR I insert, a 10.4 kb Hind III insert and a 4.5 kb Hind III insert. The 4.5 kb Hind III fragment was shown to be contained within the 12 kb EcoR I fragment. The 4.5 kb Hind III and 12 kb EcoR I clones were referred to as XD2H and XD2e, respectively. The 10.4 kb Hind III clone was referred to as XD1 (see Figure 23).

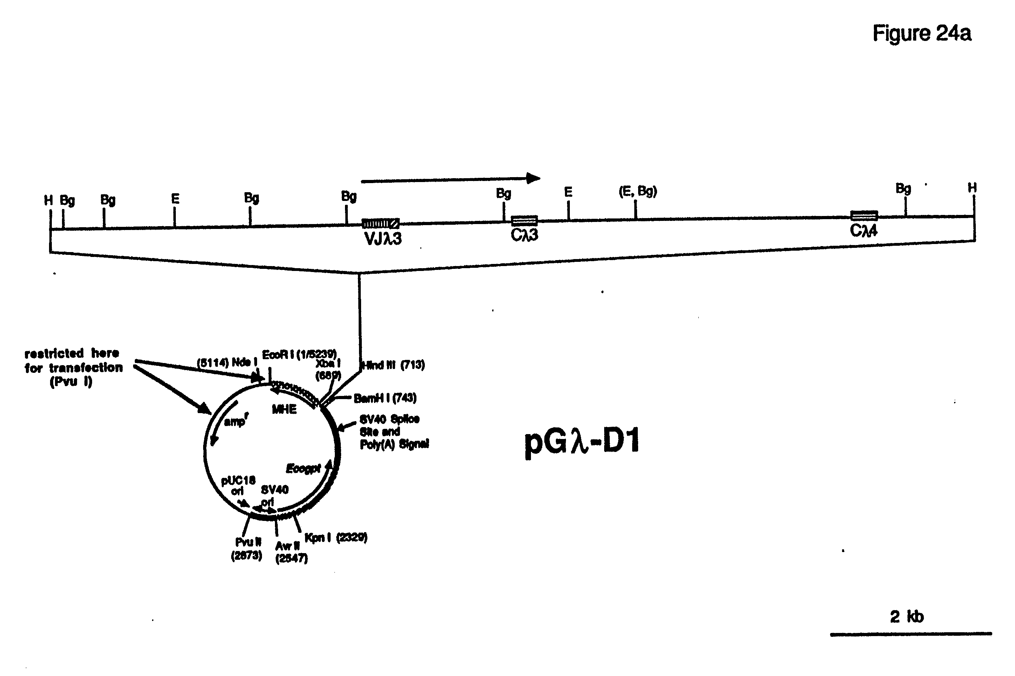

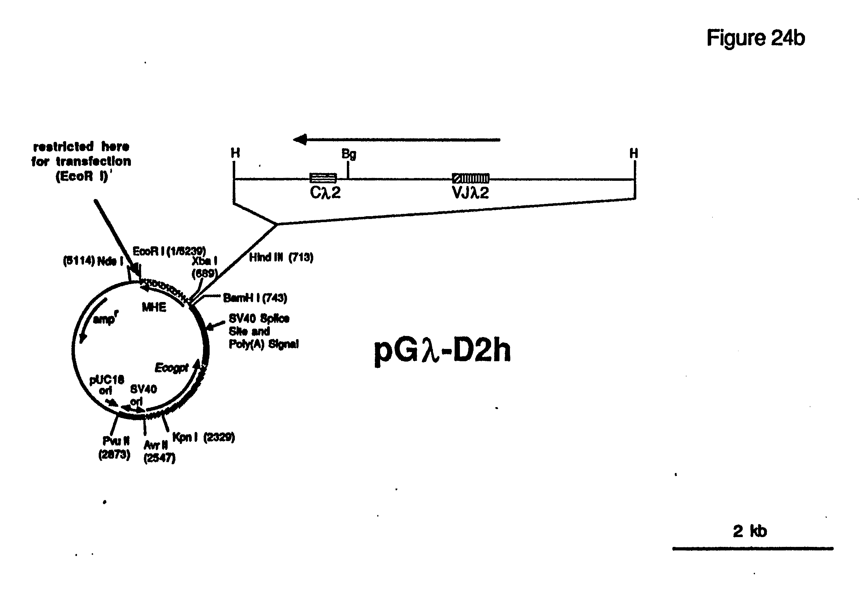

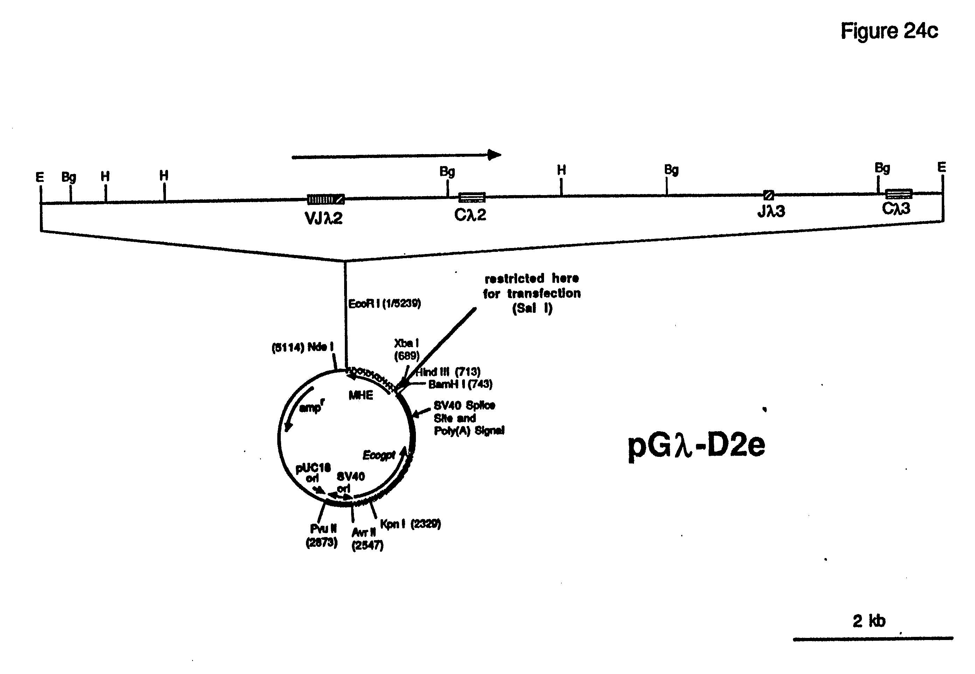

c. Subcloning λ Genes into An Expression Cassette (Fig. 24)

A cassette similar to that described for $Cx$ (pGEMC$x$, section A.3) can be prepared for CX. This would then require the insertion of the desired λ variable region genes 5' of CX. Alternatively, the VX and CX genes can be inserted into a cassette which only contains an enhancer region and a selectable marker, such as pUCG-MCSII-MHE described in Section A.1.a.iv. Ten micrograms of the pUCG-MCSII-MHE cassette, prepared as described were digested with 100 units of either Hind III or EcoR I (2 hours at 37° C) and electrophoresed through a 1% agarose gel. The desired bands were electrophoresed into NA45 DEAE paper and eluted according to the manufacturer's instructions, ethanol precipitated and resuspended to 1 $\mu$g/$\mu$l in TE buffer. The digested vector was treated with CIP (2.5 $\mu$g DNA and 40 units CIP for 40 minutes at 37° C) and 0.5 $\mu$g was then electrophoresed through low melt agarose and the appropriate band transferred to a microfuge tube.

The plasmids containing the lambda genes were digested with either Hind III or EcoR I to remove the insert from the vector (2.5 $\mu$g plasmid DNA and 20 units restriction enzyme for 1 hour at 37° C). Two hundred nanograms of the DNA was electrophoresed through low melt agarose and the bands containing the inserts were transferred to a microfuge tube.

The agarose in the microfuge tubes was melted at 70° C for 5 minutes and agarose containing 5-10 ng of vector was combined with agarose containing 50 ng of lambda region insert. The DNA fragments were ligated using T4 DNA ligase (100 μl final volume, overnight incubation at room temperature). The ligation mixture was used to transform competent DH5α cells. DNA was prepared from the ampicillin resistant colonies and screened by digestion with Hind III or EcoR I for inserts of the appropriate size. The resulting constructs were designated pGλ-D1, pGλ-D2e and pGλ-D2h and are shown in Figures 24a, 24b and 24c. Preparation of DNA for transfections is described in Example VI.

<div align="center">

EXAMPLE II

</div>

<div align="center">

PRODUCTION OF A RECOMBINANT IgG2 ANTIBODY BY 4B9 GENE TRANSFECTOMAS

</div>

This Example demonstrates a method for the production of human monoclonal antibodies in mouse cells which uses the constructs containing the 4B9 variable region genes, $V_H$ and $Vx$, described in Example I herein. In this embodiment, the 4B9 antibody has been switched from the parental IgM class to the IgG₂ class. This novel antibody possesses antigen binding specificity similar to that of the antibody secreted by the 4B9 cell line.

A. Transfection into Mouse Cells

Transformed bacteria containing pGγ2-A1H and pGγ2-A2H (Figs. 15a and 15b) were grown overnight in LB broth containing 100 μg/ml ampicillin and 1% glucose. Plasmid DNA was prepared by the alkaline lysis method and was further purified on two CsCl gradients. One hundred micrograms of plasmid DNA were then digested with EcoR I, phenol and chloroform extracted, ethanol precipitated and resuspended to 1 μg/μl in sterile TE. The digestion was not complete. Phage DNA was prepared as described in Maniatis et al., supra, pp. 76-85, using a CsCl equilibrium centrifugation step. For transfection into mammalian cells, DNA from clones A1x and A2x (Fig. 21) was digested with BamH I, phenol and chloroform extracted, ethanol precipitated and resuspended in sterile TE to 1 μg/μl. Plasmid DNA prepared as described above was transfected into Ag8.653 cells by electroporation. Ag8.653 is an established mouse myeloma cell line (ATCC No. CRL 1580) which does not produce detectable heavy or light chain immunoglobulin. Electroporation was performed essentially according to the method of Potter et al., 1984, Proc. Natl. Acad. Sci. USA, 81:7161-7165. For electroporation, cultures of cells in the log phase of growth were counted, concentrated by centrifugation and resuspended in Dulbecco's PBS at a concentration of $2 \times 10^7$ cells per ml. CaCl₂ and MgCl₂ were added to a final concentration of 2 mM each. The restricted bacteriophage DNA coding for the 4B9 kappa light chain was added to the cells at a concentration of 10 μg/ml and the heavy chain DNA containing plasmid was added to the cells at a concentration of 10 μg/ml. The following combinations were transfected:

1) A1x light chain (10 μg) + pGγ2-A1H heavy chain (10 μg);
2) A1x light chain (10 μg) + pGγ2-A2H heavy chain (10 μg);
3) A2x light chain (10 μg) + pGγ2-A1H heavy chain (10 μg); and
4) A2x light chain (10 μg) + pGγ2-A2H heavy chain (10 μg).

The mixture of cells and DNA was suspended in sterile electroporation cuvettes (BioRad) in a wet ice bath (0° C) for 5-8 minutes. The suspension was then pulsed with 2000 volts from an ISCO power supply Model 494 for a sufficient time to allow full deflection of the ammeter, usually less than one second. The cuvettes were gently mixed by agitation and incubated on ice for 8-10 minutes. One ml of serum-free RPMI 1640 was added to each cuvette, mixed by agitation and allowed to stand at room temperature for 8-10 minutes. The suspension was removed from the cuvette and placed into RPMI 1640 containing 10% FCS (fetal calf serum), to a final volume of 10 ml in a T25 tissue culture flask (Corning).

B. Cell Culture

The cultures were incubated at 37° C in a humidified atmosphere containing 6% CO₂. Forty-eight hours after electroporation, the cells were counted and stained with the vital dye Trypan blue (GIBCO) to

<div align="center">37</div>

determine viability. The cell suspension was diluted 1:1 and counted in a hemocytometer. Cells which excluded the dye were counted in addition to cells which took up the dye. The number of cells which excluded the dye was divided by the total number of cells, thereby determining the percentage of viable cells. The cells were then suspended in RPMI 1640/10% FCS (containing 5.0 $\mu$g/ml of mycophenolic acid (Sigma), 15 $\mu$g/ml hypoxanthine and 250 $\mu$g/ml xanthine (selective medium)) at a concentration of 1 x $10^5$ viable cells/ml. The suspension was plated in 24-well tissue culture dishes (Costar), 1 ml per well. The cells were fed selective medium every 3 to 4 days.

The cultures were observed every day on an inverted microscope for signs of cells proliferation. Within 10 days after plating, most of the cells in the cultures had died in the mycophenolic acid. Fourteen to twenty-one days post-plating, most of the cultures contained proliferating cells of a sufficient density to test supernatants for the presence of human kappa light chain and human gamma heavy chain, as described below. The number of samples positive for human gamma heavy chain out of the total number tested for the above-enumerated combinations were as follows: 1) 0 out of 4; 2) 4 out of 4; 3) 0 out of 4; and 4) 0 out of 3.

Cultures positive for human gamma heavy chain (those derived from combination 2, above) were subjected to limiting dilution cloning in 96-well flat-bottom tissue culture dishes (Costar). Cells were plated at a density of 1, 2, and 5 cells per well in RPMI 1640, 20% FCS, 10% NCTC (MA Bioproducts) and 50% Ag8.653 conditioned medium. Cells were fed selective medium every 3 to 5 days and were monitored daily for the outgrowth of cells. Within 10 to 14 days following limiting dilution cloning, clones appeared in most wells. Supernatants from the wells were then assayed for human heavy chain as described below. Wells positive for human gamma heavy chain and kappa light chain were expanded to larger volumes by dilution into 24-well tissue culture dishes and finally T75 tissue culture flasks (Corning).

Following isolation of single cell clones from the transfection, cells were removed from selective medium by gradually decreasing, and finally eliminating, the presence of mycophenolic acid, hypoxanthine and xanthine. It was observed that the cells grew as well in nonselective medium as those maintained in selective medium, and antibody production was nearly identical.

## C. Human IgG Heavy Chain and Kappa Light Chain Assays

Supernatants were screened for the presence of human IgG using an enzyme-linked immunosorbent assay (ELISA) (Engvall, 1977, Med. Biol., 5:193-200). The plates consisted of a series of flat-bottom 96-well Immulon 2 plates coated with a solution of goat anti-human IgG (Antibodies, Inc.).

A standard was prepared from an IgG polyclonal stock (Cappel) and dilute supernatant samples were prepared in tissue culture medium. One hundred microliters of sample were added to each well, the plates incubated, washed, and 100 $\mu$l of conjugate (goat anti-human IgG horseradish peroxidase, diluted 1:9,000 in diluent containing 1% normal goat serum in citrate buffer, pH 7.0) were added to each well. The plates were incubated at 37° C for 1 hour, washed and 100 $\mu$l of diluted chromogen (80 $\mu$g/ml tetramethylbenzidine, 0.0015% $H_2O_2$ in citrate/phosphate buffer, pH 6.0) added to each well and the plates were incubated for 30 minutes at room temperature. Reactions were stopped by adding 100 $\mu$l of 3N $H_2SO_4$ and absorbance at 450/630 nm read for each well on an automated ELISA reader. Background was defined as the absorbance at 450/630 nm of medium conditioned by the untransfected Ag8.653 cells. Supernatants which were 0.1 units above background were considered positive samples.

Supernatants were also screened for human kappa light chain in an ELISA assay. The assay was identical to that described for the heavy chain except that the conjugate antiserum was an anti-human kappa antibody (Tago, Burlingame, CA) and was diluted 1:3,000.

Quantitation of human immunoglobulin present in the culture supernatants was determined using the ELISA assays described above. IgG standards and multiple dilutions of each sample were run in quadruplicate. The standard curve was plotted and IgG concentrations were extrapolated for the samples by utilizing a series of dilutions beginning at 1:10. Samples outside the linear range of the standard curve were excluded. The dilution factor was multiplied by the concentration in order to obtain the concentration in undiluted supernatants. The values were averaged for the quadruplicate samples. These quantities were determined for both human kappa light chain and heavy chain from 15 day spent cultures. Quantitation of the antibody from five different clones yielded an IgG concentration ranging from 6.7 to 34.5 $\mu$g/ml. The parental lymphoblastoid cell line (4B9) secreted IgM at a concentration of 10 $\mu$g/ml.

## D. Detection of Functional Antibody

Supernatants positive for both human IgG and kappa chain were screened for the ability to bind to group B Streptococci utilizing an ELISA technique. The antigen plates consisted of a series of flat-bottom 96-well Immulon II plates, the wells of which contained viable Group B Streptococcus (ATCC No. 1334) affixed with poly-L-lysine (PLL). The plates were prepared as follows: fifty microliters of PLL in PBS (1 $\mu$g/ml) were added to each well and incubated for 30 minutes at room temperature. The plates were then washed three times with PBS and 50 $\mu$l of a bacterial suspension (O.D.$_{660}$ = 0.2) or PBS were added to each well. The plates were then incubated at 37°C for 60 minutes. To remove unattached bacteria, the plates were washed three times with saline and 0.02% Tween 20 (saline T).

Wells were blocked with 300 $\mu$l of a mixture containing 5% w/v non-fat dry milk, 0.001% anti-Foam A, and 0.01% w/v Thimerosol in PBS, incubated one hour at room temperature, then washed three times with 200 $\mu$l/well of saline T. Fifty microliters of culture supernatants were added to each well, plated in replicate onto both control (PLL, but no bacteria) and antigen plates. The plates were incubated at room temperature for 1 hour. The supernatants were flicked from the plates, the plates washed three times with saline T, and 100 $\mu$l of peroxidase-conjugated goat anti-human IgG and IgM in dilution buffer (PelFreeze) were added to each well. The plates were incubated 60 minutes at room temperature, the conjugate flicked from the plate, the plates washed five times in saline T, 100 $\mu$l of chromogen added to each well, the plates incubated for 15 minutes at room temperature and 100 $\mu$l of 3N H$_2$SO$_4$ added to stop the reaction. Culture supernatants which contained antibody reactive with the bacteria-coated plates were detected by measuring absorbance at 450/630 nm on an automated ELISA reader. All IgG positive clones bound to the Group B Streptococcus plates and not control plates.

Antibody from the cloned transfectomas were also assayed for reactivity to different strains of Group B Streptococcus by a modification of the standard immunoblotting technique. Briefly, the bacterial strains were spotted onto a gridded nitrocellulose paper disc, the disc reacted with the antibody, and the reacted discs developed with an alkaline phosphatase/nitro blue tetrazolium enzyme system. The details are as follows: the bacteria were grown to an O.D. 660 = 0.4, then fixed in 3% formalin (v/v) and incubated at 37°C for one hour. One microliter of fixed bacteria was spotted per grid section of a nitrocellulose paper disc (Schleicher and Schuell, 37 mm nitrocellulose disc, gridded, 0.45 $\mu$m). The spotted discs were air-dried, fixed in 25% v/v isopropanol for 30 minutes, and blocked for 10 minutes in the non-fat dry milk reagent as described for the ELISA method. The blocked discs were washed three times for 5 minutes each in PBS/Tween-20 and were transferred to the lids of 35 x 10 mm tissue culture dishes. An antibody-containing supernatant (1.0 ml) was added to the lid and incubated at room temperature for 60 minutes. Following three 5 minute washes in PBS/Tween-20, 1-2 ml of 1:1000 diluted (PBS) alkaline phosphatase conjugated goat anti-human immunoglobulin (TAGO) was added for 60 minutes at room temperature. The discs were washed as above and were submerged in 1-2 ml of fresh substrate prepared as follows: 16.5 mg of bromochlorindolylphosphate and 8.5 mg nitro blue tetrazolium were dissolved in 50 ml of alkaline phosphatase buffer (0.1 M Tris-HCl, pH 9.5 with 0.1 M NaCl and 5 mM MgCl$_2$), the solution was kept in the dark and filtered immediately before using. After adequate color development (10-15 minutes), the reaction was quenched by rinsing the disc in several changes of distilled water.

A total of one hundred thirty-two different isolates of Group B Streptococcus were tested. The recombinant IgG2 antibody produced by the cloned transfectomas gave identical reactivity patterns to the parental human IgM molecule produced by the 4B9 cell line.


## EXAMPLE III


## PRODUCTION OF ANTIBODIES BY CELLS TRANSFECTED WITH CMV CONSTRUCTS


This Example demonstrates a method for the production of human monoclonal antibodies in mouse cells which uses the constructs containing the 4B9 variable region gene, V$_H$ and pGxA1-HCMVE constructs, described in Example I, section C.1.b. herein. The Example makes use of a transfectoma derived from Example II which expresses the $\gamma$2 heavy chain of 4B9 and no detectable light chain. The Example also documents the feasibility of the sequential transfection of a heavy chain gene and subsequent transfection of a light chain gene for expression of whole immunoglobulin molecules. Similarly, the light chain can be transfected first, light chain expressing clones isolated, and followed by subsequent transfection of heavy chain genes.

A. Sequential Transfection into Mouse Cells

In Example I, section C.1.b., pGxA1-HCMVE, which contains the human CMV enhancer in the intron between 4B9 Vx and Cx genes, was constructed. A1x phage DNA was prepared as described in Example II. Bacteria transformed with pGxA1-HCMVE plasmid DNA were grown in T broth (12 g tryptone, 24 g yeast extract, 0.4% glycerol, .017 M $KH_2PO_4$ and .072 M $K_2HPO_4$) containing 100 $\mu$g/ml ampicillin. Plasmid DNA was prepared by the BRIJ-DOC lysis procedure and followed by two CsCl gradient centrifugations. Plasmid DNA was digested with Pvu I, phenol and chloroform extracted, ethanol precipitated and resuspended in TE. Plasmid and phage DNA prepared as described above were transfected by electroporation into a transfectoma producing only heavy chains of the 4B9 specificity as described herein before in Example II,A, with the following exception: electroporation was performed utilizing a Biorad Gene Pulser equipped with a capacitance extender (Biorad). The cell suspension was pulsed with 0.25 kV, 960 $\mu$farads with a time constant less than 0.1 sec. The above-mentioned transfectoma was isolated from the original transfection described in Example II. Since the original transfectomas described in Example II had been selected for mycophenolic acid resistance, the transfection of pGxA1-HCMVE and A1x phage DNA constructs required "cotransfection" of a selectable marker other than the Ecogpt to allow selection of stable transformants. The neomycin resistance gene, encoded by expression vector pN.1 described in Example I was chosen as the other selectable marker. The following combinations were performed: 1) pN.1 (1 $\mu$g) + pGxA1-HCMVE (10 $\mu$g); and 2) pN.1 (1 $\mu$g) + A1x (10 $\mu$g).

B. Cell Culture

Cell culture was identical to that described in Example II, section B with the following exceptions. Cells were suspended in RPMI 1640/20% FCS (containing 0.25 mg/ml G418 (Gibco)) at a density of 1 x $10^5$ cells/ml. The cell suspension was plated into 96-well tissue culture dishes (Costar), 100 $\mu$l per well. The cells were fed selective medium every 4 to 5 days. Fourteen to twenty-one days after the transfected cells were plated into 96-well dishes, the cultures were assayed for the presence of human gamma heavy chain, as described in Example II, section C. The number of wells positive for the above enumerated combinations were as follows: 1) 174 out of 192; and 2) 19 out of 192.

Cultures positive for human gamma heavy chain were cloned by the soft agarose method, essentially as described by Coffino, et al., 1972, J. Cell Physiol., 79:429-440. Clones which produced human immunoglobulin molecules were picked and transferred to 96-well dishes for expansion. Wells were quantitated for human immunoglobulin production by the quantitation assay described in Example II, section C. The number of 96-well cultures producing greater than 1 $\mu$g/ml of human IgG following soft agarose cloning was 13 out of 13 for the HCMVE construct and 1 out of 4 for the A1x construct.

EXAMPLE IV

PRODUCTION OF A RECOMBINANT IgG1 ANTIBODY BY 4B9 GENE TRANSFECTOMAS

This Example demonstrates methods for the production of a novel 4B9 antibody in which the class of the antibody has been switched from the parental IgM class to the IgG1 class. This novel antibody possesses an antigen binding specificity similar to that of the antibody secreted by the 4B9 parental cell line.

First, bacteria were transformed with the pGxA1-HCMVE plasmid described in Example I,C.1.b. above (see Figure 22), which contains the human CMV enhancer in the intron between the 4B9 Vx and Cx genes. The transformed DH5α bacteria were grown in T broth (12 g tryptone, 24 g yeast extract, 0.4% glycerol, 0.017 M $KH_2PO_4$ and .072 M $K_2HPO_4$) containing 100 $\mu$g/ml ampicillin. Plasmid DNA was then prepared by the BRIJ-DOC lysis procedure, followed by two CsCl gradient centrifugations. The plasmid DNA was digested with Pvu I, extracted with phenol and chloroform, then precipitated with ethanol and resuspended to 1 $\mu$g/$\mu$l in TE.

The plasmid DNA was then transfected by electroporation into Ag8.653 mouse cells as described in Example II,A. Cell culture conditions were identical to those described in Example III. 14 to 21 days after the transfected cells were plated into the dishes, the cultures were assayed for the presence of human kappa light chain as described below. Cultures positive for kappa light chain were subjected to cloning in soft agarose essentially as described by Coffino et al., 1972, J. Cell Physiol., 79: 429-440.

One clone which produced human kappa light chain was subjected to transfection by electroporation as described above with plasmid DNA prepared from the human γ1 construct pNγ1A2.1, described in Example I,B.2.b (see Figure 16). Large amounts of the pNγ1A2.1 DNA were prepared for this transfection by growing bacteria transformed with the construct in T broth and ampicillin. The DNA was then prepared by the alkaline lysis method, followed by purification via a pZ523 column (5 Prime → 3 Prime, Inc., PA), according to the manufacturer's instructions. The DNA sample was digested with BamH I prior to transfection. It should be noted that the pNγ1A2.1 construct does not follow the normal immunoglobulin gene configuration in that the variable region gene is between the switch region and the constant regions. Digestion with BamH I separates the entire immunoglobulin gene from the rest of the vector. In contrast, EcoR I removes the switch region from the rest of the construct, making the vector configuration similar to that of pNγ1A2.2.

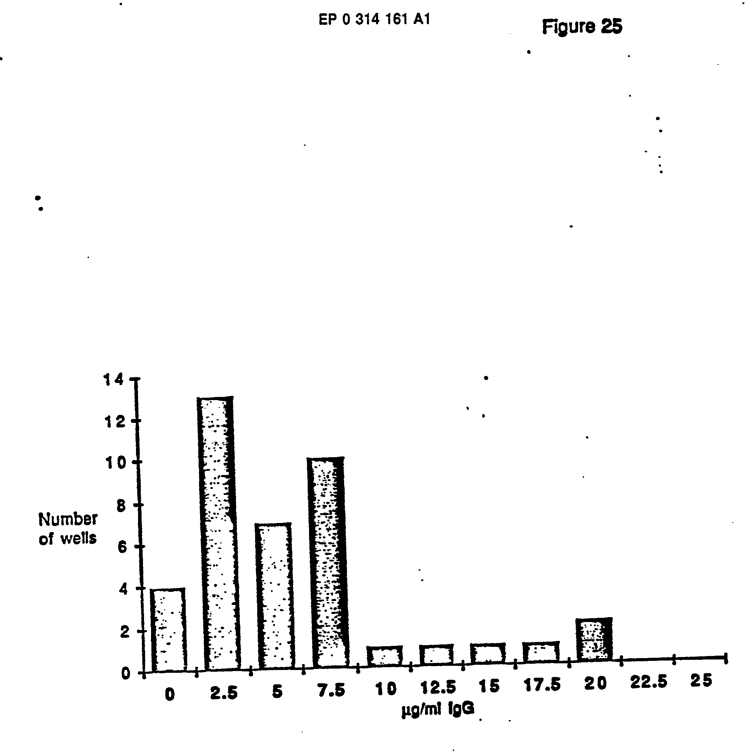

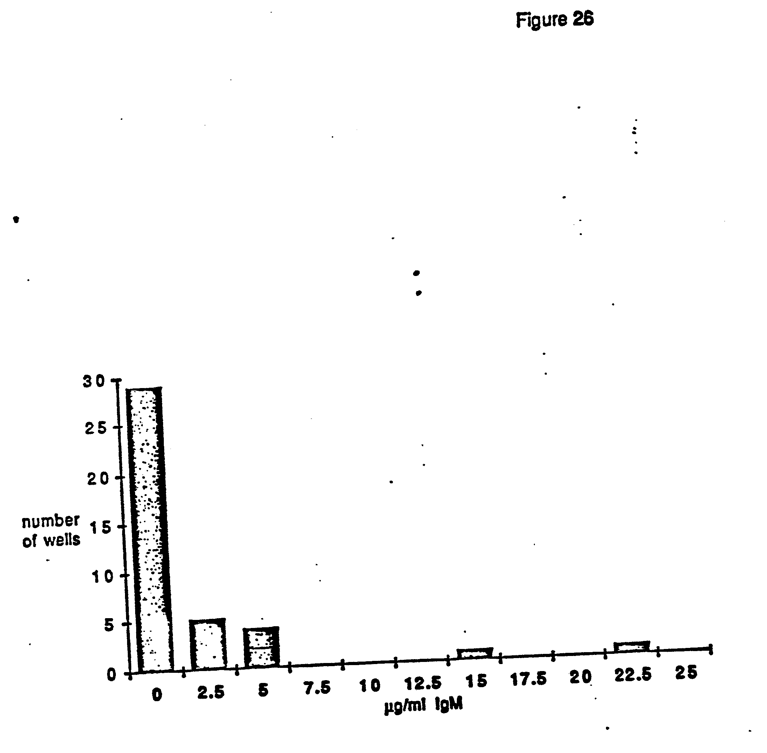

Electroporation and cell culture of the transfectoma was identical to that described above with the exception that the selective medium contained G418 (GIBCO). 14 to 21 days after the transfected cells were plated into the 96-well dishes, the cultures were assayed for the presence of human gamma heavy chain using the ELISA assay described in Example II,C. Antibody was then quantitated from the 40 masterwells producing the most immunoglobulin using the same ELISA assay, with standards having concentrations between 0 and 7 ng/ml. Masterwells producing up to 20 μg/ml were thus obtained (see Figure 25). One IgG1-producing clone produced by the method described above was designated CEP 24G-9G4. Selected cultures from these masterwells were then subjected to cloning in soft agarose.

Supernatants were also screened for the presence of human kappa light chain using an ELISA assay similar to that described in Example II,C. A standard was prepared from an IgG polyclonal stock (Cappel) and dilute supernatant samples were prepared in tissue culture medium. The assay was identical to that described in Example II,C with the exceptions that the plates were coated with a solution of mouse anti-human kappa light chain (1:300 dilution) and the conjugate was a goat anti-human kappa horseradish peroxidase (Tago) at a dilution of 1:6,000.

Recombinant IgG1 (rIgG1) was purified from culture supernatants of positive clones as follows: IgG antibodies were purified using protein A affinity chromatography (Sepharose CL-4B, Pharmacia). The cell culture supernatant was passed through the 4°C, buffer (pH 8.0, 0.14 M sodium potassium phosphate)-equilibrated protein A column. Non-protein A bound material was washed through the column with the pH 8.0 buffer (as described above) followed by a pH 6.0 buffer (0.1 M citrate) wash. When the O.D.$_{280}$ absorbancy of the flow-through material fell to baseline levels, the IgG was eluted with pH 3.5 buffer (0.1 M citrate). The peak fractions (determined as above) were pooled and immediately neutralized with 1 M Tris buffer (pH 8.0). The purified material was extensively dialyzed against PBS. Microconcentration centrifugation (Centriprep-10, Amicon Corp., Danver, MA) was used to concentrate dilute antibody preparations. The antibodies were filter sterilized through a 0.45 μm filter, aliquoted, and frozen at -70°C. The final preparations were examined for protein purity by silver nitrate staining of SDS-polyacrylamide gel electrophoresis-separated proteins.

EXAMPLE V

PRODUCTION OF A RECOMBINANT IgM ANTIBODY BY 4B9 TRANSFECTOMAS

This example demonstrates methods for the recombinant production of a 4B9 IgM antibody. This recombinant antibody possesses antigen binding and biological activity comparable to that observed with antibody secreted by the original parental 4B9 cell line.

The procedures for producing this antibody were essentially the same as described in Example IV above. Thus, the clone producing only human kappa light chain (as prepared in Example IV) was subjected to transfection by electroporation with plasmid DNA from the pNμA2.1 4B9 μ heavy chain construct prepared in Example I,B.3 (see Figure 20b).

The pNμA2.1 DNA was prepared for this transfection by growing bacteria containing the plasmid in T-broth and ampicillin as already described. The DNA was then subjected to the alkaline lysis method and purified on a PZ523 column. The DNA was digested to completion with BamH I prior to transfection.

Electroporation and cell culture of the transfected hosts were identical to that described in Example III. 14 to 21 days after the transfected cells were plated into 96-well dishes, the cultures were assayed for the presence of human μ heavy chain as described below. Cultures positive for human μ heavy chain were then subjected to cloning in soft agarose. Supernatants were screened for the presence of human IgM using an ELISA assay similar to that described in Example II,C. A standard was prepared from an IgM polyclonal stock (Cappel) and dilute supernatant samples were prepared in tissue culture medium. The assay was identical to that described in Example II,C with the exceptions that the plates were coated with a solution of goat anti-human IgM (Antibodies, Inc.) (1:1000 dilution) and the conjugate was a goat anti-human IgM horseradish peroxidase (American Qualex) at a dilution of 1:10,000. Antibody was then quantitated from the 40 masterwells producing the most immunoglobulin using the ELISA assay described, with standards having concentrations between 0 and 10 ng/ml. Masterwells producing up to 20-25 μg/ml were obtained (see Figure 26). One IgM-producing clone produced by the method described above was designated CEP 16/2B. The monoclonal antibody produced by this transfectoma was termed 16/2B (see Figs. 27-30).

Supernatants were also screened for the presence of human kappa light chain using an ELISA assay as described in Example II,C, except that a conjugate antiserum which was an anti-human kappa antibody (Tago) was used at a dilution of 1:3000.

Recombinant IgM (rIgM) was purified from culture supernatants of positive clones as follows: The IgM antibodies were purified using a murine monoclonal, anti-human IgM antibody affinity column. To prepare the anti-human IgM affinity column, 1 gram of dehydrated cyanogen bromide-activated Sepharose 4B (Pharmacia) was mixed with 15 ml of ice cold 1 mM HCl/distilled water. The hydrated gel was washed in 30 ml coupling buffer (0.1M carbonate (NaHCO₃) in 0.5 M NaCl, pH 8.2), drained to form a moist cake and was combined with the murine antibody which had been previously purified on protein A. The gel suspension was mixed end-over-end for 2 hours at room temperature and subsequently centrifuged at 200 x g for 5 min. To block still available reactive sites, the supernatant was removed, 10 ml of 1 M ethanolamine were added to the gel, and mixing was continued as above. The suspension was centrifuged at 200 x g for 5 min. and the supernatant was discarded. The gel was prepared for use with one wash in 0.1 M acetate/saline buffer (6.8 g sodium acetate trihydrate and 14.6 g NaCl dissolved in 500 ml distilled water containing 2.9 ml glacial acetic acid, pH 4.0), two washes in coupling buffer, and two washes in PBS. The gel was poured into a Pharmacia C10/10 column and stored at 4°C until use.

IgM antibodies were purified from clarified (2,000 x g) and filtered culture supernatants. If supernatant volumes greater than one liter were processed, antibodies were concentrated using Minitan ultrafiltration (Millipore Corp., Bedford, MA). Following sample loading, the column was washed with PBS, pH 8.0 until the absorbancy indicated no further protein in the flow-through. The bound antibody was eluted with 2 M MgCl₂ in PBS. The protein concentration was determined for each fraction by measuring the absorbance at 280 nm and the peak fractions pooled. The antibody pool was desalted on a G-25 Sephadex column.

The rIgM produced according to this invention was compared with the parental 4B9 IgM (parIgM) via the following assays:

a. SDS-PAGE (Fig. 27)

Purified antibodies were examined by SDS-polyacrylamide gel electrophoresis using 5-15% linear gradient gels (Laemmli, U.K., 1970, Nature (London), 227: 680). Samples (250 ng of antibody per lane) were boiled for 5 min. in sample buffer, containing 2% SDS and 5% 2-mercaptoethanol. Silver nitrate protein staining allowed visualization of the protein bands (Heukeshoven, J. and Dernck, R., 1985, Electrophoresis, 6:103-112).

The SDS-PAGE gel pattern of various novel immunoglobulins of this invention including rIgM, rIgG1 and rIgG2 are depicted in Figure 27, where the respective lanes contain the following:

lane 1: 4B9, parental lymphoblastoid IgM, kappa light chain
lane 2: 16/2B a rIgM, kappa light chain
lane 3: 3B, a rIgG1, kappa light chain
lane 4: 3B, a rIgG1, kappa light chain
lane 5: 1B1, a rIgG1, kappa light chain
lane 6: 8B8, a rIgG2, kappa light chain
lane 7: an IgG1 heteromyeloma with a lambda light chain

lane 8: molecular weight markers

The molecular weights of the heavy and light chains were estimated from a line derived from plotting the log of the molecular weight of the standards versus their migration distance. The molecular weights are given below:

| ANTIBODY | HEAVY CHAIN | LIGHT CHAIN |
|---|---|---|
| 16/2B (rIgM) | 82,000 | 25,000 |
| 4B9 (parIgM) | 79,000 | 25,000 |
| 3B (rIgG1) | 52,000 | 25,000 |
| 1B1 (rIgG1) | 52,000 | 25,000 |
| 8B8 (rIgG2) | 52,000 | 25,000 |

As shown in Figure 27, the heavy chain of rIgM has a slightly higher molecular weight than that of parIgM, (see lanes 1 and 2). It is believed that this slight variation in molecular weight is due to differences in glycosylation of the immunoglobulin protein in the recombinant host cell (mouse) vs. parental (human) cells. The amino acid sequences of the two immunoglobulins are believed to be the same. In addition, the light chains of the rIgM and pIgM also have the same molecular weights, approximately 25 kd.

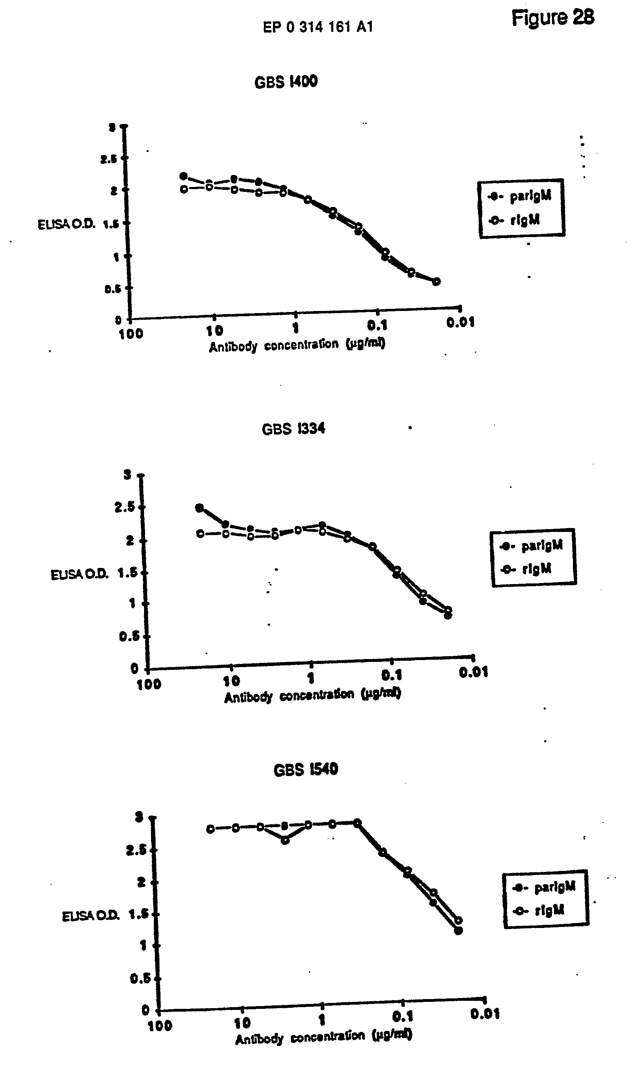

b. PLL-ELISA (Fig. 28)

The rIgM parIgM were tested for their ability to bind to various group B Streptococci (GBS) using the PLL-ELISA technique described in Example II,D. The results of these experiments are given in Figure 28. As indicated, the titration curves for the binding activity of rIgM and parIgM to Group B Streptococci clinical isolates I400 (Type Ic), 1334 (Type III) and I540 (Type III) are superimposable, thus demonstrating identical reactivity patterns for the two antibodies.

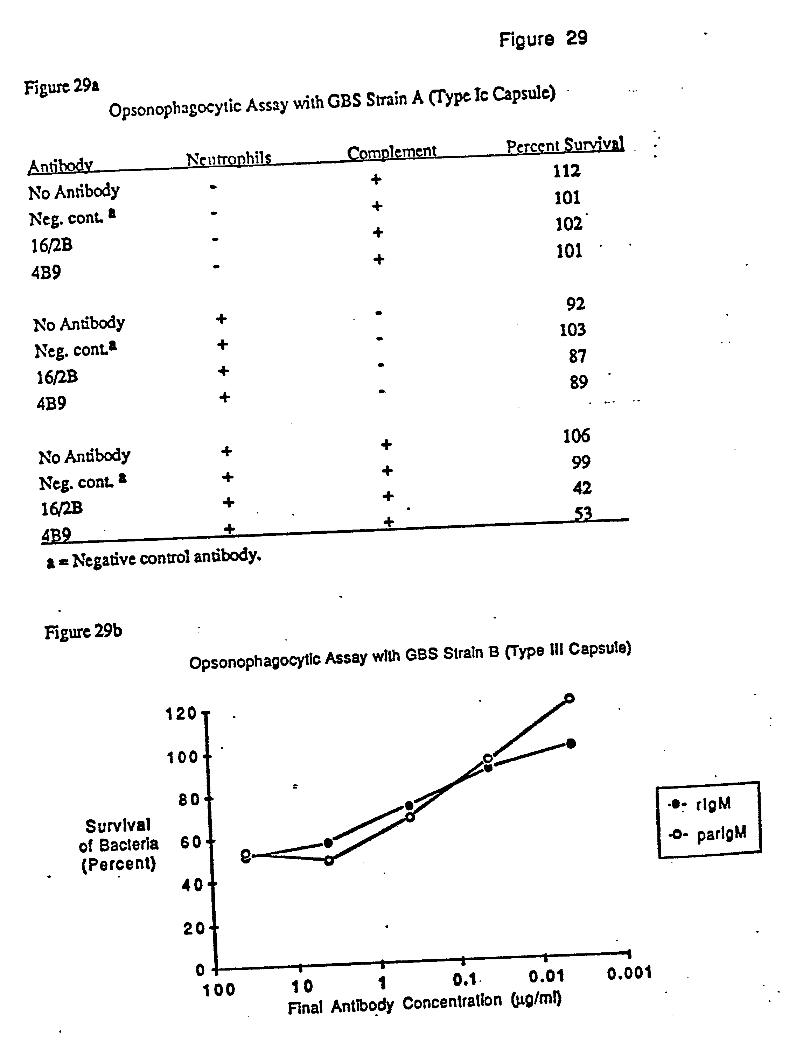

c. Opsonophagocytic Assay (Fig. 29)

The in vitro functional activity of the rIgM antibody and the parental lymphoblastoid derived antibody 4B9 were compared in an opsonophagocytic assay. This assay evaluated the ability of each monoclonal to kill bacteria as measured by decreases in colony forming units (CFU's) in the presence of human neutrophils and human complement. Neither antibody possessed the ability to decrease viable counts in the absence of either neutrophils or complement. This indicated complement dependent opsonisation as being the in vitro mechanism of action for both antibodies. In the assay format used, the opsonic capabilities of the two antibodies were indistinguishable.

Group B Streptococcus bacteria (GBS) were prepared by inoculating 10 ml of Todd Hewitt broth (catalog no. 11736, BBL Microbiology Systems, BD, MD) (supplemented with 20 mM sodium phosphate) from an overnight stationary phase culture. Broth cultures were incubated at 37° C on a shaker and grown to logarithmic phase, at which time the cultures were diluted in Hank's balanced salt solution containing 0.1% gelatin and 5 mM HEPES buffer (HBSS/gel). The bacterial concentrations were adjusted to $1 \times 10^4$ CFU's/ml by measuring the optical density at 660 nm and making appropriate dilutions.

Human neutrophils were isolated according to van Furth and van Zwet, "In Vitro Determination Of Phagocytosis And Intracellular Killing By Polymorphonuclear And Mononuclear Phagocytes," in Handbook Of Experimental Immunology., Vol. 2, D.M. Weir, (ed.), 2nd edition, Blackwell Scientific Publications, Oxford, pp. 36.1-36.24 (1973) with several modifications. 50 ml of freshly drawn heparinized blood was underlayed with Ficoll-Pacque and centrifuged. The resulting cell pellet (red blood cells and neutrophils) was washed once with PBS and resuspended to a total volume of 17 ml in 37° C PBS. This suspension was added to 34 ml of 2% dextran (in 37° C PBS) and the contents gently but thoroughly mixed end-over-end. The mixture was then incubated for 20 min. at 37° C to allow the red blood cells to sediment, the supernatant (containing neutrophils) was removed, washed twice in 4° C PBS, once in HBSS/gel, and suspended in same to $5 \times 10^7$ neutrophils/ml.

For the complement source, freshly drawn human AB serum was thrice adsorbed with live bacteria (Bjornson, A.B. and Michael, J.G., J. Inf. Dis., 130 Suppl: S119-S126 (1974)) corresponding to the organisms used in the assay. EDTA (10 mM) was added to the serum prior to the adsorption to minimize complement activation. The serum was replenished with divalent cations by adding CaCl₂ (10 mM) to it just prior to each assay. Complement was stored at -70°C until use in the assay.

The assays were performed in sterile 1.5 ml polypropylene tubes. To each assay tube was added 125 μl of bacterial suspension, 50 μl of heat inactivated fetal calf serum (FCS, treated at 56°C for 30 min.), and 200 μl of purified antibody (i.e., rlgM or parlgM) in HBSS/gel. After 30 min. incubation at 37°C, 75 μl of human complement and 50 μl of the neutrophil suspension were added and the tubes tumbled at 37°C for 1 hour. The final mixture included 10% heat inactivated FCS, 15% human complement, $5 \times 10^6$ neutrophils/ml, $2.5 \times 10^3$ CFU's/ml, and antibody. As the negative control (100% survival) for each assay, the test antibodies were replaced with an irrelevant human IgM monoclonal antibody in the reaction mixtures. The surviving bacteria (CFU's) in each reaction mixture were quantitated using a standard agar pour plate method. Molten trypticase soy broth containing 0.4% agarose was aseptically aliquoted in 3 ml volumes and kept at 45°C. To each tube were added 20 μl from one reaction mixture. The tubes were vortexed and poured into 100 mm plastic Petri dishes which contained 30 ml of typticase soy agar (catalog no. 11043, BBL Microbiology Systems, BD, MD). The agarose was allowed to solidify and then overlayed with 3-4 ml of trypticase soy/agarose. After incubation for 18 hour at 37°C, the CFU's were enumerated by counting colonies. Percentage survival was calculated by dividing the CFU's in each test sample mixture by the CFU's in the negative control mixtures.

Figure 29a shows that the reduction in CFU's (decreased survival) occurred only in the presence of neutrophils, complement and specific antibody (40 μg/ml in this assay). These data indicate that these antibodies, like other IgM antibodies, require complement to mediate opsonisation.

Figure 29b shows titrations of the rlgM antibody (16/2B) versus parlgM (4B9) in the opsonophagocytic assay. Equal concentrations of these two purified antibodies resulted in approximately equivalent levels of opsonisation.

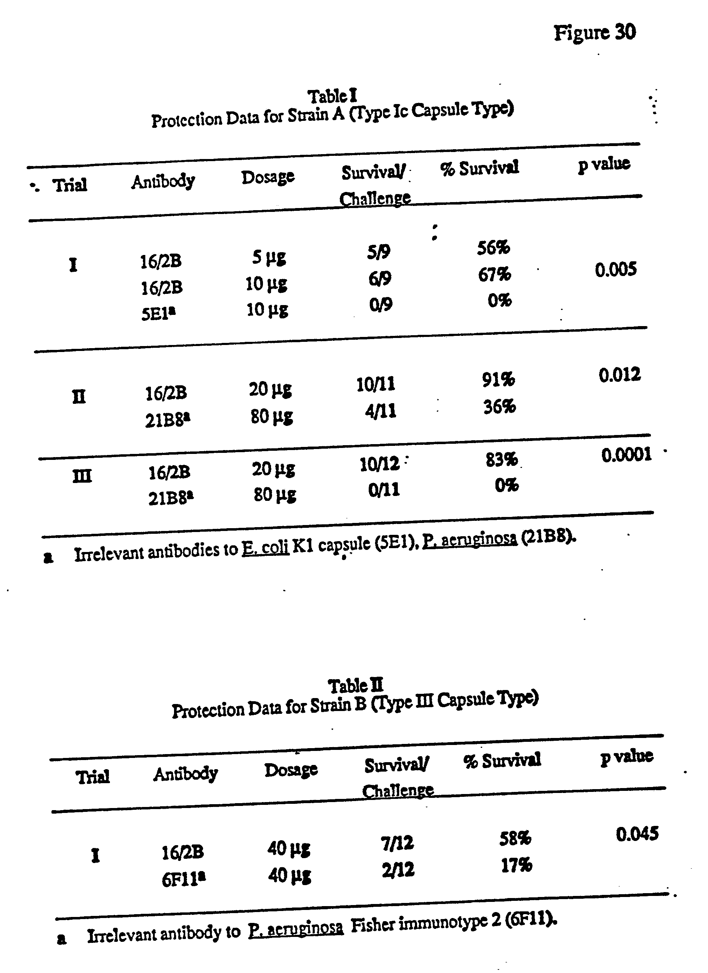

<u>d. In Vivo Protection Studies</u> (Fig. 30)

A neonatal rat model was used for therapeutic protection studies using the rlgM produced according to this invention. Three to four day old outbred Sprague-Dawley (BK:SD) rat pups (housed with their dams), were injected intraperitoneally with 40 μl of antibody 4 hours after intraperitoneal challenge with 40 μl of either Strain A (Type 1c Capsule Type) or Strain B (Type III Capsule Type) GBS.

To prepare the bacteria, Todd Hewitt broth (supplemented with 20 mM sodium phosphate) culture tubes were inoculated from overnight stationary phase cultures. At logarithmic growth phase, the tubes were centrifuged at 22°C, 4550 x g for 10 min., washed once with 25 ml broth, and resuspended in same to appropriate OD₆₆₀. For each experiment, the challenge dose was quantitated by plating dilutions of the bacteria on trypitcase soy agar plates. Culture purity was confirmed by plating on blood agar. Challenge doses consisted of 400-600 colony forming units per rat (10-50 LD50's depending on the challenge strain).

Rat litters were prescreened for sensitivity to infection to ensure uniform susceptibility to infection. Two pups from each litter were injected with 500 CFUs of the challenge strain one day prior to initiating the protection experiment. Eighteen hours later, the pups were scored for survival and only those litters with group B Streptococcal sensitive pups were used in the subsequent assay. Each litter was divided into all treatment groups to eliminate litter to litter variation. The pups were examined twice daily for symptoms and scored for survival over 5 days. All experiments included an irrelevant human monoclonal antibody as a negative control.

These GBS therapeutic protection studies are presented in Tables 1 and 2 of Figure 30. The significance of the protection was calculated using the Fisher exact test (B. Rosner, Fundamentals Of Biostatistics, Duxbury Press 1982). Antibody 16/2B (rlgM) demonstrated consistent protection against Group B Streptococcal clinical isolates of high virulence in this neonatal rat model system. Matched doses of 16/2B and the parental antibody 4B9 provided approximately equivalent protection (data not shown).

EXAMPLE VI

PRODUCTION OF ANTIBODIES BY 9D1 V GENE TRANSFECTOMAS

This Example demonstrates a method for the production of human monoclonal antibody in mouse cells which uses the constructs containing the 9D1 variable region genes, $V_H$ and $V_L$, described in Example I herein. The antibody expressed by the 9D1 cell line was of the $IgG_2$ subclass and the novel antibody was re-expressed in mouse cells as an $IgG_2$. The novel antibody possessed functional activity directed against the Pseudomonas aeruginosa genus Fisher serotypes 3 and 7, similar to that of the parent antibody secreted by the 9D1 cell line.

## A. Transfection into Mouse Cells

Transformed bacteria containing pN$\gamma$2-D2H or pN$\gamma$2-D1H were grown overnight in LB broth containing 100 $\mu$g/ml ampicillin and 0.5% glucose. Plasmid DNA was prepared by the alkaline lysis method and was further purified on two CsCl gradients. One hundred micrograms of plasmid DNA from sample pN$\gamma$2-D2H was digested to completion with BamH I, then ethanol precipitated and resuspended to 1 $\mu$g/$\mu$l in sterile TE for transfection. Sample pN$\gamma$2-D1H was not linearized prior to transfection. One hundred micrograms of plasmid DNA from sample pG$\lambda$-D2e was linearized with Sal I, sample pG$\lambda$-D2h with Eco RI, and sample pG$\lambda$-D1 with Pvu I, and then ethanol precipitated and resuspended in sterile TE to 1 $\mu$g/$\mu$l. Plasmid DNA prepared as described above, was transfected into Ag8.653 cells by electroporation as described in Example II, section A. Two heavy chain clones and three lambda light chain clones, two of which represented the same allele (pG$\lambda$-D2e and pG$\lambda$-D2h) were transfected in the various combinations as follows:

(1) pN$\gamma$2-D1H heavy chain (10 $\mu$g) + pG$\lambda$-D2e light chain (10 $\mu$g);
(2) pN$\gamma$2-D1H heavy chain (10 $\mu$g) + pG$\lambda$-D2h light chain (10 $\mu$g);
(3) pN$\gamma$2-D1H heavy chain (10 $\mu$g) + pG$\lambda$-D1 light chain (10 $\mu$g);
(4) pN$\gamma$2-D2H heavy chain (10 $\mu$g) + pG$\lambda$-D2e light chain (10 $\mu$g);
(5) pN$\gamma$2-D2H heavy chain (10 $\mu$g) + pG$\lambda$-D2h light chain (10 $\mu$g);
(6) pN$\gamma$2-D2H heavy chain (10 $\mu$g) + pG$\lambda$-D1 light chain (10 $\mu$g).

## B. Cell Culture

Cell culture was identical to that described in Example II, section B. Fourteen to twenty-one days after plating in 24-well dishes, the cultures were assayed for the presence of human gamma heavy chain, as described in Example II, section C, and human lambda light chain, as described below. The number of wells positive for human gamma heavy chain out of the total number tested for the above-enumerated combinations were as follows: 1) 18 out of 48; 2) 9 out of 48; 3) 0 out of 48; 4) 0 out of 24; 5) 0 out of 24; and 6) 0 out of 24.

Cultures positive for human gamma heavy chain were screened for human lambda light chain in an ELISA assay. The assay was identical to that described for the human gamma heavy chain, except that the peroxidase conjugate was a goat anti-human lambda specific antibody at a dilution of 1:7,500.

Cultures positive for both human gamma heavy chain and human lambda light chain were cloned by limiting dilution, as described in Example II, section B. Quantitation of the antibody from 15 day spent cultures of four different clones yielded IgG concentration ranging from 45 to 60 $\mu$g/ml. The parental lymphoblastoid cell line (9D1) secreted IgG at a concentration of 1 $\mu$g/ml.

## C. Detection of Functional Activity

### 1. Antigen Binding Activity

Supernatants positive for both human gamma heavy chain and human lambda light chain were screened for the presence of anti-P. aeruginosa activity using the ELISA technique described in Example II, section D, except that the plates were coated with viable P. aeruginosa organisms of the F3 immunotype. All supernatants positive for human IgG bound to the P. aeruginosa plates but not control plates without bacteria.

Antibodies produced by the cloned transfectoma cell lines were further characterized as described in Example II, section D, except that the nitrocellulose discs were spotted with 65 different isolates of P. aeruginosa. The immunoglobulin molecules produced by the transfectoma reacted identically to the parental antibody produced by the 9D1 cell line with respect to all isolates tested.

2. Opsonophagocytic Assay

Functional activity of the transfectoma antibody was further examined in an in vitro opsonophagocytosis assay which compared the bactericidal activity of the antibody with its parental counterpart in the presence and absence of both human neutrophils and human complement.

Bacteria were prepared by inoculating 10 ml of trypticase soy broth with 50 $\mu$l of an overnight broth culture of P. aeruginosa, IATS serotypes 5 and 11. The cultures were incubated at 37° C on a shaker for 3 hours, 1.5 ml was centrifuged for 3 minutes at 10,000 x g, and the pellet resuspended in 1.5 ml of Hank's balanced salt solution containing 0.1% gelatin and 5 mM HEPES (HBSS/Gel). This bacterial suspension was diluted in HBSS/Gel to yield a concentration of approximately 1 x $10^5$ CFU/ml. Human neutrophils were isolated according to van Furth and van Zwet, Handbook of Experimental Immunology, Vol. 2, D. M. Weir, (ed.), 2nd edition, Blackwell Scientific Publications, Oxford, pages 36.1-36.24 (1973) with several modifications. Twenty-five ml of heparinized blood was layered over 15 ml of Lymphocyte Separation Medium (Litton Bionetics) and centrifuged. The pellet was washed once in PBS and resuspended in three volumes of dextran. After a 20 minute incubation at 37° C to allow the RBCs to sediment, the neutrophil-rich supernatant was removed, washed twice in 4° C PBS, once in HBSS/Gel, and suspended in HBSS/Gel to 5 x $10^5$ neutrophils/ml. For a source of complement, serum from the same donor as had supplied neutrophils was adsorbed four times with bacteria corresponding to the organisms used in the assay.

For the bactericidal assay, 50 $\mu$l of transfectoma or parental antibody (diluted in HBSS/Gel) or appropriate control antibody and 100 $\mu$l of the bacterial suspension were added to sterile 1.5 ml microfuge tubes and incubated at room temperature for 30 minutes. This was followed by the addition of 50 $\mu$l each of the neutrophil preparation and adsorbed serum, and 250 $\mu$l HBSS/Gel. To assess the contribution of complement to the bactericidal activity of the antibodies, in some assays the adsorbed serum was heat inactivated at 56° C for 30 minutes. Fifty microliters of HBSS/Gel were substituted in assays where no neutrophils were added. After adding all assay components, the tubes were rotated end-over-end in a mixer for one hour at 37° C. To assess the extent of killing, 10 $\mu$l were removed and plated on TSA plates using standard pour-plate techniques. After incubating the plates overnight at 37° C, the colonies were counted. All assays were performed in duplicate.

In each experiment, the parental LCL 9D1 antibody had specific activity against the IATS 5 strain in the bacterial destruction assay. The IgG2 transfectoma form of the parental antibody displayed comparable opsonophagocytic activity in the assay.

EXAMPLE VII

PRODUCTION OF HUMAN ANTIBODIES WITH HEAVY AND LIGHT CHAINS OF DIFFERENT ANTIGENIC SPECIFICITY

This Example demonstrates the production of human monoclonal antibodies in mouse cells using the constructs containing the 4B9 variable region heavy chain gene, $V_H$, described in Example I, and the 9D1 variable region light chain gene, $V_L$, described in Example I. The Example makes use of a transfectoma derived from Example II which expresses the $\gamma$2 heavy chain of 4B9 and no detectable light chain. The novel immunoglobulin molecule expressed by the transfectoma possessed antigen binding activity against both Group B Streptococcus and Pseudomonas aeruginosa.

Transformed bacteria containing pG$\lambda$-D2e were grown overnight in LB broth containing 100 $\mu$g/ml ampicillin and 0.5% glucose. One hundred micrograms of plasmid DNA were linearized with Sal I, ethanol precipitated and resuspended in sterile TE to 1 $\mu$g/$\mu$l. Plasmid DNA prepared as described above was transfected by electroporation as described in Example III into a transfectoma producing only heavy chains of the 4B9 specificity, described in Example II. The dominant selectable marker utilized was the neomycin resistance expression vector pN.1 described in Example I. The following combination was performed: pN.1

(1 μg) + pGλ-D2e (10 μg).

Cell culture was identical to that described in Example II, section B with the following exceptions. Viable cells were suspended in RPMI 1640/20% FCS (containing 0.25 mg/ml G418 (Gibco)) at a density of 1 x 10$^5$ cells/ml. The cell suspension was plated into 24-well tissue culture dishes (Costar), 1 ml/per well. The cells were fed selective medium every 4 to 5 days. Fourteen to twenty-one days after the transfected cells were plated into 24-well dishes, the cultures were assayed for the presence of human gamma heavy chain and human lambda light chain, as described in Example II, section C and Example VI. Cultures positive for human gamma heavy chain and lambda light chain were expanded to tissue culture flasks in order to determine levels of immunoglobulin production and antigen binding activity.

Supernatants positive for both human gamma heavy chain and human lambda light chain were screened for the presence of antibody activity against both Group B Streptococcus and P. aeruginosa in the nitrocellulose disc assay described in Example II, section D. The immunoglobulin molecules produced by the transfectoma bound strongly to the Group B Streptococcus isolates and weakly to the P. aeruginosa isolates.

From the foregoing, it will be appreciated that methods of the present invention are capable of providing recombinant human immunoglobulin molecules which specifically bind to antigenic targets bound by monoclonal antibodies produced by the donor cell lines. Moreover, the recombinant compositions of the invention possess effector functions which may have important effects in vitro and/or in vivo, such as opsonophagocytosis. This allows therapeutic compositions to be more economically and easily developed against a variety of human diseases. In addition, the recombinant human immunoglobulin compositions find uses in diagnosis, including immunoassays and other well known procedures.

Transfectomas prepared by the processes described herein and producing novel recombinant antibodies of this invention are exemplified by cultures deposited in the American Type Culture Collection, Rockville, Maryland. One culture was deposited on August 30, 1988 and was there identified as: CEP 16/2B, ATCC No. CRL 9806, and a second culture was deposited on September 14, 1988 and was identified as: CEP 24G-9G4, ATCC No. CRL 9824.

Although the present invention has been described in some detail by way of illustration and example for purposes of clarity of understanding, it will be apparent that certain changes and modifications may be practiced within the scope of the appended claims.

## Claims

1. A method for producing a recombinant human immunoglobulin in a eucaryotic host cell, which method comprises:
transfecting into said eucaryotic cell operably linked first and second genes, wherein the first gene codes for the constant region of a human heavy chain and the second gene codes for the variable region of a human heavy chain;
transfecting into said cell operably linked genes coding for the variable and constant regions of a human light chain; and
culturing said transfected cell and recovering therefrom said immunoglobulin.

2. The method of claim 1, wherein the genes coding for the constant and variable regions of the human heavy chain or of the human light chain are cloned from the same or different donor cells, or
wherein the gene coding for the variable region of the human heavy chain and the gene coding for the variable region of the human light chain are cloned from the same or different donor cells, or
wherein the gene coding for the constant region of the human heavy chain and the gene coding for the constant region of the human light chain are cloned from the same or different donor cells.

3. The method of claim 1, wherein the recombinant human immunoglobulin is a single heavy-light chain pair functionally associated within said transfected host cell, or
wherein the recombinant human immunoglobulin is a tetramer or aggregates thereof, or
wherein the recombinant human immunoglobulin produced by said transfected host cell culture is substantially free of other human proteins.

4. The method of claim 1, wherein the transfected host cell is mammalian, such as human or murine, preferably a lymphocyte which preferably is of a B-cell lineage, preferably selected from the group consisting of a lymphoblastoid cell, a B-cell, and a plasmacytoma.

5. The method of claim 1, wherein said first gene codes for a constant region of a human heavy chain selected from the group consisting of μ, γ1, γ2, γ3, γ4, α1, α2, δ, and ε.

6. The method of claim 1, wherein the genes coding for the human heavy chain constant and variable regions are operably linked in an expression vector, or wherein the genes coding for the human heavy chain and the genes coding for the human light chain are transfected simultaneously or sequentially into the host cell.

7. The method of claim 1, wherein the human light chain is a kappa chain, or a lambda chain.

8. A method for switching the class of a human immunoglobulin from a first class to a second class, comprising:

(a) transfecting into a eucaryotic host cell a gene coding for the heavy chain variable region of said immunoglobulin, wherein the gene is operably linked to a gene which codes for a heavy chain constant region of said second class;

(b) transfecting into the host cell a gene coding for the light chain variable region of said immunoglobulin, wherein the light chain variable region gene is operably linked to a gene coding for a light chain constant region; and

(c) cultivating said transfected host under culturing conditions and recovering a human immunoglobulin having the characteristics of said second class.

9. The method of claim 8, wherein said first class is IgM and said second class is IgG.

10. The method of claim 9, wherein the genes coding for the human heavy chain variable and constant regions are operably linked in an expression vector.

11. The method of claim 8, wherein the genes coding for the human heavy chain and human light chain are transfected simultaneously or sequentially into the host cell.

12. A recombinant human immunoglobulin.

13. The recombinant human immunoglobulin of claim 12, wherein the immunoglobulin comprises a single heavy-light chain pair, or

wherein the immunoglobulin comprises a tetramer or aggregates thereof, or wherein the variable and constant regions of the heavy or light chain of said immunoglobulin are coded for by genes cloned from the same or different donor cells or

wherein the variable regions of the heavy and light chains of said immunoglobulin are coded for by genes cloned from the same or different donor cells, or

wherein the constant regions of said heavy and light chains are coded for by genes cloned from the same or different donor cells.

14. An Fab, F(ab')$_2$ or Fv fragment of the recombinant immunoglobulin of claim 12,

15. A recombinant human immunoglobulin comprising the variable region of the 4B9 monoclonal antibody and a constant region selected from the group consisting of IgG1, IgG2 and IgM;

the recombinant human immunoglobulin produced by a transfectoma having the identifying characteristics of ATCC No. CRL 9806 or ATCC No. CRL 9824 as deposited with the ATCC; or

a recombinant human immunoglobulin comprising the variable region of the 9D1 monoclonal antibody.

16. A recombinant human immunoglobulin molecule produced by the process of culturing a eucaryotic host cell line transfected with (a) a DNA sequence coding for a human heavy chain, and/or (b) a DNA sequence coding for a human light chain, wherein said cell line is capable of expressing said transfected DNA sequences, and preferably is a plasmacytoma producing either a human light chain or a human heavy chain.

17. A DNA construct for expression of a recombinant human immunoglobulin heavy or light chain, comprising:

a first DNA sequence coding for a human variable region polypeptide of one of said chains; and

a second DNA sequence coding for a constant region polypeptide, wherein the second DNA sequence is joined directly or through an intron in reading frame at its 5' end to the 3' end of said first DNA sequence.

18. A DNA construct according to claim 17 having transcriptional and translational initiation and termination regulatory sequences recognized by a mammalian host, in regulatory relationship with said first and second DNA sequences.

19. A eucaryotic cell line producing a recombinant human immunoglobulin molecule, preferably containing DNA constructs for expression of recombinant human immunoglobulin molecules, said DNA constructs comprising:

a first DNA construct comprising a DNA sequence coding for a human heavy chain variable region polypeptide operably linked to a DNA sequence coding for a human heavy chain constant region polypeptide;

a second DNA construct comprising a DNA sequence coding for a human light chain variable region polypeptide operably linked to a DNA sequence coding for a human light chain constant region polypeptide;

48

and each of said first and second DNA constructs containing transcriptional and translational regulatory initiation and termination sequences functional in said eucaryotic cell line and in regulatory relationship with said first and second DNA constructs.

20. A DNA construct coding for the expression of a human immunoglobulin light chain polypeptide in a eucaryotic cell, wherein said construct contains a murine or human heavy chain transcriptional enhancer sequence.

21. A method for expressing DNA sequences coding for a mammalian immunoglobulin light chain polypeptide, preferably a lambda chain in a mammalian cell, wherein said mammalian cell is transfected with a DNA construct coding for said immunoglobulin polypeptide, comprising:

inserting in said DNA construct a DNA sequence coding for a functional region of a murine or human heavy chain transcriptional enhancer.

22. A DNA construct for expressing an immunoglobulin polypeptide, in particular a light or heavy chain polypeptide in a mammalian cell, wherein said construct contains a cytomegalovirus sequence from the upstream region of the major immediate early genes of cytomegalovirus.

23. The DNA construct of claim 22, wherein the cytomegalovirus sequence is a transcriptional enhancer sequence, preferably selected from the group consisting of a sequence for the enhancer of human cytomegalovirus, simian cytomegalovirus, and murine cytomegalovirus or a promoter sequence preferably selected from the group consisting of a sequence for the promoter of human cytomegalovirus, simian cytomegalovirus, and murine cytomegalovirus.

24. A DNA construct according to claim 23, additionally containing a DNA sequence coding for a cytomegalovirus promoter sequence, preferably
selected from the group consisting of a sequence for the promoter of human cytomegalovirus, simian cytomegalovirus, and murine cytomegalovirus.

25. A method for expressing DNA sequences coding for immunoglobulin polypeptides in a mammalian cell, wherein said mammalian cell is transfected with a DNA construct coding for said immunoglobulin polypeptide, comprising:
inserting in said DNA construct a DNA sequence from the upstream region of the immediate early genes of cytomegalovirus coding for a transcriptional enhancer, preferably selected from the group consisting of a sequence for the enhancer of human cytomegalovirus, simian cytomegalovirus, and murine cytomegalovirus.

26. The method according to claim 25, wherein the DNA sequence for the transcriptional enhancer is inserted in said DNA construct between sequences coding for the rearranged variable region and the constant region.

27. A pharmaceutically acceptable composition useful in treating or preventing bacterial infection in humans, comprising a recombinant human immunoglobulin molecule and a pharmaceutically acceptable excipient.

49

Figure 1a

pSV2-GPT

pUC18

pG

pUCG

2 kb

Figure 1b

Figure 1c

Figure 1d

EP 0 314 161 A1

Figure 1e

Figure 1f

EP 0 314 161 A1

Figure 2a

pSV2-NEO

EcoR I (1/5728)
Pst I (207)
BamH I (752)
SV40 splice sites and poly(A) signal
(4977) Pst I
amp'
Pst I (1637)
pBR322 ori
SV40 ori
neo
Nde I (3665)
Pst I (2560)
Avr II (3108)
Hind III (2976)
Bgl II (2772)

Hind III Digest
Klenow fill in
Ligate and Transform

pN.1

EcoR I (1/5732)
Pst I (207)
BamH I (752)
SV40 splice sites and poly(A) signal
(4981) Pst I
amp'
Pst I (1637)
pBR322 ori
SV40 ori
neo
Nde I (3669)
Pst I (2560)
Avr II (3112)
Bgl II (2772)

1 kb

Figure 2b

pN.1

EcoR I (1/5732)
Pst I (207)

(4981) Pst I

BamH I (752)

amp'

SV40
splice sites
and
poly(A) signal

pBR322
ori

SV40
ori

neo

Pst I (1637)

Nde I
(3669)

Avr II
(3112)

Bgl II
(2772)

Pst I (2560)

Not I Linkers

5' TAGCGGCCGCA 3'
3'    CGCCGGCGTAT 5'

Heat to 65°
Cool

Nde I digest

Ligate
Transform

EcoR I (1/5740)
Pst I (207)

(4989) Pst I

BamH I (752)

amp'

SV40
splice sites
and
poly(A) signal

pN.2

pBR322
ori

SV40
ori

neo

Pst I (1637)

Nde I/Not I
(3669)

Avr II
(3112)

Bgl II
(2772)

Pst I (2560)

1 kb

Figure 3

Phage 5A

pUC18

Cγ2 gene

pγ2

2 kb

Figure 4

1 kb

Figure 5

Figure 6

Figure 7

2 kb

EP 0 314 161 A1

Figure 8

EP 0 314 161 A1

2 kb

Figure 9

EP 0 314 161 A1

Figure 10

2 kb

EP 0 314 161 A1

Figure 11

Figure 12

Figure 13

Figure 14

Figure 15a

EP 0 314 161 A1

Figure 15b

restricted here for
transfection
(EcoR I)

A2H  L-VDJ gene

Bg

E   H

H         Bg

3'                                                                                                      5'

EP 0 314 161 A1

Hind III

Bgl II

BamH I | Xho I

Cγ2  gene

BamH I

pGγ 2-A2H

3'                                                                                      5'

restricted here for
transfection
(EcoR I)

EcoR I (1/6177)

Pst I (207)

BamH I
(752)

Pst I
(4425)

amp^r

SV40
splice sites
and
poly(A) signal

pBR322
ori

Ecogpt

SV40
ori

(3113) Nde I

Pvu II
(2882)

Avr II
(2556)

Kpn I (2338)

2 kb

Figure 16

# Phage A2H

Hind III    Hind III    EcoR I    Bgl II    L-VDJ gene →    Bgl II    Hind III

Hind III digest

Hind III digest
Phosphatase

BamH I    (Bgl II)    Hind III    Cγ1 gene    poly(A) site    Pvu II    Bgl II  BamH I

EcoR I (1/5740)    Pst I (207)
(4989) Pst I    BamH I (752)
amp^r    SV40 splice sites and poly(A) signal
pBR322 ori    SV40 ori    neo
Nde I (3669)    Pst I (1637)
Avr II (3112)    Bgl II (2772)    Pst I (2560)

pNγ1.1

Ligate
Transform

restricted at BamH I sites for transfection

Hind III    EcoR I    Bgl II    L-VDJ gene →    Bgl II    Hind III

restricted at BamH I sites for transfection

BamH I    (Bgl II)    Hind III    Cγ1 gene    poly(A) site    Pvu II    Bgl II  BamH I    Pst I

EcoR I (1/5740)    Pst I (207)
(4989) Pst I    BamH I (752)
amp^r    SV40 splice sites and poly(A) signal
pBR322 ori    SV40 ori    neo
Nde I (3669)    Pst I (1637)
Avr II (3112)    Bgl II (2772)    Pst I (2560)

pNγ1A2.1

2 kb

Figure 17

Figure 18

EP 0 314 161 A1

Figure 19a

EP 0 314 161 A1

Figure 19b

D2H L-VDJ gene

Hind III    Bgl II    Bgl II    Hind III

5'    3'

e

restricted here for transfection (BamH I)

restricted here for transfection (BamH I)

Hind III

Cγ2 gene

BamH I    Xho I    Bgl II    BamH I

5'    3'

pNγ 2-D2H

EcoR I (1/5732)
Pst I (207)

(4961) Pst I

amp'

SV40 splice sites and poly(A) signal

BamH I (752)

pBR322 ori

SV40 ori

neo

Pst I (1637)

Nde I (3669)

Avr II (3112)    Bgl II (2772)    Pst I (2560)

2 kb

EP 0 314 161 A1

Figure 20a

## Phage μ

Hind III    Hind III

Hind III    μ switch region    Cμ gene    μ membrane domain

EcoR I    EcoR I    EcoR I    BamH I  Hind III

## pUC18

(183)
Nde I    Pvu II(306)    POLYLINKER
Hind III (399)
amp^r
2686 bp    BamH I (429)
Pvu II (528)    EcoR I (450)

Hind III Digest

Hind III Digest
Phosphatase

Ligate
Transform

Hind III    μ switch region    Cμ gene    μ membrane domain

EcoR I    EcoR I    EcoR I

EcoR I
BamH I
Hind III
BamH I

Nde I
(183)    Pvu II
(306)

amp^r    Pvu II (528)    pμ.2

2 kb

Figure 20b

Figure 21

Figure 22

Phage A1κ

pGκ A1-HCMVE

2 kb

Figure 23

germline λ

HP2-Cλ#5, pCλ3/4

λD1

λD2e

λD2h

5 kb

Figure 24a

pGλ-D1

2 kb

EP 0 314 161 A1

Figure 24b

pGλ-D2h

2 kb

EP 0 314 161 A1

Figure 24c

pGλ-D2e

2 kb

EP 0 314 161 A1

Figure 25

Figure 26

EP 0 314 161 A1

Figure 27

Figure 28

Figure 29

Figure 29a

Opsonophagocytic Assay with GBS Strain A (Type Ic Capsule)

| Antibody | Neutrophils | Complement | Percent Survival |
|---|---|---|---|
| No Antibody | - | + | 112 |
| Neg. cont. [a] | - | + | 101 |
| 16/2B | - | + | 102 |
| 4B9 | - | + | 101 |
| | | | |
| No Antibody | + | - | 92 |
| Neg. cont. [a] | + | - | 103 |
| 16/2B | + | - | 87 |
| 4B9 | + | - | 89 |
| | | | |
| No Antibody | + | + | 106 |
| Neg. cont. [a] | + | + | 99 |
| 16/2B | + | + | 42 |
| 4B9 | + | + | 53 |

a = Negative control antibody.

Figure 29b

Opsonophagocytic Assay with GBS Strain B (Type III Capsule)

Survival of Bacteria (Percent) vs Final Antibody Concentration (µg/ml)

-•- rIgM
-o- parIgM

Figure 30

## Table I
### Protection Data for Strain A (Type Ic Capsule Type)

| Trial | Antibody | Dosage | Survival/ Challenge | % Survival | p value |
|---|---|---|---|---|---|
| I | 16/2B | 5 µg | 5/9 | 56% | |
| | 16/2B | 10 µg | 6/9 | 67% | 0.005 |
| | 5E1[a] | 10 µg | 0/9 | 0% | |
| II | 16/2B | 20 µg | 10/11 | 91% | 0.012 |
| | 21B8[a] | 80 µg | 4/11 | 36% | |
| III | 16/2B | 20 µg | 10/12 | 83% | 0.0001 |
| | 21B8[a] | 80 µg | 0/11 | 0% | |

[a] Irrelevant antibodies to E. coli K1 capsule (5E1), P. aeruginosa (21B8).

## Table II
### Protection Data for Strain B (Type III Capsule Type)

| Trial | Antibody | Dosage | Survival/ Challenge | % Survival | p value |
|---|---|---|---|---|---|
| I | 16/2B | 40 µg | 7/12 | 58% | 0.045 |
| | 6F11[a] | 40 µg | 2/12 | 17% | |

[a] Irrelevant antibody to P. aeruginosa Fisher immunotype 2 (6F11).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | JOURNAL OF IMMUNOLOGICAL METHODS, vol. 100, nos. 1/2, 1987, pages 5-40, Elsevier Science Publishers B.V. (Biomedical Division), Amsterdam, NL; K. JAMES et al.: "Human monoclonal antibody production. Current status and future prospects" * Pages 32,33 * | 1-20,26 ,27 | C 12 N  15/00 A 61 K  39/395 C 12 N  5/00 |
| A | NATURE, vol. 321, 29th May 1986, pages 522-525; P.T. JONES et al.: "Replacing the complementarity-determining regions in a human antibody with those from a mouse" * Introduction * | 1-20,26 ,27 | |
| A | NUCLEIC ACIDS RESEARCH, vol. 12, no. 22, November 1984, pages 8407-8414, IRL Press Ltd, Cambridge, GB; Y. TSUJIMOTO et al.: "Molecular cloning of a human immunoglobulin gamma chain variable sequence" * Whole article * | 1-27 | TECHNICAL FIELDS SEARCHED (Int. Cl.4) C 12 N C 12 P A 61 K |

The present search report has been drawn up for all claims

| Place of search | Date of completion of the search | Examiner |
|---|---|---|
| THE HAGUE | 14-02-1989 | CUPIDO M. |

EPO FORM 1503 03.82 (P0401)